# EUROPEAN PATENT SPECIFICATION

(11) **EP 3 856 894 B1**
(45) Date of publication and mention of the grant of the patent: **02.08.2023**
(21) Application number: 19769366.6
(22) Date of filing: 25.09.2019
(51) Int. Cl.: C12N 5/077

(54) **METHODS FOR DIFFERENTIATING MESENCHYMAL STEM CELLS**
VERFAHREN ZUR UNTERSCHEIDUNG MESENCHYMALER STAMMZELLEN
PROCÉDÉS DE DIFFÉRENCIATION DE CELLULES SOUCHES MÉSENCHYMATEUSES

(30) Priority: 25.09.2018 EP 18196717; 12.04.2019 EP 19169084
(43) Date of publication of application: 04.08.2021
(73) Proprietor: BioSenic SA, 1435 Mont-Saint-Guibert (BE)
(72) Inventor: CHAMPLUVIER, Benoît, 1300 Wavre (BE); PIETRI, Sandra, 4520 Moha (BE); NORMAND, Sylvain, 1000 Bruxelles (BE); DE TROY, Delphine, 1030 Schaerbeek (BE); BRENNER, Carmen, Bruxelles 1050 (BE); LEBRUN, Anne-Sophie, 1780 Wemmel (BE); TCHAMEKH, Bahia, 5530 Yvoir (BE); IONESCU, Alexandra, 1190 Forest (BE); HERTZOG, Laure, 68480 Werentzhouse (FR); LARUELLE, Pierre-Yves, 5190 Moustier-sur-Sambre (BE)
(74) Representative: De Clercq & Partners
(86) International application number: PCT/EP2019/075790
(87) International publication number: WO 2020/064791

(56) References cited:
- WO-A1-2009/087213
- WO-A1-2019/076591
- US-A1- 2003 139 410
- JENNIFER LEI ET AL: "Cell number and chondrogenesis in human mesenchymal stem cell aggregates is affected by the sulfation level of heparin used as a cell coating : Cell Number and Chondrogenesis in MSC Aggregates", JOURNAL OF BIOMEDICAL MATERIALS RESEARCH. PART A, vol. 104, no. 7, 28 March 2016 (2016-03-28), pages 1817-1829, XP055394286, HOBOKEN, NY, US ISSN: 1549-3296, DOI: 10.1002/jbm.a.35713
- LING LING ET AL: "Effect of heparin on the biological properties and molecular signature of human mesenchymal stem cells", GENE, ELSEVIER, AMSTERDAM, NL, vol. 576, no. 1, 17 October 2015 (2015-10-17), pages 292-303, XP029333434, ISSN: 0378-1119, DOI: 10.1016/J.GENE.2015.10.039
- SIMANN MEIKE ET AL: "Heparin affects human bone marrow stromal cell fate: Promoting osteogenic and reducing adipogenic differentiation and conversion", BONE, PERGAMON PRESS., OXFORD, GB, vol. 78, 7 May 2015 (2015-05-07), pages 102-113, XP029161033, ISSN: 8756-3282, DOI: 10.1016/J.BONE.2015.04.039

## Description

### FIELD OF THE INVENTION

The present invention is situated in the field of regenerative therapy, in particular in the field of bone cell therapy products administrable via minimally invasive techniques. In particular, the invention relates to methods for obtaining mesenchymal stem cell (MSC)-derived cells of chondro-osteoblastic lineage from MSC, to MSC-derived cells of chondro-osteoblastic lineage and cell populations, and to products comprising such cells and cell populations, methods and uses.

### BACKGROUND OF THE INVENTION

Transplantation of stem cells capable of undergoing osteogenic differentiation, of cells that are committed towards osteogenic differentiation or of cells with bone-forming ability is a promising avenue for the treatment of bone-related diseases, in particular when the treatment requires production of new bone tissues.

Mesenchymal stem cells (MSC) have been used previously to treat bone disorders (Gangji et al., 2005 Expert Opin Biol Ther 5: 437-42). However, although such relatively undifferentiated stem cells can be transplanted, they are not committed to an osteoblastic lineage and therefore a considerable proportion of so transplanted stem cells may not eventually contribute to the formation of the desired bone tissue. Moreover, the quantity of such stem cells is frequently unsatisfactory.

Manufacturing processes to generate bone-forming cells *ex vivo* without genetic modification of cells have been described in the art.

WO 2007/093431 concerns a method for *in vitro* expansion of isolated MSC, which yielded cells displaying an osteoblastic phenotype. In said method, human MSC were cultured in the presence of serum or plasma and basic fibroblast growth factor (FGF-2).

WO 2009/087213 concerns a method for obtaining osteoprogenitors, osteoblasts or osteoblast phenotype cells from human MSC *in vitro* or *ex vivo,* comprising contacting said MSC with human plasma or serum, FGF-2 and transforming growth factor beta (TGF-β).

However, major concerns regarding the development of an allogeneic bone-forming cell product remain, such as the production volume, i.e. number of doses issued from one bone marrow donation, availability and cost-effectiveness of the product. Furthermore, with the productivity of the manufacturing process, dozens of bone marrows will need to be qualified yearly to ensure manufacturing capacity. Therefore, there remains a need to increase productivity obtained from one bone marrow donation, and more generally for further and/or improved methods for obtaining MSC-derived cells and cell products useful in among others regenerative therapy.

### SUMMARY OF THE INVENTION

As corroborated by the experimental section, which illustrates certain representative embodiments of the invention, the inventors realized that the production volume of mesenchymal stem cells (MSC)-derived cells of chondro-osteoblastic lineage can be considerably improved when said cells are obtained by a manufacturing method comprising a tertiary culture (and hence addition of an intermediate cell passage "P2"), and controlling one or more settings of the culturing duration of secondary culture, the culturing duration of tertiary culture, addition of a cryopreservation step at the end of tertiary culture, or the plating density.

Hence, in an aspect, the invention provides a method for obtaining, such as differentiating and/or expanding, MSC-derived cells of chondro-osteoblastic lineage from MSC, the method comprising:
(a) culturing MSC recovered from a biological sample of a subject in a culture medium comprising fibroblast growth factor-2 (FGF-2), transforming growth factor beta (TGFβ) and heparin or a derivative or analogue thereof at a concentration of at least 0.01 IU/ml, thereby obtaining mesenchymal stem cell (MSC)-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are cultured in step (b) for a period of x days, wherein day x is the last day on which at least 20% of the MSC-derived cells are proliferating (e.g. in S phase, G2 phase or M phase of the cell cycle).

In a further aspect, the invention provides a method for obtaining MSC-derived cells of chondro-osteoblastic lineage from MSC, the method comprising:
(a) culturing MSC recovered from a biological sample of a subject in a culture medium comprising FGF-2, TGFβ and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are plated for the further culturing in step (b) at a density of 3×10² to 1×10³ cells/cm², preferably at a density of 3×10² to 8×10² cells/cm².

In a further aspect, the invention provides a method for obtaining MSC-derived cells of chondro-osteoblastic lineage from MSC, the method comprising:
(a) culturing MSC recovered from a biological sample of a subject in a culture medium comprising FGF-2, TGFP and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a);
(c) passaging the MSC-derived cells for a second time and further culturing the MSC-derived cells in a medium as defined in (a), thereby obtaining the MSC-derived cells of chondro-osteoblastic lineage;
(d) resuspending the MSC-derived cells of chondro-osteoblastic lineage in a cryopreservation medium suitable for administration to a subject; and
(e) cryopreserving the MSC-derived cells of chondro-osteoblastic lineage.

In a further aspect, the invention relates to a population of MSC-derived cells of chondro-osteoblastic lineage obtainable or obtained by the methods as defined herein.

In a further aspect, the invention provides a population of MSC-derived cells of chondro-osteoblastic lineage obtainable or obtained by *in vitro* or *ex vivo* expansion of MSC, whereby at least 90% of the MSC-derived cells of chondro-osteoblastic lineage in suspension have a diameter equal to or less than 25 µm (D₉₀ ≤ 25 µm) and wherein at most 1 % of the MSC-derived cells of chondro-osteoblastic lineage in suspension have a diameter of more than 35 µm.

In a further aspect, the invention provides a pharmaceutical formulation comprising the population of MSC-derived cells of chondro-osteoblastic lineage as defined herein.

In another aspect, the invention provides the population of MSC-derived cells of chondro-osteoblastic lineage as defined herein, or the pharmaceutical formulation as defined herein, for use as a medicament, preferably for use in the treatment of a subject in need of transplantation of cells of chondro-osteoblastic lineage.

The inventors found that the present methods can provide for one or more advantages as discussed below. The present methods allow to substantially increase culture yields and productivity of MSC-derived cells of chondro-osteoblastic lineage obtained from one bone marrow sample. As a result, one donation of bone marrow is sufficient to cover satisfying production volumes. Furthermore, the present methods increase the availability of MSC-derived cells of chondro-osteoblastic lineage for clinical use. Furthermore, the cryopreservation of the MSC-derived cells of chondro-osteoblastic lineage obtained by the present methods leads to a cell product which is directly administrable to a subject in need of transplantation of cells of chondro-osteoblastic lineage. The cryopreservation of the MSC-derived cells of chondro-osteoblastic lineage obtained by the present methods allows direct distribution and delivery of the cell product upon request, and hence immediately treatment of a subject in need of transplantation of cells of chondro-osteoblastic lineage. Further, cryopreservation of the MSC-derived cells of chondro-osteoblastic lineage obtained by the present methods allows to conduct all release tests of the cell product and obtain the results before administration of the cell product. In addition, the MSC-derived cells of chondro-osteoblastic lineage obtained by the methods as defined herein advantageously have both osteoinductive and osteogenic potential.

These and further aspects and preferred embodiments of the invention are described in the following sections and in the appended claims. The subject-matter of the appended claims is hereby specifically incorporated in this specification.

### DESCRIPTION OF THE DRAWINGS

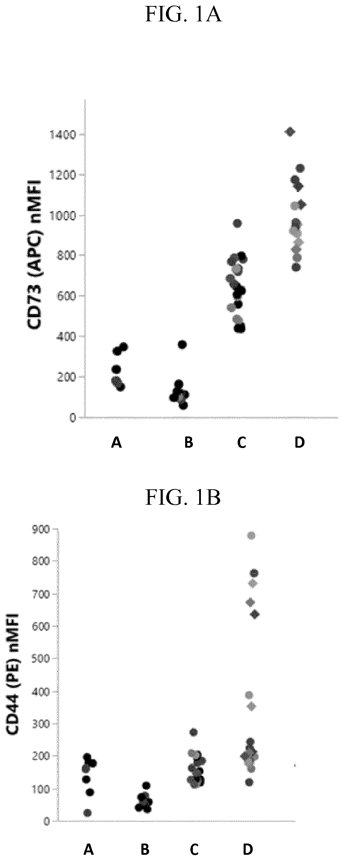

**FIG. 1** represents graphs illustrating CD73 (FIG. 1A) and CD44 (FIG. 1B) expression levels analysed by flow cytometry in A: MSC; B: cell product A; C: cell product B; D: cell product C - fresh (N=12, 6, 22, 15 (CD73) and N= 22, 8, 22, 18 (CD44) for MSC, cell product A, B and C respectively).

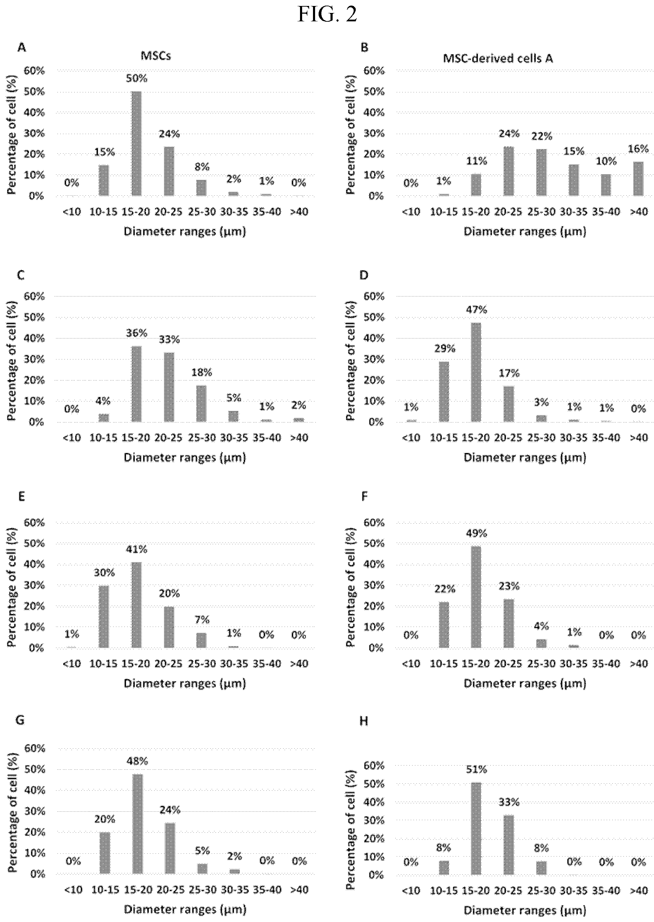

**FIG. 2** represents graphs illustrating the cell size of comparative cell products according to the prior art and cell products illustrating the invention: A: MSC; B: cell product A; C: cell product B; D: cell product C - fresh; E: cell product C - cryo CS10; F: cell product C - cryo CS10 diluted HSA 1:1; G: cell product C - cryo CS10 5%HSA; H: cell product C - cryo HTS 10%HSA 10%DMSO.

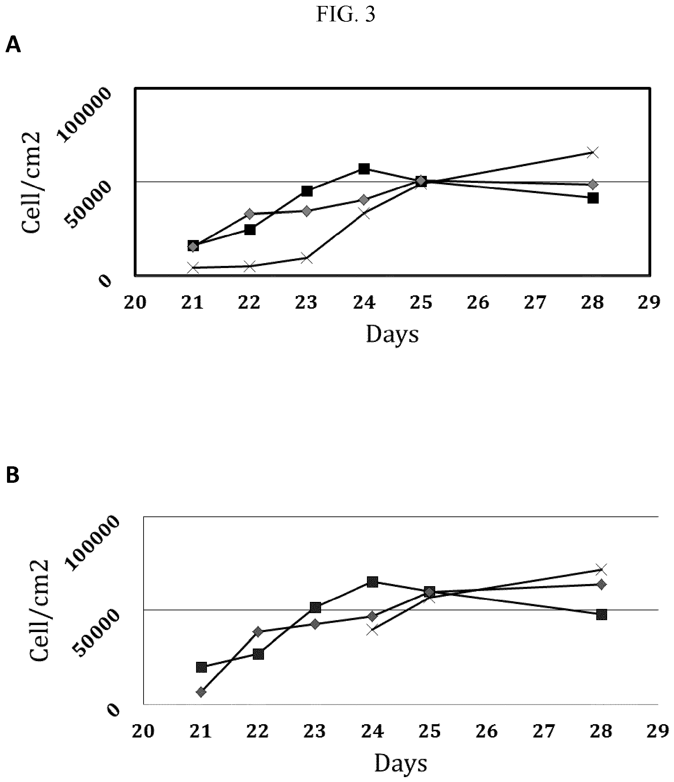

**FIG. 3** represents a graph illustrating the cell density according to the culture duration. The cells were counted: FIG. 3A: manually (Trypan Blue method with Bürker chamber) or FIG. 3B: by cytometry (BD Trucount^{™}).

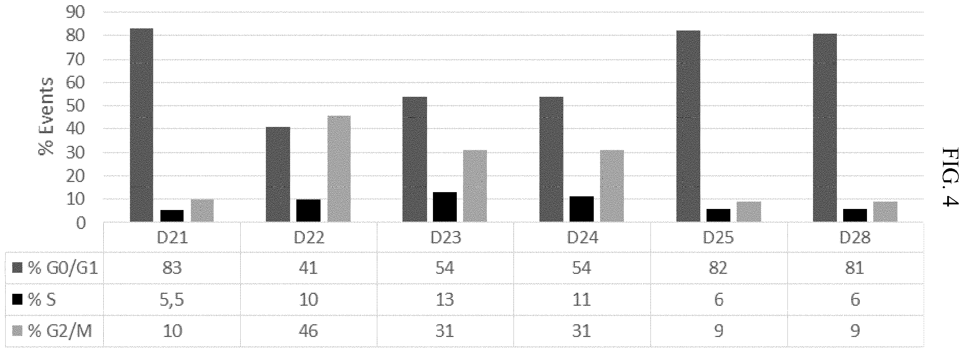

**FIG. 4** represents a graph illustrating cell cycle analysis with the events/phases (G0/G1, S and G2/M) of the cells in secondary culture at different culture durations. At D24, 42% of the cells are still proliferating (11% S and 31% G2/M) whereas from D25, the cells are mainly exited from the cell cycle.

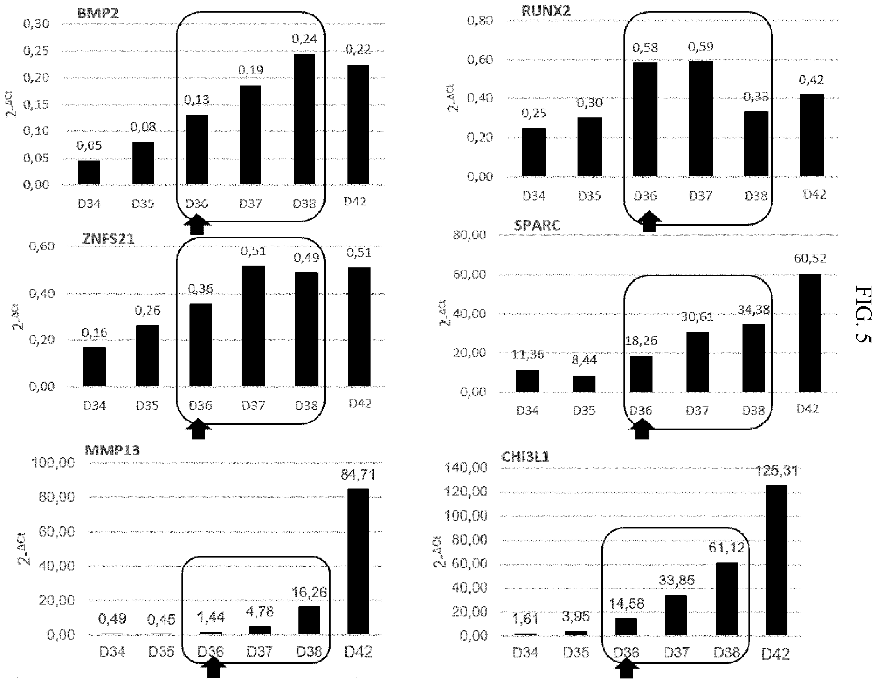

**FIG. 5** represents graphs illustrating the expression level of cell differentiation markers (BMP2, RUNX2, ZNFS21, SPARC, MMP13, CHI3L1) at different culture durations (N=8 for time points D34 to D38, and N=6 for D42).

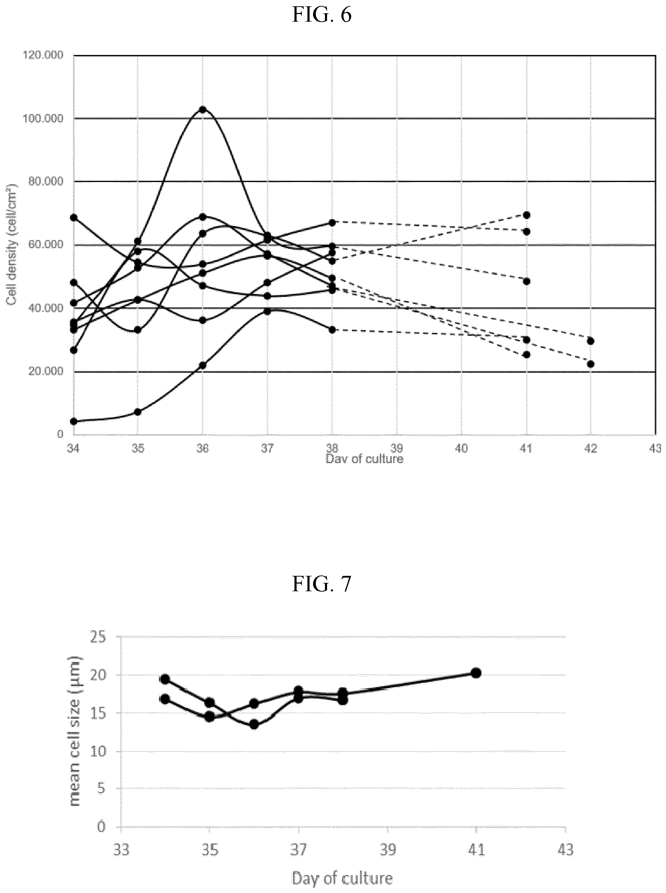

**FIG. 6** represents a graph illustrating the cell density of cell populations at different culture durations (N=8).
**FIG. 7** represents a graph illustrating the mean cell diameter size of two cell populations at different culture durations (N=2).

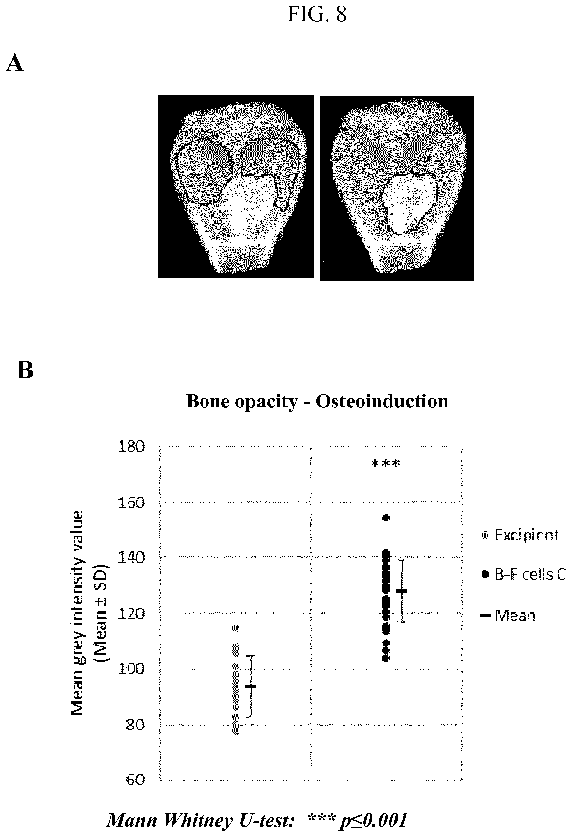

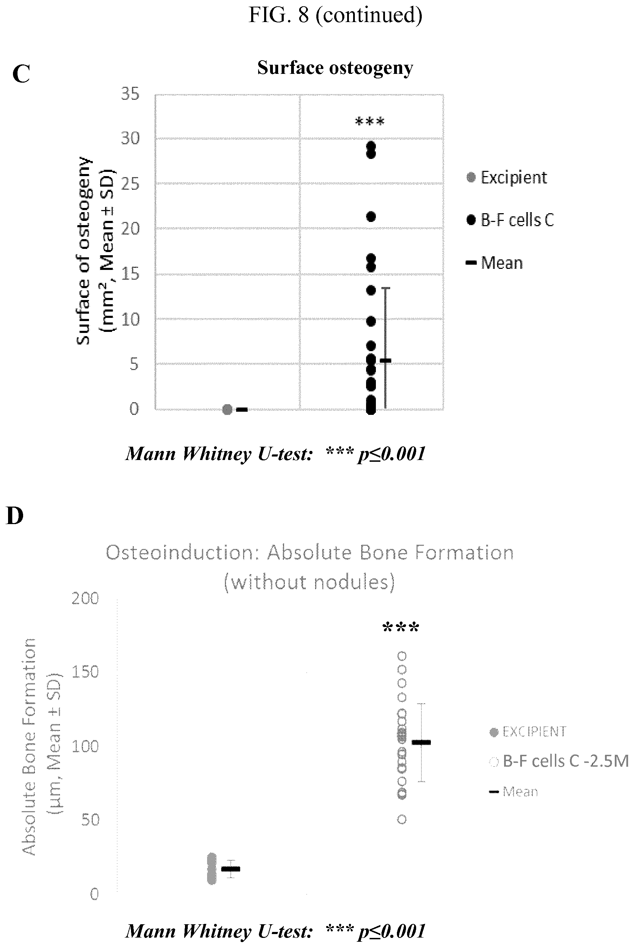

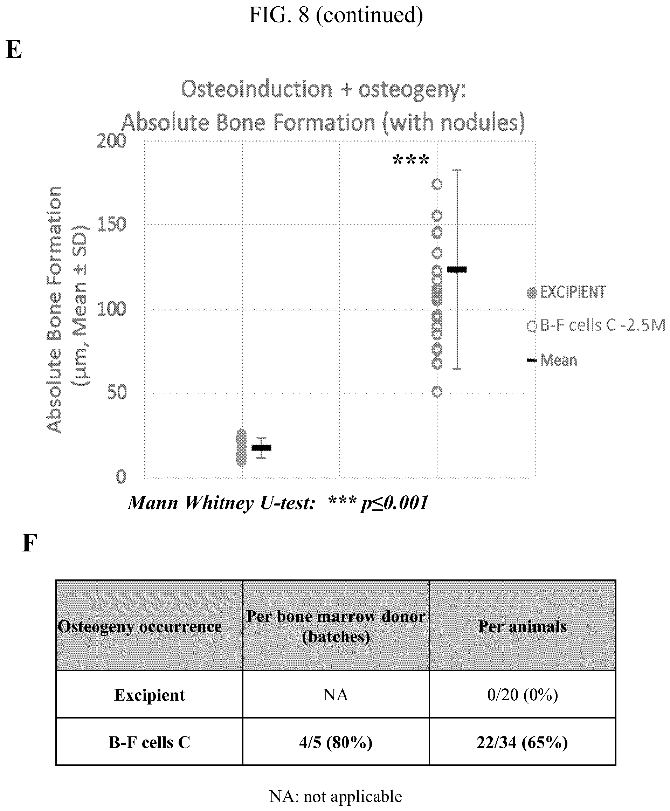

**FIG. 8** illustrates the osteoinduction and osteogeny assessed by X-ray analysis. A: Osteoinduction (A, left panel) is assessed by measuring the grey intensity value which is directly correlated to the bone opacity and therefore to the bone thickness. Osteogeny (A, right panel) is assessed by measuring the surface of the nodule that appears more refringent by X-ray imaging. **B**: The bone opacity is significantly higher for bone-forming cells C cryopreserved ("B-F cells C") compared to excipient (n=20 (excipient) and n=34 (B-F cells C from 5 different batches). **C:** The surface of osteogeny is significantly higher compared to excipient in which no mineralized nodules were observed (n=20 (excipient) and n=34 (B-F cells C from 5 different batches). **D-E:** Osteoinduction with (Fig. 8D) or without (FIG. 8E) osteogeny (represented by the absolute bone formation) is significantly higher for bone-forming cells C cryopreserved ("B-F cells C") compared to excipient. Mann Whitney U-test: ***p<0.001. **F:** in addition to osteoinduction activities, bone-forming cells C cryopreserved ("B-F cells C") promote a high osteogenic activity indicated by the presence of at least one mineralized nodule in 4/5 bone marrow donors (or batch production) and 65% of mice (n=20 (excipient) and n=34 (B-F cells C from 5 different batches).

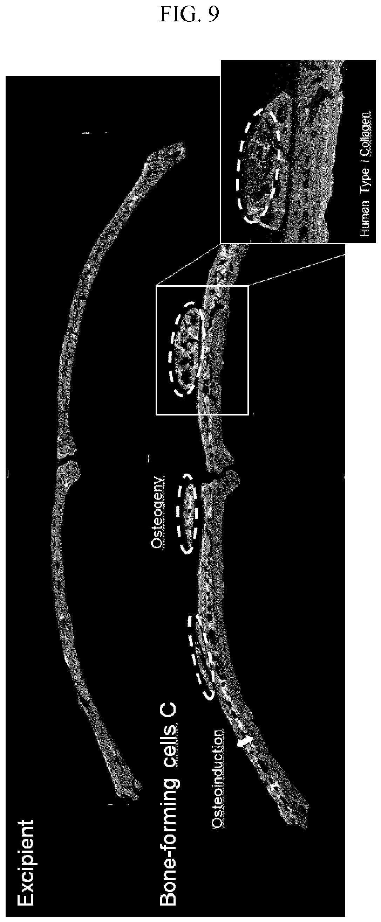

**FIG. 9** illustrates coronal histological section 4 weeks after a single administration of bone-forming cells C cryopreserved or excipient. Bone-forming cells C cryopreserved display activity through two mechanisms: (i) "osteoinduction": stimulation of host bone formation through paracrine secretion leading to intramembranous ossification and (ii) "osteogeny": promotion of "direct" bone formation (from donor/human origin) by endochondral ossification.

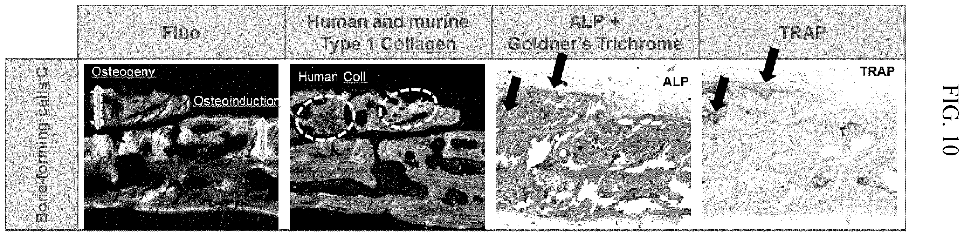

**FIG. 10** illustrates histology analysis of mice calvaria 4 weeks after having received a single injection of bone-forming cell C cryopreserved. Bone-forming cells C cryopreserved displayed osteoinduction and osteogenic properties ("fluo"). Human bone formation ("human type I collagen") was highlighted in mineralized nodules (osteogeny). Osteoblast ("ALP" indicated by black arrows in the 3^{th} panel) and osteoclast ("TRAP", indicated by black arrows in the 4^{th} panel) activities were mostly detected in mineralized nodules showing that the bone remodeling process in the nodules was still ongoing 4 weeks post-administration. No osteoid ("Goldner's Masson trichrome staining") was highlighted indicating that the bone formation process is completed.

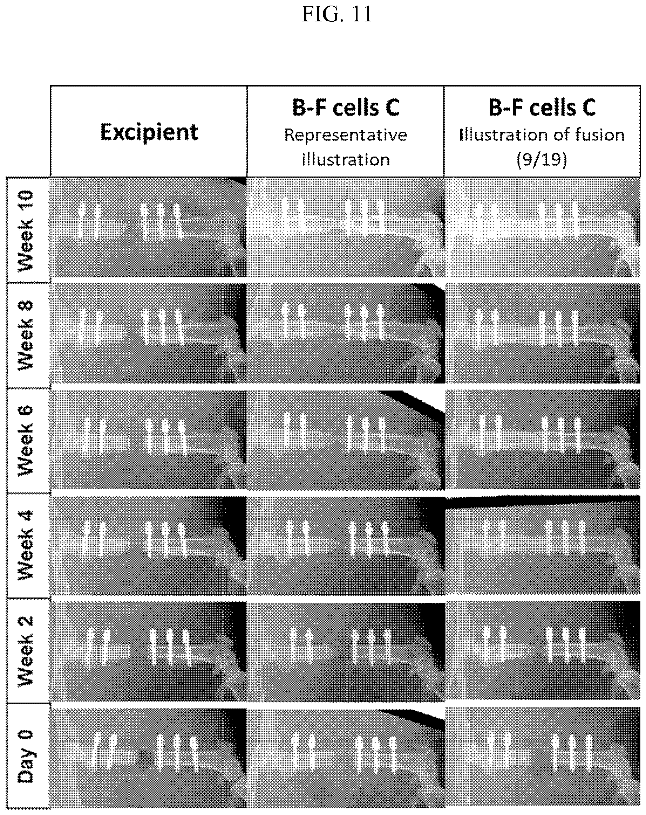

**FIG. 11** illustrates the effect of bone-forming cells C cryopreserved ("B-F cells C") in a segmental femoral sub-critical size defect model. X-ray images represent segmental femoral defects at Day 0 until Week 10 after administration of the excipient alone or bone-forming cells C cryopreserved.

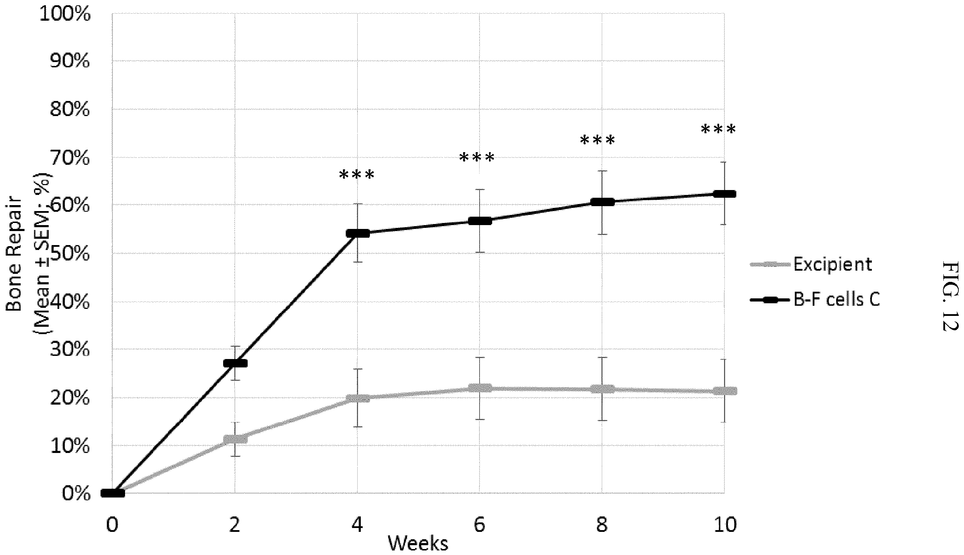

**FIG. 12** illustrates the effect of bone-forming cells C cryopreserved in a segmental femoral sub-critical size defect model (sub-CSD model). The graph represents the percentage of bone repair on X-ray images on the day of the surgical procedure/item administration ("W0") and over time up to 10 weeks ("W10") after administration of the excipient alone, or bone-forming cells C cryopreserved ("B-F cells C"); means ± SEM, *** p<0.001 (two-way repeated measures ANOVA).

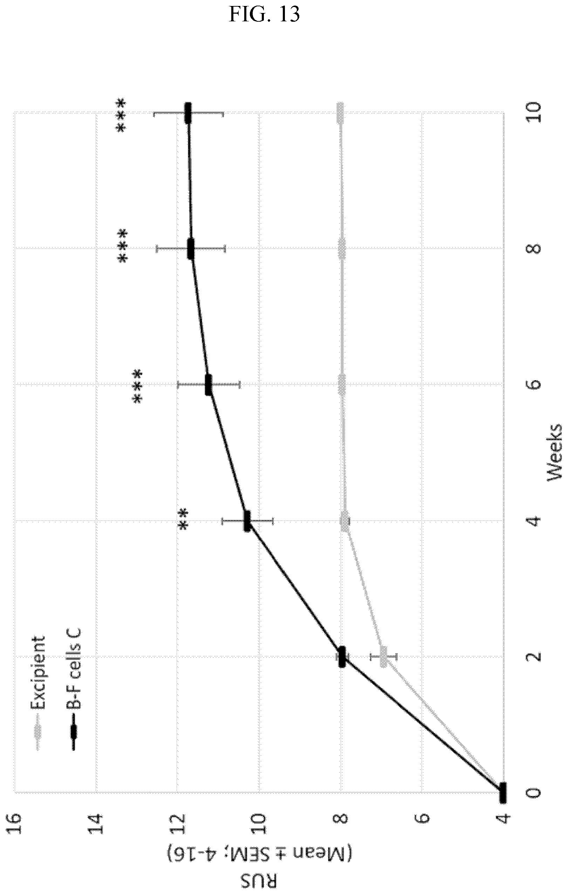

**FIG. 13** illustrates the effect of bone-forming cells C cryopreserved in a segmental femoral sub-critical size defect model (sub-CSD model). The graph represents the RUS score determined from X-ray images on the day of the surgical procedure/item administration ("W0") and over time up to 10 weeks ("W10") after administration of the excipient alone, or bone-forming cells C cryopreserved (B-F cells C); means ± SEM, ** p<0.01, *** p<0.001 (two-way repeated measures ANOVA).

### DETAILED DESCRIPTION OF THE PREFERRED EMBODIMENTS

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of", which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of ± 10% or less, preferably ± 5% or less, more preferably ± 1% or less, and still more preferably ± 0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g. any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e. also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In an aspect, the invention provides a method for obtaining MSC-derived cells of chondro-osteoblastic lineage from MSC, the method comprising:
(a) culturing MSC recovered from a biological sample of a subject in a culture medium comprising FGF-2, TGFP and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage.

The term "mesenchymal stem cell" or "MSC" as used herein refers to an adult, mesoderm-derived stem cell that is capable of generating cells of mesenchymal lineages, typically of two or more mesenchymal lineages, more typically three or more mesenchymal lineages, e.g. chondro-osteoblastic (cartilage and bone), osteoblastic (bone), chondroblastic (cartilage), myocytic (muscle), tenocytic (tendon), fibroblastic (connective tissue), adipocytic (fat) and stromogenic (marrow stroma) lineage. MSC may be isolated from a biological sample, preferably a biological sample of a human subject, e.g. bone marrow, trabecular bone, blood, umbilical cord, placenta, foetal yolk sac, skin (dermis), specifically foetal and adolescent skin, periosteum, dental pulp, tendon and adipose tissue.

The term "biological sample" or "sample" as used herein refers to a sample obtained from a biological source, e.g. from an organism, such as an animal or human subject, cell culture, tissue sample, etc. A biological sample of an animal or human subject refers to a sample removed from an animal or human subject and comprising cells thereof. The biological sample of an animal or human subject may comprise one or more tissue types and may comprise cells of one or more tissue types. Methods of obtaining biological samples of an animal or human subject are well known in the art, e.g. tissue biopsy or drawing blood. Human MSC, their isolation, *in vitro* expansion, and differentiation, have been described in, e.g. US Pat. No. 5,486,359; US Pat. No. 5,811,094; US Pat. No. 5,736,396; US Pat. No. 5,837,539; or US Pat. No. 5,827,740. Any MSC described in the art and isolated by any method described in the art may be suitable in the present method. In particular, MSC may be defined as displaying the capacity for *in vitro* trilineage mesenchymal differentiation into osteoblasts, adipocytes, and chondroblasts (Dominici et al., 2006, vol. 8, 315).

The term "MSC" also encompasses the progeny of MSC, e.g. progeny obtained by *in vitro* or *ex vivo* proliferation (propagation/expansion) of MSC obtained from a biological sample of an animal or human subject.

The term "stem cell" refers generally to an unspecialized or relatively less specialized and proliferation-competent cell, which is capable of self-renewal, i.e. can proliferate without differentiation, and which or the progeny of which can give rise to at least one relatively more specialized cell type. The term encompasses stem cells capable of substantially unlimited self-renewal, i.e. wherein the progeny of a stem cell or at least part thereof substantially retains the unspecialized or relatively less specialized phenotype, the differentiation potential, and the proliferation capacity of the mother stem cell, as well as stem cells which display limited self-renewal, i.e. wherein the capacity of the progeny or part thereof for further proliferation and/or differentiation is demonstrably reduced compared to the mother cell. By means of example and not limitation, a stem cell may give rise to descendants that can differentiate along one or more lineages to produce increasingly relatively more specialized cells, wherein such descendants and/or increasingly relatively more specialized cells may themselves be stem cells as defined herein, or even to produce terminally differentiated cells, i.e. fully specialized cells, which may be post-mitotic.

The term "adult stem cell" as used herein refers to a stem cell present in or obtained from (such as isolated from) an organism at the foetal stage or preferably after birth (e.g. particularly but without limitation for a human organism, at least one month of age after birth, e.g. at least 2 months, at least 3 months, e.g. at least 4 months, at least 5 months, e.g. at least 6 months of age after birth, such as, for example, 1 year or more, 5 years or more, at least 10 years or more, 15 years or more, 20 years or more, or 25 years or more of age after birth), such as for example after achieving adulthood. By means of example, adult stem cells can be obtained from human subjects which would otherwise be described in the conventional terms "infant", "child", "youth", "adolescent" or "adult".

Preferable MSC have the potential of generating cells of at least the chondro-osteoblastic lineage, such as cells of the osteoblastic lineage, such as chondro-osteoprogenitors and/or osteoprogenitors and/or pre-osteoblasts and/or osteoblasts and/or osteocytes, and/or of the chondroblastic lineage, such as chondro-osteoprogenitors and/or chondroprogenitors and/or pre-chondroblasts and/or chondroblasts and/or chondrocytes.

Further preferable MSC have the potential of generating cells of at least the osteoblastic (bone) lineage, such as, e.g. chondro-osteoprogenitors and/or osteoprogenitors and/or pre-osteoblasts and/or osteoblasts and/or osteocytes, etc.; or of at least the chondroblastic (cartilage) lineage, such as, e.g. chondro-osteoprogenitors and/or chondroprogenitors and/or pre-chondroblasts and/or chondroblasts and/or chondrocytes; fibroblastic (connective tissue) lineage, such as, e.g. fibroblasts, fibrocytes; or of at least synoviocytes (synovial fluid); or tenocytes etc.

Except when noted, "subject" or "patient" are used interchangeably and refer to animals, preferably vertebrates, more preferably mammals, and specifically includes human patients and non-human mammals. Preferred patients are human subjects. Animal subjects include prenatal forms of animals, such as, e.g. foetuses. Human subjects may include foetuses, and not embryos.

In certain embodiments of the methods, uses, or cell products as taught herein, the subject may be a human subject.

The term "cell product" as used herein refers to MSC-derived cells, MSC-derived cells of chondro-osteoblastic lineage as taught herein, a population of MSC-derived cells of chondro-osteoblastic lineage as taught herein, or a pharmaceutical formulation comprising MSC-derived cells of chondro-osteoblastic lineage as taught herein or a population of MSC-derived cells of chondro-osteoblastic lineage as taught herein, such as cell products suitable for administration (e.g. MSC-derived cells of chondro-osteoblastic lineage in a cryopreservation medium).

In an embodiment, MSC may be obtained from a healthy subject, which may help to ensure the functionality of MSC-derived cells obtained from said MSC.

In another embodiment, MSC are obtained from a human subject who is in need of transplantation of MSC-derived cells.

In certain embodiments of the methods, uses, or cell products as taught herein, the MSC or MSC-derived cells of chondro-osteoblastic lineage may be allogeneic to the subject to be treated. The terms "allogeneic" or "homologous" with reference to MSC or MSC-derived cells of chondro-osteoblastic lineage denotes that the MSC or MSC-derived cells of chondro-osteoblastic lineage are obtained from one or more (pooled) subjects other than the subject to be contacted or treated with the MSC-derived cells.

In certain embodiments of the methods, uses, or cell products as taught herein, the MSC or MSC-derived cells of chondro-osteoblastic lineage may be autologous to the subject to be treated. The term "autologous" with reference to MSC or MSC-derived cells of chondro-osteoblastic lineage denotes that the MSC or MSC-derived cells of chondro-osteoblastic lineage are obtained from the same subject to be contacted or treated with the MSC-derived cells of chondro-osteoblastic lineage.

In certain embodiments of the methods, uses, or cell products as taught herein, the MSC or MSC-derived cells of chondro-osteoblastic lineage may comprise a mixture of autologous and allogeneic (i.e. homologous) MSC or MSC-derived cells of chondro-osteoblastic lineage. Preferably, the MSC or MSC-derived cells of chondro-osteoblastic lineage are allogeneic to the subject to be treated.

The term "mesenchymal stem cell-derived cells" or "MSC-derived cells" as used herein refer to cells of mesenchymal lineage (e.g. chondro-osteoblastic (bone and cartilage), osteoblastic (bone), chondroblastic (cartilage), myocytic (muscle), tenocytic (tendon), fibroblastic (connective tissue), adipocytic (fat), or stromogenic (marrow stroma) lineage) obtained by differentiation of MSC, in particular obtained by *in vitro* (including *ex vivo*) differentiation of MSC.

Differentiation of MSC may involve culturing MSC under conditions capable of inducing the differentiation of MSC towards the desired cell type, more typically culturing MSC in a medium comprising one or more agents (e.g. growth factors) capable of inducing the differentiation of MSC towards the desired cell type. Protocols for differentiation of MSC are known *per se* (see, *inter alia,* WO 2007/093431; and further REGER, R.L. et al. 'Differentiation and Characterization of Human MSCs'. In: Mesenchymal Stem Cells: Methods and Protocols (Methods in Molecular Biology), Edited by D.J. Prockop et al. Humana Press, 2008, Vol. 449, p. 93-107; VERMURI, M.C. et al. (Eds.). Mesenchymal Stem Cell Assays and Applications (Methods in Molecular Biology). Humana Press, 2011, Vol. 698, especially pages 201 to 352).

The term "growth factor" as used herein refers to a biologically active substance which influences proliferation, growth, differentiation, survival and/or migration of various cell types, and may affect developmental, morphological and functional changes in an organism, either alone or when modulated by other substances. A growth factor may typically act by binding, as a ligand, to a receptor (e.g. surface or intracellular receptor) present in cells responsive to the growth factor. A growth factor herein may be particularly a proteinaceous entity comprising one or more polypeptide chains. By means of example and not limitation, the term "growth factor" encompasses the members of the fibroblast growth factor (FGF) family, bone morphogenetic protein (BMP) family, platelet-derived growth factor (PDGF) family, transforming growth factor beta (TGFβ) family, nerve growth factor (NGF) family, epidermal growth factor (EGF) family, insulin-like growth factor (IGF) family, growth differentiation factor (GDF) family, hepatocyte growth factor (HGF) family, hematopoietic growth factors (HeGFs), platelet-derived endothelial cell growth factor (PD-ECGF), angiopoietin, vascular endothelial growth factor (VEGF) family, glucocorticoids, and the like. The skilled person will understand that the growth factor or combination of growth factors may be any growth factor or combination of growth factors known of being capable of inducing differentiation of MSC towards a desired cell type. The skilled person will appreciate that *in vitro* methods for inducing differentiation of MSC towards a desired cell type (e.g. towards cells of chondro-osteoblastic lineage) may result in a substantially pure (i.e. composed primarily of) cell population of the desired cell type. Without limitation, so-derived cell population may contain at least 90% (by number) of the desired cell type, such as, e.g. ≥91%, ≥92%, ≥93%, ≥94%, ≥95%, ≥96%, ≥97%, ≥98%, ≥99%, or 100% of the desired cell type.

In particular embodiments, the MSC-derived cells are of chondro-osteoblastic lineage (cartilage and bone).

The recitation "MSC-derived cells of chondro-osteoblastic lineage" as used herein may refer to progenitor cells which have the ability to differentiate into cells of the osteoblastic lineage, such as chondro-osteoprogenitors, osteoprogenitors and/or pre-osteoblasts and/or osteoblasts and/or osteocytes, etc., or into cells of the chondroblastic lineage, such as chondro-osteoprogenitors, chondroprogenitors and/or pre-chondroblasts and/or chondroblasts and/or chondrocytes. The skilled person will understand that the progenitor cells will either differentiate into cells of the osteoblastic lineage (e.g. pre-osteoblasts or osteoblasts), or into cells of the chondroblastic lineage (e.g. pre-chondroblasts or chondroblasts) depending on conditions they are exposed to *in vitro* or *in vivo,* such as physical factors, and/or chemical or biological components, such as growth factors.

In certain embodiments, the recitation "cells of the osteoblastic lineage" or "MSC-derived cells of the osteoblastic lineage" may refer to cell types having an osteoblastic phenotype, and that can contribute to, or are capable of developing to cells which can contribute to, the formation of bone material or bone matrix, such as chondro-osteoprogenitors, osteoprogenitors, pre-osteoblasts, osteoblasts, or osteocytes, or mixtures thereof. As used herein, "osteoprogenitors" may particularly comprise early and late osteoprogenitors. Preferably, "cells of the osteoblastic lineage" or "MSC-derived cells of osteoblastic lineage" may equally refer to chondro-osteoprogenitors, osteoprogenitors, pre-osteoblasts, or osteoblasts, or mixtures thereof, yet more preferably the phrase may refer to chondro-osteoprogenitors or pre-osteoblasts or osteoblasts, or mixtures thereof, such as in certain examples the phrase may refer to pre-osteoblasts, or in certain other examples the phrase may refer to osteoblasts. All these terms are well-known *per se.*

By means of further guidance and not limitation, osteoprogenitors, pre-osteoblasts and osteoblasts, as well as cell populations comprising osteoprogenitors pre-osteoblasts and/or osteoblasts may display the following characteristics:
a) the cells comprise expression of Runt-related transcription factor 2 (Runx2), a multifunctional transcription factor that regulates osteoblast differentiation and the expression of many extracellular matrix protein genes during osteoblast differentiation;
b) the cells comprise expression of at least one of the following: alkaline phosphatase (ALP), more specifically ALP of the bone-liver-kidney type; and more preferably also comprise expression of one or more additional bone markers such as osteocalcin (OCN, BGLAP), procollagen type 1 amino-terminal propeptide (P1NP), osteonectin (ON, SPARC), osteopontin (OPST, SPP1, OPN) and/or bone sialoprotein (BSP), and/or one or more additional bone matrix proteins such as decorin and/or osteoprotegerin (OPG);
c) the cells substantially do not express CD45 (e.g. less than about 10%, preferably less than about 5%, more preferably less than about 2% of the cells may express CD45);
d) the cells show evidence of ability to mineralize the external surroundings, or synthesize calcium-containing extracellular matrix (e.g. when exposed to osteogenic medium; see Jaiswal et al. J Cell Biochem, 1997, vol. 64, 295-312). Calcium accumulation inside cells and deposition into matrix proteins can be conventionally measured for example by culturing in ⁴⁵Ca²⁺, washing and re-culturing, and then determining any radioactivity present inside the cell or deposited into the extracellular matrix (US 5,972,703), or using an alizarin red-based mineralization assay (see, e.g. Gregory et al. Analytical Biochemistry, 2004, vol. 329, 77-84);
e) the cells substantially do not differentiate towards neither of cells of adipocytic lineage (e.g. adipocytes) or chondroblastic lineage (e.g. chondroblasts, chondrocytes). The absence of differentiation towards such cell lineages may be tested using standard differentiation inducing conditions established in the art (e.g. see Pittenger et al. Science, 1999, vol. 284, 143-7), and assaying methods (e.g. when induced, adipocytes typically stain with oil red O showing lipid accumulation; chondrocytes typically stain with alcian blue or safranin-orange). Substantially lacking propensity towards adipogenic and/or chondrogenic differentiation may typically mean that less than 20%, or less than 10%, or less than 5%, or less than 1% of the tested cells would show signs of adipogenic or chondrogenic differentiation when applied to the respective test.

In certain embodiments, the recitations "cells of the chondroblastic (cartilage) lineage" or "MSC-derived cells of the chondroblastic (cartilage) lineage" may refer to cell types having a chondroblastic phenotype, and that can contribute to, or are capable of developing to cells which can contribute to, the formation of cartilage or cartilaginous matrix. As used herein, "chondroprogenitors" may particularly comprise early and late chondroprogenitors. Preferably, "cells of the chondroblastic (cartilage) lineage" or "MSC-derived cells of the chondroblastic (cartilage) lineage" may refer to chondro-osteoprogenitors, chondroprogenitors, pre-chondroblasts, or chondroblasts, or mixtures thereof, yet more preferably the phrase may refer to pre-chondroblasts or chondroblasts, or mixtures thereof, such as in certain examples the phrase may refer to pre-chondroblasts, or in certain other examples the phrase may refer to chondroblasts. All these terms are well-known *per se.*

By means of further guidance and not limitation, cells of chondro-osteoblastic and/or chondroblastic lineage, such as chondro-osteoprogenitors, chondroprogenitors, pre-chondroblasts and chondroblasts, as well as cell populations comprising chondro-osteoprogenitors, chondroprogenitors, pre-chondroblasts and/or chondroblasts may display the following characteristics:
a) the cells comprise expression of SOX9, a transcription factor that plays a central role during chondroblast differentiation and cartilage formation;
b) the cells comprise expression of at least one of the following: aggrecan (ACAN), type-II collagen, or CD90;
c) the cells substantially do not express CD45 (e.g. less than about 10%, preferably less than about 5%, more preferably less than about 2% of the cells may express CD45);
d) the cells show evidence of ability to produce high level of collagen types II, IX, and XI and proteoglycans, the main constituents of the hyaline extracellular matrix (ECM) *in situ.* Cartilage formation can be conventionally measured for example by using a safranin-orange/fast green assay to stain proteoglycans and non-collagenous protein, respectively (see, e.g. Lee et al. Tissue Engineering, 2011, vol. 18, 484-98);
e) human articular chondrocytes may display cell expression characteristics as summarised in Diaz-Romero et al. 2005 (J Cell Physiol, vol. 202(3), 731-42), e.g. they may express integrins and other adhesion molecules (CD49a, CD49b, CD49c, CD49e, CD49f, CD51/61, CD54, CD106, CD166, CD58, CD44), tetraspanins (CD9, CD63, CD81, CD82, CD151), receptors (CD105, CD119, CD130, CD140a, CD221, CD95, CD120a, CD71, CD14), ectoenzymes (CD10, CD26), and other surface molecules (CD90, CD99). During monolayer culture, chondrocytes may up-regulate certain markers regarded as distinctive for mesenchymal stem cells (CD10, CD90, CD105, CD166). Such markers may thus also be expressed by the less mature pre-chondroblasts or chondroblasts.
f) the cells substantially do not differentiate towards neither of cells of adipocytic lineage (e.g. adipocytes) or osteoblastic lineage (e.g. osteoblasts, osteocytes). The absence of differentiation towards such cell lineages may be tested using standard differentiation inducing conditions established in the art (e.g. see Pittenger et al. Science, 1999, vol. 284, 143-7), and assaying methods (e.g. when induced, adipocytes typically stain with oil red O showing lipid accumulation; pre-osteoblasts and osteoblasts typically stain for ALP). Substantially lacking propensity towards adipogenic and/or osteoblastic differentiation may typically mean that less than 20%, or less than 10%, or less than 5%, or less than 1% of the tested cells would show signs of adipogenic or osteoblastic differentiation when applied to the respective test.

In certain embodiments, the MSC-derived cells of chondro-osteoblastic lineage may have osteogenic properties.

The terms "osteogenic properties", "osteogenic potential" or "osteogenic activity" as used herein refer to the ability of cells to (trans)differentiate into bone-matrix-secreting cells or to the ability of cells to secrete bone matrix (i.e. without the need of a (trans)differentiation step), *in vivo,* and optionally *in vitro.* The term encompasses the ability of cells to form bone tissue by intramembranous ossification or endochondral ossification. The ability of the cells to form bone tissue by intramembranous ossification typically represents the ability of the cells to form bone tissue without the need of a calcified cartilage matrix as a template. The ability of the cells to form bone tissue by endochondral ossification typically represents the ability of the cells to form bone tissue by first forming a calcified cartilage matrix and subsequently using said calcified cartilage matrix as a template for bone tissue formation. The term does not encompass the osteoinductive potential of cells.

For instance, cell potency of MSC-derived cells of chondro-osteoblastic lineage can be determined by measuring osteogenic activity of such cells. The osteogenic activity of human MSC-derived cells of chondro-osteoblastic lineage can be measured *in vivo* for example by determining the presence of at least one mineralized nodule (e.g. of human or mixed human-murine origin) after administration of the cells to mice by subcutaneous injection over the calvaria. The osteogenic activity of human MSC-derived cells of chondro-osteoblastic lineage can be measured *in vivo* for example by evaluating the thickness of newly mineralized nodules (e.g. of human or mixed human-murine origin) after administration of the cells to mice by subcutaneous injection over the calvaria, or by evaluating the degree of bone repair in a mouse model of femoral segmental sub-critical size defect (sub-CSD).

For instance, human MSC-derived cells of chondro-osteoblastic lineage, such as of 2.5 × 10⁶ cells formulated in 100 µl excipient, can be administered to nude mice by a single subcutaneous administration over the calvaria bone. To label bone neo-formation over time, calcium-binding fluorochromes such as alizarin red (red), calcein (green), calcein (blue) and tetracycline (yellow) can be sequentially administered to mice intraperitoneal injection 3 days before and 4, 8, and 12 days after cell administration of the MSC-derived cells of chondro-osteoblastic, respectively. Mice can be euthanized 2 weeks after cell administration and the calvaria of each mouse can be harvested to assess bone formation properties by histomorphometry (e.g. quantification of bone formation). The initial and final thicknesses of the calvaria can be used to calculate the percentage of bone neo-formation following administration of the cells. Furthermore, bone formation properties can also be assessed by immunofluorescence (e.g. murine or human origin of the bone formation). Osteoblastic activity can be assessed on calvaria sections using ALP enzymatic activity detection method. Osteoclastic activity can be assessed on calvaria sections using TRAP enzymatic activity detection methods. The status of mineralization of the neo-formed bone can be assessed using Masson Trichrome Goldner staining on the calvaria sections stained with ALP for instance using commercially available kits (e.g. Bio-Optica^{®}). Cartilage formation can be assessed using safranin-orange staining on calvaria sagittal paraffin sections.

In a further example, human MSC-derived cells of chondro-osteoblastic lineage, such as of 1.25 × 10⁶ cells formulated in 50 µl excipient, can be administered to mice locally at the site of the bone defect by percutaneous injection one day after they were subjected to the femoral segmental sub-critical size defect. Bone repair can be quantified by X-ray imaging. The bone defect size can be quantified by measuring the distance between the two edges of the bone defect.

In certain embodiments, the MSC-derived cells of chondro-osteoblastic lineage may have osteoinductive properties.

The terms "osteoinductive properties", "osteoinductive potential" or "osteoinductive activity" as used herein refers to the ability of cells to attract other bone-matrix-secreting cells and/or to induce the (trans)differentiation of other cells into bone-matrix-secreting cells.

For instance, cell potency of MSC-derived cells of chondro-osteoblastic lineage can be determined by measuring osteoinductive activity of such cells. The ability of MSC-derived cells of chondro-osteoblastic lineage to induce bone formation can be measured *in vivo* for example by evaluating the thickness of newly mineralized bone after administration of the cells to mice by subcutaneous injection over the calvaria or by evaluating the bone repair in a mouse model of femoral segmental sub-critical size defect (sub-CSD). The ability of MSC-derived cells of chondro-osteoblastic lineage to induce bone formation can also be measured for example through the alkaline phosphatase (ALP) activity assessment by an ALP substrate staining.

In certain embodiments, the MSC-derived cells of chondro-osteoblastic lineage may have both osteoinductive and osteogenic properties. Advantageously, the MSC-derived cells of chondro-osteoblastic lineage as taught herein, upon transplantation into a subject in need thereof, allow bone neo-formation which exceeds bone neo-formation as compared to transplantation with MSCs or MSC-derived cells obtained by prior art methods.

By means of example but without limitation, suitable cell surface markers to evaluate cell identity of MSC-derived cells of chondro-osteoblastic lineage may include CD73, CD105, CD10, and CD44. These cell surface markers can for instance be detected by commercially available monoclonal antibodies, such as fluorochrome-labelled monoclonal antibodies allowing for cell detection by flow cytometry. In particular, CD73 and CD105 are mesenchymal markers; CD44 is an adhesion marker; and CD10 is an osteochondroblastic marker which are typically expressed by a high fraction of MSC-derived cells of chondro-osteoblastic lineage. The quantity of CD73 on the cell surface of MSC-derived cells of chondro-osteoblastic lineage is typically high; the quantity of CD105 on the cell surface of MSC-derived cells of chondro-osteoblastic lineage is typically low; and the quantity of CD44 on the cell surface of MSC-derived cells of chondro-osteoblastic lineage is typically high.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD44 and CD10.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are negative for CD34.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD44 and CD10; and substantially all (e.g. at least 90% (by number), such as, e.g. ≥91%, ≥92%, >_93%, >_94%, >_95%, >_96%, >_97%, >_98%, ≥99%, or 100%) MSC-derived cells of chondro-osteoblastic lineage are negative for CD34.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are negative for CD45, CD34 and CD3.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD44 and CD10; and substantially all (e.g. at least 90% (by number), such as, e.g. ≥91%, ≥92%, >_93%, >_94%, >_95%, >_96%, >_97%, >_98%, ≥99%, or 100%) MSC-derived cells of chondro-osteoblastic lineage are negative for CD45, CD34 and CD3.

In particular embodiments, the MSC-derived cells of chondro-osteoblastic lineage have any one or more of a normalized Median of Fluorescence Intensity (nMFI) for CD73 (nMFI_{CD73}) of at least 500, a nMFI for CD44 (nMFI_{CD44}) of at least 100 or a nMFI for CD105 (nMFI_{CD105}) of at most 150. For instance, the MSC-derived cells of chondro-osteoblastic lineage have any one or more of a nMFI_{CD73} of at least 550, at least 600, at least 650, at least 700, at least 750, at least 800, at least 850 or at least 900; a nMFI_{CD44} of at least 110, at least 120, at least 130, at least 140, at least 150, at least 200, at least 250, at least 300 or at least 350; or a nMFI_{CD105} of at most 180, at most 170, at most 160, at most 150, at most 140, at most 130, at most 120, at most 110 or at most 100. Preferably, the MSC-derived cells of chondro-osteoblastic lineage have a nMFI_{CD73} of at least 500, a nMFI_{CD44} of at least 100, and a nMFI_{CD105} of at most 150.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD10 and CD44 (i.e. express CD73, CD105, CD10 and CD44 on the cell surface), and the MSC-derived cells of chondro-osteoblastic lineage have any one or more of a nMFI_{CD73} of at least 500, a nMFI_{CD44} of at least 100 or a nMFI_{CD105} of at most 150. For instance, substantially all (e.g. at least 90% (by number), such as, e.g. ≥91%, ≥92%, ≥93%, ≥94%, ≥95%, ≥96%, ≥97%, ≥98%, ≥99%, or 100%) MSC-derived cells of chondro-osteoblastic lineage are positive for CD73, CD105, CD10 and CD44 (i.e. express CD73, CD105, CD10 and CD44 on the cell surface), and the MSC-derived cells of chondro-osteoblastic lineage have any one or more of a nMFI_{CD73} of at least 550, at least 600, at least 650, at least 700, at least 750, at least 800, at least 850 or at least 900; a nMFI_{CD44} of at least 110, at least 120, at least 130, at least 140, at least 150, at least 200, at least 250, at least 300 or at least 350; and a nMFI_{CD105} of at most 180, at most 170, at most 160, at most 150, at most 140, at most 130, at most 120, at most 110 or at most 100. Preferably, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD10 and CD44 (i.e. express CD73, CD105, CD10 and CD44 on the cell surface), and the MSC-derived cells of chondro-osteoblastic lineage have a nMFI_{CD73} of at least 500, a nMFI_{CD44} of at least 100, and a nMFI_{CD105} of at most 150.

The "normalized Median of Fluorescence Intensity" or "nMFI" as used herein refers to the ratio of the MFI of the whole analyzed cell population labeled with one or more fluorochrome-conjugated antibodies (MFI_{marker_channel}) to the MFI of the cell population labeled with one or more fluorochrome-conjugated isotype control antibodies (MFI_{isotype_channel}), such as immunoglobulin G (IgG) control conjugated with a fluorochrome such as fluorescein isothiocyanate (FITC), allophycocyanin (APC) or phycoerythrin (PE). nMFI results are proportional to the quantity of markers present on cell surface of a population of interest. The (n)MFI is typically linked to the wavelength at which the emission of the fluorescent signal is measured.

The recitations "a nMFI for CD73" or "nMFI_{CD73}" as used herein refer to the ratio of the MFI of the whole analyzed cell population labeled with an APC-conjugated antibody against CD73 (e.g. BD Biosciences^{®}, Cat N°:560847) to the MFI of the cell population labeled with IgG control conjugated with APC (e.g. BD Biosciences^{®}, Cat N°: 555751). Preferably, the nMFI_{CD73} is measured with an excitation wavelength of 633 nm and an emission wavelength of 660 nm for APC.

The recitations "a nMFI for CD44" or "nMFI_{CD44}" as used herein refer to the ratio of the MFI of the whole analyzed cell population labeled with PE-conjugated antibody against CD44 (e.g. BD Biosciences^{®}, Cat N°: 550989) to the MFI of the cell population labeled with IgG control conjugated with PE (e.g. BD Biosciences^{®}, CatN°: 556650). Preferably, the nMFI_{CD44} is measured with an excitation wavelength of 488 nm and an emission wavelength of 580 nm for PE.

The recitations "a nMFI for CD105" or "nMFI_{CD105}" as used herein refer to the ratio of the MFI of the whole analyzed cell population labeled with APC-conjugated antibodies against CD105 (e.g. BD Biosciences^{®}, Cat N°: 562408) to the MFI of the cell population labeled with IgG control conjugated with APC (e.g. BD Biosciences^{®}, Cat N°: 555751). Preferably, the nMFI_{CD105} is measured with an excitation wavelength of 633 nm and an emission wavelength of 660 nm for APC.

The recitations "a nMFI for CD10" or "nMFI_{CD10}" as used herein refer to the ratio of the MFI of the whole analyzed cell population labeled with PE-conjugated antibody against CD10 (e.g., BD Biosciences^{®}, Cat N°: 555375) to the MFI of the cell population labeled with IgG control conjugated with PE (e.g., BD Biosciences^{®}, Cat N°: 556650). Preferably, the nMFI_{CD10} is measured with an excitation wavelength of 488 nm and an emission wavelength of 580 nm for PE.

As described earlier, the above detailed methods can yield MSC-derived cells of chondro-osteoblastic lineage, or populations thereof, with superior characteristics, such as in particular (i) high expression of ALP, which represents the cell's commitment towards the chondro-osteoblastic or osteoblastic lineage, and (ii) low HLA-DR expression, which represents the limited immunogenicity of the MSC-derived cells of osteochondroblastic or osteoblastic lineage, indicating that the cells are more suitable for cell transplantation, for instance to allogeneic subjects.

Accordingly, in particular embodiments, at least 70% (by number) (e.g. at least 75% (by number), such as, e.g. ≥80%, ≥85%, ≥90%, ≥95%, ≥96%, ≥97%, ≥98%, ≥99%, or 100%) of the MSC-derived cells of chondro-osteoblastic lineage are positive for alkaline phosphatase (ALP); and less than 10% (by number) (e.g. less than 5% (by number), such as, e.g. less than 3%, less than 2%, or less than 1%) of the MSC-derived cells of chondro-osteoblastic lineage are positive for HLA-DR.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD10 and CD44; substantially all (e.g. at least 90% (by number), such as, e.g. ≥91%, ≥92%, >_93%, >_94%, >_95%, >_96%, >_97%, >_98%, >_99%, or 100%) MSC-derived cells of chondro-osteoblastic lineage are negative for CD34; at least 70% (e.g. at least 75% (by number), such as, e.g. ≥80%, ≥85%, ≥90%, >_95%, >_96%, >_97%, >_98%, >_99%, or 100%) of the MSC-derived cells of chondro-osteoblastic lineage are positive for alkaline phosphatase (ALP); and less than 10% (e.g. less than 5% (by number), such as, e.g. less than 3%, less than 2%, or less than 1%) of the MSC-derived cells of chondro-osteoblastic lineage are positive for HLA-DR.

In particular embodiments, substantially all (e.g. at least 90% (by number), such as, e.g. ≥91%, ≥92%, ≥93%, >_94%, >_95%, ≥96%, >_97%, >_98%, >_99%, or 100%) MSC-derived cells of chondro-osteoblastic lineage are positive for CD73, CD105, CD10 and CD44; substantially all (e.g. at least 90% (by number), such as, e.g. ≥91%, ≥92%, >_93%, >_94%, >_95%, >_96%, >_97%, >_98%, >_99%, or 100%) MSC-derived cells of chondro-osteoblastic lineage are negative for CD45, CD34 and CD3; at least 70% (e.g. at least 75% (by number), such as, e.g. ≥80%, ≥85%, ≥90%, >_95%, >_96%, >_97%, ≥98%, ≥99%, or 100%) of the MSC-derived cells of chondro-osteoblastic lineage are positive for alkaline phosphatase (ALP); and less than 10% (e.g. less than 5% (by number), such as, e.g. less than 3%, less than 2%, or less than 1%) of the MSC-derived cells of chondro-osteoblastic lineage are positive for HLA-DR.

Wherein a cell is said to be positive for (or to express or comprise expression of) a particular marker, this means that a skilled person will conclude the presence or evidence of a distinct signal, e.g. antibody-detectable or detection by reverse transcription polymerase chain reaction, for that marker when carrying out the appropriate measurement, compared to suitable controls. Where the method allows for quantitative assessment of the marker, positive cells may on average generate a signal that is significantly different from the control, e.g. but without limitation, at least 1.5-fold higher than such signal generated by control cells, e.g. at least 2-fold, at least 4-fold, at least 10-fold, at least 20-fold, at least 30-fold, at least 40-fold, at least 50-fold higher or even higher.

The expression of the above cell-specific markers can be detected using any suitable immunological technique known in the art, such as immunohitochemistry or affinity adsorption, Western blot analysis, flow cytometry, ELISA, etc., or by any suitable biochemical assay of enzyme activity (e.g. for ALP), or by any suitable technique of measuring the quantity of the marker mRNA, e.g. Northern blot, semi-quantitative or quantitative RT-PCR, etc. Sequence data for markers listed in this disclosure are known and can be obtained from public databases such as GenBank (http://www.ncbi.nlm.nih.gov/).

In certain embodiments, the MSC-derived cells of chondro-osteoblastic lineage may be animal cells, preferably warm-blooded animal cells, more preferably mammalian cells, such as human cells or non-human mammalian cells, and most preferably human cells.

The term *"in vitro"* as used herein is to denote outside, or external to, animal or human body. The term *"in vitro"* as used herein should be understood to include *"ex vivo".* The term *"ex vivo"* typically refers to tissues or cells removed from an animal or human body and maintained or propagated outside the body, e.g. in a culture vessel.

MSC-derived cells of chondro-osteoblastic lineage as intended herein are preferably adherent, i.e. require a surface for growth, and typically grow as an adherent monolayer on said surface (i.e. adherent cell culture), rather than as free-floating cells in a culture medium (suspension culture). Adhesion of cells to a surface, such as the surface of a tissue culture plastic vessel, can be readily examined by visual inspection under inverted microscope. Cells grown in adherent culture require periodic passaging, wherein the cells may be removed from the surface enzymatically (e.g. using trypsin), suspended in growth medium, and re-plated into new culture vessel(s). In general, a surface or substrate which allows adherence of cells thereto may be any substantially hydrophilic substrate. As known in the art, tissue culture vessels, e.g. culture flasks, well plates, dishes, or the like, may be usually made of a large variety of polymeric materials, suitably surface treated or coated after moulding in order to provide for hydrophilic substrate surfaces. The term "contacting" as used herein means bringing together, either directly or indirectly, one or more molecules, components or materials with another, thereby facilitating interactions there between. Typically, one or more agents capable of inducing expansion and/or differentiation of MSC or MSC-derived cells may be contacted with MSC or MSC-derived cells by means of their inclusion in the media, in which the MSC or MSC-derived cells are cultured.

In certain embodiments, the methods as taught herein may comprise (a) culturing MSC recovered from a biological sample of a subject in a culture medium comprising FGF-2, TGFP and heparin or a derivative or analogue thereof at a concentration of at least 0.01 IU/ml, thereby obtaining MSC-derived cells. Step (a) may also be referred to herein as "primary culture".

The term "fibroblast growth factor 2 (FGF-2)", "basic FGF", "FGF-b", "FGFB", "BFGF", "heparin-binding growth factor 2 (HBGF-2)", or "prostatropin", can be used interchangeably and refers to so-known member of the fibroblast growth factor family. The inventors have realised that FGF-2 is particularly effective in the method of the present invention.

The term "transforming growth factor beta (TGFP)", "TGFB" or "TGFbeta" as used herein refers to a member of the transforming growth factor beta (TGFP) family. The inventors have realized that TGFP is particularly effective in the method of the present invention. In a further embodiment, the said member of the TGFβ family is chosen from the group consisting of TGF-beta-1, TGF-beta-2, TGF-beta-3, TGF-beta-4, GDF1 (Growth differentiation factor 1), GDF-2, GDF-3, GDF-5, GDF-6, GDF-7, GDF-8, GDF-9, GDF-11, GDF-15, INHA (inhibin alpha chain), INHBA (inhibin beta A chain), INHBB (inhibin beta B chain), INHBC (inhibin beta C chain), INHBE (inhibin beta E chain), MIS (Muellerian-inhibiting factor), and further of members of GDNF subfamily, including GDNF (glial cell line-derived neurotrophic factor), NRTN (neurturin), PSPN (persephin), and mixtures thereof.

In certain embodiments of the methods, uses, or cell products as taught herein, TGFβ may be selected from the group consisting of TGFβ1, TGFβ2, TGFβ3, and mixtures thereof. Preferably, TGFβ is TGFβ1.

In a further embodiment, MSC or MSC-derived cells may be - in addition to FGF-2 and TGFβ - contacted with one or more additional, exogenously added growth factors other than FGF-2 and TGFβ. In another embodiment, FGF-2 and TGFβ may be the sole exogenous growth factors with which the MSC or MSC-derived cells are contacted.

In a preferred embodiment, the growth factor used in the present method is a human growth factor. As used herein, the term "human growth factor" refers to a growth factor substantially the same as a naturally occurring human growth factor. For example, where the growth factor is a proteinaceous entity, the constituent peptide(s) or polypeptide(s) thereof may have primary amino acid sequence identical to a naturally occurring human growth factor. The use of human growth factors in the present method is preferred, as such growth factors are expected to elicit a desirable effect on cellular function.

The term "naturally occurring" is used to describe an object or entity that can be found in nature as distinct from being artificially produced by man. For example, a polypeptide sequence present in an organism, which can be isolated from a source in nature and which has not been intentionally modified by man in the laboratory, is naturally occurring. When referring to a particular entity, e.g. to a polypeptide or protein, the term encompasses all forms and variants thereof which occur in nature, e.g. due to a normal variation between individuals. For example, when referring to a proteinaceous growth factor, the term "naturally occurring" encompasses growth factors having differences in the primary sequence of their constituent peptide(s) or polypeptide(s) due to normal allelic variation between individuals.

The present method may employ a biologically active variant or fragment of a growth factor. In the method of the invention, "biologically active" variants or fragment of a growth factor achieve at least about the same degree of obtaining MSC-derived cells of chondro-osteoblastic lineage as taught herein from MSCs as the respective growth factor, when other conditions are substantially the same.

A "variant" of a polypeptide has an amino acid sequence which is substantially identical (i.e. largely but not wholly identical) to the amino acid sequence of the polypeptide. Herein, "substantially identical" refers to at least 85% identical, e.g. at least 90% identical, preferably at least 95% identical, e.g. least 99% identical. Sequence differences may result from insertion (addition), deletion and/or substitution of one of more amino acids.

In another embodiment, the growth factors used in the present method, namely at least FGF-2 and TGFP, may be non-human animal growth factors, and particularly non-human mammal growth factors, or biologically active variants or derivatives thereof. As used herein, the terms "non-human animal growth factor" and "non-human mammal growth factor" refer to a growth factor substantially the same as, respectively, a naturally occurring non-human animal or non-human mammal growth factor. For example, where the growth factor is a proteinaceous entity, the constituent peptide(s) or polypeptide(s) thereof may have primary amino acid sequence identical to a naturally occurring non-human animal or non-human mammal growth factor. A skilled person will understand that non-human animal or non-human mammal growth factors may be applicable in the present method, albeit to a lesser extent than human animal growth factors, since the latter are of the same origin as the MSC cells. In particular, non-human animal or non-human mammal growth factors may be used if they elicit the desired effect, e.g. an effect similar to an (analogous) human growth factor.

In a preferred embodiment, the growth factors or a biologically active variants or derivatives thereof are recombinant, i.e. produced by a host organism through the expression of a recombinant nucleic acid molecule, which has been introduced into the host organism or an ancestor thereof, and which comprises a sequence encoding said polypeptide. The term "recombinant nucleic acid molecule" as used herein refers to a nucleic acid molecule (e.g. a DNA or cDNA molecule) which is comprised of segments joined together using recombinant DNA technology.

In certain embodiments of the methods, uses, or cell products as taught herein, the MSC or MSC-derived cells are additionally contacted with, such as wherein the medium additionally comprises one or more of, plasma, serum or a substitute thereof.

The term "plasma" is as conventionally defined and comprises fresh plasma, thawed frozen plasma, solvent/detergent-treated plasma, processed plasma (e.g. PRP), or a mixture of any two or more thereof. Plasma is usually obtained from a sample of whole blood, provided or contacted with an anticoagulant, (e.g. heparin (at very low concentrations, typically about 15 ×10⁻⁵ IU/ml, citrate, oxalate or EDTA). Subsequently, cellular components of the blood sample are separated from the liquid component (plasma) by an appropriate technique, typically by centrifugation. By means of a specific example but not limitation, to obtain plasma suitable for use in the present invention, a blood sample may be drawn into a vacutainer tube containing the anticoagulant EDTA (ethylenediaminetetraacetic acid) (e.g. BD Vacutainer plastic EDTA tube, 10 ml, 1.8 mg/mL). The sample is gently shaken and then centrifuged during 10 min at room temperature at 1,000-2,000 g to separate the plasma from red blood cells. The supernatant (plasma) is collected, optionally pooled (if a plurality of blood samples is used), and aliquoted into cryovials, which are stored at - 80°C until use. The term "plasma" refers to a composition which does not form part of a human or animal body. The term "plasma" may in certain embodiments specifically include processed plasma, i.e. plasma subjected after its separation from whole blood to one or more processing steps which alter its composition, specifically its chemical, biochemical, or cellular composition. Accordingly, the term "plasma" as intended herein may include platelet-rich plasma (PRP), i.e. plasma that has been enriched with platelets. Typically, PRP may contain about 1.0×10⁶ platelets/µl, whereas platelet concentration in whole blood may be about 1.5×10⁵ to 3.5×10⁵/µL.

Plasma may be solvent/detergent-treated. The terms "solvent/detergent-treated plasma", "S/D-treated plasma", or "S/D plasma" generally refer to decellularized plasma obtainable or obtained by a method comprising the steps of: (a) treating plasma with a solvent and a detergent and (b) filtering the solvent/detergent-treated plasma. Solvents suitable for such treatment are solvents such as di- or trialkylphosphates and detergents which are described in US 4,764,369. The detergent used for preparing S/D plasma preferably is a non-toxic detergent (e.g. Tween^{®} 20 or Tween^{®} 80).

The term "serum" is as conventionally defined and comprises fresh serum, thawed frozen serum or serum prepared from plasma, or a mixture of any two or more thereof. Serum can be usually obtained from a sample of whole blood by first allowing clotting to take place in the sample and subsequently separating the so formed clot and cellular components of the blood sample from the liquid component (serum) by an appropriate technique, typically by centrifugation. Clotting can be facilitated by an inert catalyst, e.g. glass beads or powder. Alternatively, serum can be obtained from plasma by removing the anticoagulant and fibrin. By means of a specific example but not limitation, to obtain serum suitable for use in the present invention, a blood sample may be drawn into a vacutainer tube containing no anticoagulant (e.g. BD Vacutainer Plus plastic serum tube, 10 ml) and incubated for 30 to 45 min at room temperature to allow clotting. The tube is then centrifuged for 15 min at room temperature at 1,000-2,000 g to separate the serum from red blood cells. The supernatant (serum) is collected, optionally pooled (if a plurality of blood samples is used) and aliquoted into cryovials which are stored at -80°C until use. The term "serum" hence refers to an acellular composition which does not form part of a human or animal body. The serum as intended herein is human serum, i.e. obtained from a single human subject or from a plurality of human subjects (e.g. serum mixed pool). The serum may be unprocessed serum, i.e. serum derived by separation from whole blood and not subjected to downstream processing steps which alter its chemical, biochemical, or cellular composition, other than optional heat inactivation, storage (cryogenic or non-cryogenic), sterilisation, freeze-drying and/or filtration. In certain embodiments, the serum may be obtained from solvent/detergent-treated plasma.

The isolated plasma, serum or substitute thereof can be used directly in the method of the present invention. They can also be appropriately stored for later use (e.g. for shorter time periods, e.g. up to about 1-2 weeks, at a temperature above the respective freezing points of plasma, serum or substitute thereof, but below ambient temperature, this temperature will usually be about 4°C to 5°C; or for longer times by freeze storage, usually at between about -70°C and about -80°C).

The isolated plasma, serum or substitute thereof can be heat inactivated as known in the art, particularly to remove the complement. Where the present method employs plasma, serum, or substitute thereof autologous to the cells cultured in the presence thereof, it may be unnecessary to heat inactivate the plasma, serum or substitute thereof. Where the plasma, serum or substitute thereof is at least partly allogeneic to the cultured cells, it may be advantageous to heat inactivate the plasma, serum or substitute thereof. Optionally, the plasma, serum or substitute thereof may also be sterilized prior to storage or use, using conventional microbiological filters, preferably with pore size of 0.2 µm or smaller.

In an embodiment, the present method may employ human plasma, serum or substitute thereof which is autologous to human MSC or MSC-derived cells contacted therewith. The term "autologous" with reference to plasma, serum or substitute thereof denotes that the plasma, serum or substitute thereof is obtained from the same subject as are MSC or MSC-derived cells to be contacted with the said plasma, serum or substitute thereof. The use of autologous plasma, serum or substitute thereof may ensure optimal acceptance of the cells by the subject and/or avoid accidental transmission of infectious agents from, e.g. other sera.

In another embodiment, the method may employ human plasma, serum or substitute thereof which is "homologous" or "allogeneic" to human MSC or MSC-derived cells contacted therewith, i.e. obtained from one or more (pooled) human subjects other than the subject from which the MSC are obtained.

In a further embodiment, the method may employ a mixture of autologous and allogeneic (i.e. homologous) plasma, sera or substitute thereof as defined above. The phrase "substitute of serum or plasma" as used herein, refers to a natural or artificial non-toxic composition having one or more of the functions of plasma and/or serum, such as compositions capable of inducing growth and/or expansion of MSC or MSC-derived cells. Non-limiting examples of substitutes of serum or plasma include platelet lysate and compositions for cell culture comprising one or more fractionated components of plasma or serum, such as human serum albumin. A skilled person appreciates that human plasma, serum and substitutes thereof are complex biological compositions, which may comprise one or more growth factors, cytokines or hormones.

It is intended that growth factors FGF-2 and TGFP or their respective biologically active variants or derivatives are provided in addition to, i.e. exogenously to or in supplement to, one or more of plasma, serum or a substitute thereof.

The term "heparin" as used herein refers to a polymer of the glycosaminoglycan family of carbohydrates with a molecular weight ranging from 3 to 30 kDa characterized by its anticoagulating effects. The potency of heparin or a derivative or analogue thereof may be determined *in vitro* by a biological assay wherein the concentration of heparin necessary to prevent the clotting of sheep or goat or human plasma is compared to the concentration of an internationally accepted reference standard an international accepted reference standard based on units of heparin activity per milligram. One mg of heparin is typically equal to 140 - 180 international units (IU).

The term "IU" or "international units" is a standard measure of the quantity of a biological substance expressed as the biological activity or effect of said biological substance. For every substance to which this unit is assigned, there is an internationally accepted biological activity or effect expected with a dose of 1 IU when tested according to an internationally accepted biological procedure.

In certain embodiments, the heparin or heparin derivative or analogue thereof may be selected from the group consisting of unfractionated heparin (UFH); low molecular weight heparin (LMWH), such as enoxaparin, dalteparin, nadroparin, tinzaparin, certoparin, reviparin, ardeparin, parnaparin, bemiparin, or mixtures thereof; a heparinoid, such as heparan sulfate, dermatan sulfate, chondroitin sulfate, acharan sulfate, keratan sulfate, or mixtures thereof, such as danaparoid; a heparin salt; a heparinoid salt; a heparin fragment; a heparinoid fragment; and mixtures thereof. Preferably, the heparin or heparin derivative or analogue is selected from the group consisting of UFH, dalteparin, danaparoide and heparan sulfate.

In particular embodiments, said FGF-2, said TGFβ, said heparin or a derivative or analogue thereof, and optionally one or more of plasma, serum or substitute thereof, are included in a medium, commonly a liquid cell culture medium. Typically, the medium will comprise a basal medium formulation as known in the art. Many basal media formulations (available, e.g. from the American Type Culture Collection, ATCC; or from Invitrogen, Carlsbad, California) can be used to culture the cells herein, including but not limited to Eagle's Minimum Essential Medium (MEM), Dulbecco's Modified Eagle's Medium (DMEM), alpha modified Minimum Essential Medium (alpha-MEM), Basal Medium Essential (BME), BGJb, F-12 Nutrient Mixture (Ham), Iscove's Modified Dulbecco's Medium (IMDM), or X-VIVO^{™} serum free medium (clinical grade), available from Invitrogen or Cambrex (New Jersey), and modifications and/or combinations thereof. Compositions of the above basal media are generally known in the art and it is within the skill of one in the art to modify or modulate concentrations of media and/or media supplements as necessary for the cells cultured. Such basal media formulations contain ingredients necessary for mammal cell development, which are known *per se.* By means of illustration and not limitation, these ingredients may include inorganic salts (in particular salts containing Na, K, Mg, Ca, Cl, P and possibly Cu, Fe, Se and Zn), physiological buffers (e.g. HEPES, bicarbonate), nucleotides, nucleosides and/or nucleic acid bases, ribose, deoxyribose, amino acids, vitamins, antioxidants (e.g. glutathione) and sources of carbon (e.g. glucose, sodium pyruvate, sodium acetate), etc.

For use in culture, basal media can be supplied with one or more further components. For example, additional supplements can be used to supply the cells with the necessary trace elements and substances for optimal growth and expansion. Such supplements include insulin, transferrin, selenium salts, and combinations thereof. These components can be included in a salt solution such as, but not limited to, Hanks' Balanced Salt Solution (HBSS), Earle's Salt Solution. Further antioxidant supplements may be added, e.g. β-mercaptoethanol. While many basal media already contain amino acids, some amino acids may be supplemented later, e.g. L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin. Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

In particular embodiments, one or more of human plasma, serum or a substitute thereof may be comprised in said media at a proportion (volume of one or more of plasma, serum, or a substitute thereof / volume of medium) between about 0.5% and about 30%, preferably between about 1% and about 15%, more preferably between 2% and 10%. The present methods may perform satisfactorily with relatively low amounts of one or more of plasma, serum or a substitute thereof, e.g. about 5 or 10 volume % or below, e.g. about 1, about 2, about 3 or about 4 volume %, allowing to decrease the volume of one or more of plasma, serum or a substitute thereof that needs to be obtained in order to culture the MSC or MSC-derived cells.

In yet further embodiments, one or more of concentrated plasma products (e.g. plasma concentrates such as concentrates from frozen plasma), concentrated serum products or products of a concentrated substitute of plasma or serum may be employed. Such concentrated products may be included in the composition at a concentration lower than the desired concentration of one or more of plasma, serum or a substitute thereof, such as to offset (counterbalance, compensate for) the concentration factor.

In particular embodiments, combinations or mixtures of any two or more of human plasma, serum and/or a substitute thereof may be used.

In particular embodiments, FGF-2 and TGFP are comprised in said medium at concentrations sufficient to induce differentiation towards a desired cell-type.

In particular embodiments, FGF-2 and TGFP are comprised in said medium at concentrations sufficient to induce differentiation of MSC into MSC-derived cells of a chondro-osteoblastic lineage. Typically, FGF-2 or a biologically active variant or fragment thereof can be included in the media at a concentration of between 0.1 and 100 ng/ml, preferably between 0.5 and 20 ng/ml, e.g. at about 19, 18, 17, 16, 15, 14, 13, 12, 11, 10, 9, 8, 7 or 6 ng/ml, or at about 5 ng/ml or less, e.g. at about 4, 3, 2, 1 or 0.5 ng/ml. Typically, TGFP, such as TGFβ1 or a biologically active variant or fragments thereof can be included in the media at a concentration of between 0.1 and 100 ng/ml, preferably between 0.25 and 20 ng/ml, e.g. at about 19, 18, 17, 16, 15, 14, 13, 12,11, 10, 9, 8, 7 or 6 ng/ml, or at about 5 ng/ml or less, e.g. at about 4, 3, 2, 1 or 0.5 ng/ml. Said values are intended to refer to concentrations of the respective growth factors or a biologically active variants or fragments thereof, as exogenously supplemented to the media.

In particular embodiments, heparin or a derivative or analogue thereof is comprised in said medium at a concentration of at least 0.01 IU/ml, at least 0.02 IU/ml, at least 0.03 IU/ml, at least 0.04 IU/ml, at least 0.05 IU/ml, at least 0.06 IU/ml, at least 0.07 IU/ml, at least 0.08 IU/ml, at least 0.09 IU/ml, at least 0.1 IU/ml, at least 0.5 IU/ml, at least 1 IU/ml, at least 5 IU/ml, at least 10 IU/ml, at least 20 IU/ml, at least 30 IU/ml, at least 40 IU/ml, at least 50 IU/ml, at least 60 IU/ml, at least 70 IU/ml, at least 80 IU/ml, at least 90 IU/ml, or at least 100 IU/ml. In particular embodiments, heparin or a derivative or analogue thereof is comprised in said medium at a concentration of at least 0.10 IU/ml. In certain preferred embodiments, heparin or a derivative or analogue thereof is comprised in said medium at a concentration of about 0.1 IU/ml. In certain embodiments, heparin or a derivative or analogue thereof may be comprised in said medium at a concentration of about 0.10 IU/ml, 0.20 IU/ml, 0.30 IU/ml, 0.40 IU/ml, 0.50 IU/ml, 0.60 IU/ml, 0.70 IU/ml, 0.80 IU/ml, 0.90 IU/ml or 1.0 IU/ml.

In certain embodiments of the methods or uses as taught herein, the concentration of heparin or derivative or analogue thereof may be at least 0.05 IU/ml, preferably about 0.1 IU/ml.

In an embodiment, the above concentrations may refer to the total concentration of growth factors or biologically active variants or fragments thereof or of said heparin or derivative or analogue thereof in the medium, i.e. to the sum concentration of said growth factors or biologically active variants or fragments thereof or of said heparin or derivative or analogue thereof as contributed by the plasma, serum or substitute thereof and as provided in addition thereto.

In certain embodiments, the above concentrations may refer to the concentration of said growth factors or biologically active variants or fragments thereof or of said heparin or derivative or analogue thereof as provided in addition to that already contributed by the plasma or serum. Understandably, if the growth factors or heparin or derivative or analogue thereof to-be-added is normally not present (not detectable) in the plasma, serum or substitute thereof, the total and added concentration of the growth factors or heparin or derivative or analogue thereof will be (substantially) the same.

In a preferred embodiment, MSC recovered from a biological sample of a subject as defined elsewhere herein are cultured in a culture vessel. The culture vessel may provide for a plastic surface to enable cell adherence. In another embodiment, the surface may be a glass surface. In yet another embodiment, the surface may be coated with an appropriate material enable adherence and growth of cells, e.g. Matrigel^{®}, laminin or collagen.

In particular embodiments, the MSC may be recovered from bone marrow (or other sources) by selecting those (mononuclear) cells which can adhere to a substrate surface, e.g. plastic surface.

In certain embodiments, the method as taught herein may comprise (e.g. as part of step (a)) removing non-adherent matter and further culturing adherent cells in the medium as defined in (a).

In particular embodiments, cells may be allowed to attach for about 1 and 8 days, more typically between about 2 and 6 days, more typically about 4 days before removing the non-adherent matter. Otherwise, removing the non-adherent matter may be performed at most 8 days, at most 6 days, at most 4 days, preferably at most 4 days, after initiating step (a).

In certain embodiments, the method as taught herein may comprise (e.g. as part of step (a)) replacing part of or all of the culture medium by a medium as defined in (a). Advantageously, this allows to renew the growth factors. In certain embodiments, the replacement may be performed at least once, such as once, twice or three times, during primary culture.

In certain embodiments of the methods, uses, or cell products as taught herein, the MSC-derived cells may be cultured in step (a) for a period of from about 13 days to about 15 days. Preferably, the MSC-derived cells are cultured in step (a) for a period of about 14 days.

In certain embodiments, the methods as taught herein may comprise (b) passaging the MSC-derived cells for a first time ("passage 1" or "P1") and further culturing the MSC-derived cells in a medium as defined in (a). Step (b) may also be referred to herein as "secondary culture".

In an embodiment, step (b) may comprise collecting the MSC-derived cells obtained in step (a).

In an embodiment, step (b) may comprise detaching, replating and further culturing the MSC-derived cells in a medium as defined in (a), i.e. a medium comprising FGF-2, TGFP and heparin or a derivative or analogue thereof, preferably at a concentration of at least 0.01 IU/ml.

In certain embodiments of the methods, uses, or cell products as taught herein, the MSC derived-cells are cultured in step (b) for a period of from about 8 days to about 12 days. In certain embodiments, the MSC derived-cells are cultured in step (b) for a period of from about 9 days to about 11 days. Preferably, MSC derived-cells are cultured in step (b) for a period of about 10 days.

In certain embodiments, the MSC-derived cells may be cultured in step (b) for a period of x days, wherein day x is the last day on which at least 20% of the MSC-derived cells are proliferating.

Hence, an aspect relates to a method for obtaining mesenchymal stem cell (MSC)-derived cells of chondro-osteoblastic lineage from MSC, the method comprising:
(a) culturing MSC recovered from a biological sample of a subject in a culture medium comprising FGF-2, TGFβ, and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are cultured in step (b) for a period of x days, wherein day x is the last day on which at least 20% of the MSC-derived cells are proliferating.

In certain embodiments, the MSC-derived cells may be cultured in step (b) for a period of x days, wherein day x is the last day on which at least 25% of the MSC-derived cells are proliferating (e.g. in S and G2/M phases). For instance, the MSC-derived cells may be cultured in step (b) for a period of x days, wherein day x is the last day on which at least 30%, at least 35%, at least 40%, at least 45, at least 50%, at least 55, or at least 60% of the MSC-derived cells are proliferating (e.g. in S and G2/M phases).

In certain embodiments of the methods, uses, or cell products as taught herein, the proliferating MSC-derived cells are in S phase, G2 phase or M phase of the cell cycle. The cell cycle analysis with the events/phases (G0/G1, S and G2/M phases) may be performed by flow cytometry such as by fluorescence-activated cell sorting (FACS).

In certain embodiments, the MSC-derived cells may be cultured in step (b) for a period of x days, wherein day x is the last day on which the number of MSC-derived cells is higher than at day x-1. For instance, in order to determine day x as the last day on which the number of MSC-derived cells is higher than at day x-1, the MSC-derived cells may be cultured during secondary culture for a period of 28 days (according to prior art methods), samples may be harvested every day, and the number of cells or cell density (e.g. expressed as # cells/cm²) may be determined for each sample, for instance by counting manually (e.g. Bürker chamber) or by flow cytometry (e.g. BD Trucount^{™}). Based on the cell numbers, one can determine the last day (i.e. day x) on which the number of MSC-derived cells is higher than at the previous day (i.e. day x-1).

In certain embodiments, the MSC-derived cells may be cultured in step (b) for a period of x days, wherein day x is the last day on which at least 20% of the MSC-derived cells are proliferating, and the number of MSC-derived cells is higher than at day x-1.

In certain embodiments, the MSC-derived cells may be plated for the further culturing in step (b) at a density of 3×10² to 1×10³ cells/cm². In certain embodiments, the MSC-derived cells may be plated for the further culturing in step (b) at a density of 5×10² to 1×10³ cells/cm². Preferably, the MSC-derived cells may be plated for the further culturing in step (b) at a density of 3×10² to 8×10² cells/cm².

Accordingly, in a further aspect, the invention provides a method for obtaining MSC-derived cells of chondro-osteoblastic lineage from MSC, the method comprising:
(a) culturing MSC recovered from a biological sample of a subject in a culture medium comprising FGF-2, TGFβ and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are plated for the further culturing in step (b) at a density of 3×10² to 1×10³ cells/cm², preferably at a density of 3×10² to 8×10² cells/cm².

The terms "density" and "cell density" may be used interchangeably herein and refer to the number of cells per unit surface (e.g. expressed as cells/cm²).

In certain embodiments, the methods as taught herein may comprise step (c) passaging the MSC-derived cells for a second time ("passage 2" or "P2") and further culturing the MSC-derived cells in a medium as defined in (a), thereby obtaining the MSC-derived cells of chondro-osteoblastic lineage. Step (c) may also be referred to herein as "tertiary culture".

The MSC-derived cells of chondro-osteoblastic lineage obtained in step (c) may be considered "passage 3" or "P3" cells.

In certain embodiments of the methods, uses, or cell products as taught herein, the MSC-derived cells are cultured in step (c) for a period of from about 10 days to about 14 days. In certain embodiments, the MSC derived-cells are cultured in step (c) for a period of from about 11 days to about 13 days. Preferably, the MSC derived-cells are cultured in step (c) for a period of about 12 days.

In certain embodiments of the methods, uses, or cell products as taught herein, the MSC-derived cells may be plated for the further culturing in step (c) at a density of 3×10² to 1×10³ cells/cm². In certain embodiments, the MSC-derived cells may be plated for the further culturing in step (c) at a density of 5×10² to 1×10³ cells/cm². Preferably, the MSC-derived cells may be plated for the further culturing in step (c) at a density of 3×10² to 8×10² cells/cm².

Accordingly, in a further aspect, the invention provides a method for obtaining MSC-derived cells of chondro-osteoblastic lineage from MSC, the method comprising:
(a) culturing MSC recovered from a biological sample of a subject in a culture medium comprising FGF-2, TGFP and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are plated for the further culturing in step (c) at a density of 3×10² to 1×10³ cells/cm², preferably at a density of 3×10² to 8×10² cells/cm².

The skilled person will understand that the methods as taught herein may comprise further passages. The skilled person will understand that this may generate cell cultures of passage 4 (P4), passage 5 (P5), passage 6 (P6), passage 7 (P7), passage 8 (P8), passage 9 (P9) or passage 10 (P10). Passage 0 (P0) may refer to MSC or MSC-derived cells which have not been detached and/or replated.

In certain embodiments, the methods as taught herein may comprise cryopreserving the MSC-derived cells of chondro-osteoblastic lineage. In certain embodiments, the methods as taught herein may comprise (d) resuspending the MSC-derived cells of chondro-osteoblastic lineage in a cryopreservation medium suitable for administration to a subject. By resuspending the MSC-derived cells of chondro-osteoblastic lineage in a cryopreservation medium suitable for administration to a subject, a cell product suitable for direct administration to a subject can be obtained. The cell product can conveniently be stored by cryopreservation, the cryopreserved cell product can easily be transported, and delivered upon request. Therefore, the cell product suitable for administration is immediately available for treatment upon request. Furthermore, cryopreservation of the cell product obtained by the present methods allows to conduct all release tests of the cell product and to obtain the results before administration of the cell product.

In certain embodiments, the methods as taught herein may comprise (d) resuspending the MSC-derived cells of chondro-osteoblastic lineage in a cryopreservation medium suitable for administration to a subject; and (e) cryopreserving the MSC-derived cells of chondro-osteoblastic lineage.

Hence, in a further aspect, the invention relates to a method for obtaining MSC-derived cells of chondro-osteoblastic lineage from MSC, the method comprising:
(a) culturing MSC recovered from a biological sample of a subject in a culture medium comprising FGF-2, TGFP and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a);
(c) passaging the MSC-derived cells for a second time and further culturing the MSC-derived cells in a medium as defined in (a), thereby obtaining the MSC-derived cells of chondro-osteoblastic lineage;
(d) resuspending the MSC-derived cells of chondro-osteoblastic lineage (of step (c)) in a cryopreservation medium suitable for administration to a subject; and
(e) cryopreserving the MSC-derived cells of chondro-osteoblastic lineage (of step (d)).

In certain embodiments, the methods as taught herein may comprise (d) resuspending the MSC-derived cells of chondro-osteoblastic lineage at a clinical concentration in a cryopreservation medium suitable for administration to a subject; and (e) cryopreserving the MSC-derived cells of chondro-osteoblastic lineage. Such clinical concentration advantageously allows to administer the MSC-derived cells of chondro-osteogenic lineage obtained by the present methods directly to a subject without any further dilution or concentration steps or without additional washing steps of the MSC-derived cells of chondro-osteoblastic lineage before administration.

In certain embodiments, the methods as taught herein may comprise cryopreserving the MSC-derived cells of chondro-osteoblastic lineage, thereby obtaining a cell product suitable for administration. The cell product suitable for administration may be directly delivered to a subject in need thereof without additional washing step before administration.

In certain embodiments of the methods, uses, or cell products as taught herein, the MSC-derived cells of chondro-osteoblastic lineage are resuspended in the cryopreservation medium in step (d) at a concentration of between about 1×10⁷ cells/ml and about 1×10⁸ cells/ml. For instance, the MSC-derived cells of chondro-osteoblastic lineage are resuspended in the cryopreservation medium in step (d) at a concentration of between about 1×10⁷ cells/ml and about 8×10⁷ cells/ml, between about 1x10' cells/ml and about 6×10⁷ cells/ml, or between about 2×10⁷ cells/ml and about 5×10⁷ cells/ml. Preferably, the MSC-derived cells of chondro-osteoblastic lineage are resuspended in the cryopreservation medium in step (d) at a concentration of between about 2×10⁷ cells/ml and about 4×10⁷ cells/ml.

In certain embodiments, the cell product suitable for administration comprises the MSC-derived cells of chondro-osteoblastic lineage at a concentration of between about 1×10⁷ cells/ml and about 1×10⁸ cells/ml. For instance, the cell product suitable for administration comprises the MSC-derived cells of chondro-osteoblastic lineage at a concentration of between about 1×10⁷ cells/ml and about 8×10⁷ cells/ml, between about 1×10⁷ cells/ml and about 6×10⁷ cells/ml, or between about 2×10⁷ cells/ml and about 5×10⁷ cells/ml. Preferably, the cell product suitable for administration comprises the MSC-derived cells of chondro-osteoblastic lineage at a concentration of between about 2×10⁷ cells/ml and about 4×10⁷ cells/ml.

In certain embodiments, the cell product suitable for administration comprises the MSC-derived cells of chondro-osteoblastic lineage in a cryopreservation medium at a concentration of between about 1x10' cells/ml and about 1×10⁸ cells/ml. For instance, the cell product suitable for administration comprises the MSC-derived cells of chondro-osteoblastic lineage in a cryopreservation medium at a concentration of between about 1×10⁷ cells /ml and about 8×10⁷ cells/ml, between about 1×10⁷ cells/ml and about 6×10⁷ cells/ml, or between about 2×10⁷ cells/ml and about 5×10⁷ cells/ml. Preferably, the cell product suitable for administration comprises the MSC-derived cells of chondro-osteoblastic lineage in a cryopreservation medium at a concentration of between about 2×10⁷ cells/ml and about 4×10⁷ cells/ml.

In certain embodiments of the methods, uses, or cell products as taught herein, the cryopreservation medium may comprise an anti-oxidative compound such as benzopyran.

In certain embodiments of the methods, uses, or cell products as taught herein, the cryopreservation medium may comprise dimethylsulfoxide (DMSO), human serum albumin (HSA), adenosine, polypeptide, benzopyran, or a combination thereof. In certain embodiments of the methods, uses, or cell products as taught herein, the cryopreservation medium may comprise DMSO, HSA, adenosine, (poly)peptides, an anti-oxidative compound, or a combination thereof. Such cryopreservation medium advantageously allows to administer the MSC-derived cells of chondro-osteogenic lineage obtained by the present methods without additional washing step of the MSC-derived cells of chondro-osteogenic lineage before administration to a subject.

In certain embodiments, the cell product suitable for administration comprises the MSC-derived cells of chondro-osteoblastic lineage in a cryopreservation medium comprising DMSO, HSA, adenosine, polypeptide, benzopyran, or a combination thereof, at a concentration of between about 1x10' cells/ml and about 1×10⁸ cells/ml. For instance, the cell product suitable for administration comprises the MSC-derived cells of chondro-osteoblastic lineage in a cryopreservation medium comprising DMSO, HSA, adenosine, polypeptide, benzopyran, or a combination thereof, at a concentration of between about 1×10⁷ cells/ml and about 8×10⁷ cells/ml, between about 1×10⁷ cells/ml and about 6×10⁷ cells/ml, or between about 2×10⁷ cells/ml and about 5×10⁷ cells/ml. Preferably, the cell product suitable for administration comprises the MSC-derived cells of chondro-osteoblastic lineage in a cryopreservation medium comprising DMSO, HSA, adenosine, polypeptide, benzopyran, or a combination thereof, at a concentration of between about 2×10⁷ cells/ml and about 4×10⁷ cells/ml.

In certain embodiments, the cell product suitable for administration comprises the MSC-derived cells of chondro-osteoblastic lineage in a cryopreservation medium comprising DMSO, HSA, adenosine, (poly)peptides, an anti-oxidative compound, or a combination thereof, at a concentration of between about 1x10' cells/ml and about 1×10⁸ cells/ml. For instance, the cell product suitable for administration comprises the MSC-derived cells of chondro-osteoblastic lineage in a cryopreservation medium comprising DMSO, HSA, adenosine, (poly)peptides, an anti-oxidative compound, or a combination thereof, at a concentration of between about 1×10⁷ cells/ml and about 8×10⁷ cells/ml, between about 1x10' cells/ml and about 6×10⁷ cells/ml, or between about 2×10⁷ cells/ml and about 5×10⁷ cells/ml. Preferably, the cell product suitable for administration comprises the MSC-derived cells of chondro-osteoblastic lineage in a cryopreservation medium comprising DMSO, HSA, adenosine, (poly)peptides, an anti-oxidative compound, or a combination thereof, at a concentration of between about 2×10⁷ cells/ml and about 4×10⁷ cells/ml.

In certain embodiments of the methods, uses, or cell products as taught herein, the cryopreservation medium may be a composition comprising nucleoside (e.g. adenosine), sugars (e.g. dextran-40, dextrose, sucrose, mannitol, lactobionic acid), tripeptide (e.g. L-glutathione), buffer (e.g. N-(2-Hydroxyethyl) piperazine-N'-(2-ethanesulfonic acid)), salts (e.g. sodium hydroxide, potassium chloride, potassium bicarbonate, potassium phosphate, calcium chloride, magnesium chloride) and DMSO. A commercially available cryopreservation medium is CryoStor^{®} CS10 cell cryopreservation medium (C2874, Merck KGaA, Darmstadt, Germany). Other examples are CryoStor^{®} CS5, CS2 or CSB cell cryopreservation medium (Merck KGaA, Darmstadt, Germany).

In certain embodiments, the cryopreservation medium may comprise at least 1%, at least 2%, at least 5%, or at least 10% of DMSO.

In certain embodiments of the methods, uses, or cell products as taught herein, the cryopreservation medium may be a composition comprising nucleoside (e.g. adenosine), sugars (e.g. dextran-40, dextrose, sucrose, mannitol, lactobionic acid), tripeptide (e.g. L-glutathione), buffer (e.g. N-(2-Hydroxyethyl) piperazine-N'-(2-ethanesulfonic acid)), salts (e.g. sodium hydroxide, potassium chloride, potassium bicarbonate, potassium phosphate, calcium chloride, magnesium chloride) and benzopyran (e.g. 6-hydroxy-2,5,7,8-tetramethylchroman-2-carboxylic acid). A commercially available cryopreservation medium is HypoThermosol^{®} FRS Preservation Solution (H4416, Merck KGaA, Darmstadt, Germany).

The terms "Trolox" or "6-hydroxy-2,5,7,8-tetramethylchroman-2-carboxylic acid" can be used interchangeably.

In certain embodiments, the cryopreservation medium may comprise at least 1%, at least 2%, at least 5%, or at least 10% of benzopyran. In certain embodiments, the cryopreservation medium may comprise at least 1%, at least 2%, at least 5%, or at least 10% of 6-hydroxy-2,5,7,8-tetramethylchroman-2-carboxylic acid.

In certain embodiments of the methods as taught herein, the MSC-derived cells may be cultured in step (a) for a period of from about 13 days to about 15 days, and in step (b) for a period of from about 8 days to about 12 days. In certain embodiments of the methods as taught herein, the MSC-derived cells may be cultured in step (a) for a period of from about 13 days to about 15 days, and in step (c) for a period of from about 10 days to about 14 days. In certain embodiments of the methods as taught herein, the MSC-derived cells may be cultured in step (b) for a period of from about 8 days to about 12 days, and in step (c) for a period of from about 10 days to about 14 days. In certain embodiments of the methods as taught herein, the MSC-derived cells may be cultured in step (a) for a period of from about 13 days to about 15 days, in step (b) for a period of from about 8 days to about 12 days, and in step (c) for a period of from about 10 days to about 14 days.

In certain embodiments of the methods as taught herein, the MSC-derived cells may be cultured in step (a) for a period of about 14 days, and in step (b) for a period of about 10 days. In certain embodiments of the methods as taught herein, the MSC-derived cells may be cultured in step (a) for a period of about 14 days, and in step (c) for a period of about 12 days. In certain embodiments of the methods as taught herein, the MSC-derived cells may be cultured in step (b) for a period of about 10 days, and in step (c) for a period of about 12 days. In certain embodiments of the methods as taught herein, the MSC-derived cells may be cultured in step (a) for a period of about 14 days, in step (b) for a period of about 10 days, and in step (c) for a period of about 12 days.

In certain embodiments of the methods as taught herein, the MSC-derived cells may be plated for the further culturing in step (b) at a density of 3×10² to 1×10³ cells/cm², and the further culturing in step (c) at a density of 3×10² to 1×10³ cells/cm².

In certain embodiments of the methods as taught herein, the MSC-derived cells may be plated for the further culturing in step (b) at a density of 3×10² to 8×10² cells/cm², and the further culturing in step (c) at a density of 3×10² to 8×10² cells/cm².

In a further aspect, the invention provides a population of MSC-derived cells of chondro-osteoblastic lineage obtainable or obtained by the methods as defined herein.

The recitations "population of MSC-derived cells of chondro-osteoblastic lineage" or "MSC-derived cells of chondro-osteoblastic lineage" may be used interchangeably herein.

The term "population" as used herein refers to a substantially pure (i.e. composed primarily of) and homogeneous group of cells of a desired cell type, such as of MSC-derived cells of chondro-osteoblastic lineage.

A further aspect relates to a population of MSC-derived cells of chondro-osteoblastic lineage obtainable or obtained by *in vitro* or *ex vivo* expansion of MSC, whereby at least 90% of the MSC-derived cells of chondro-osteoblastic lineage in suspension have a diameter equal to or less than 25 µm (D₉₀ ≤ 25 µm) and wherein at most 1 % of the MSC-derived cells of chondro-osteoblastic lineage in suspension have a diameter of more than 35 µm. In certain embodiments of the population of MSC-derived cells of chondro-osteoblastic lineage, the MSC-derived cells of chondro-osteoblastic lineage are obtainable or obtained by the methods as defined herein.

As illustrated in the example section, the methods as taught herein yield MSC-derived cells of chondro-osteoblastic lineage, or populations comprising such, with superior characteristics, such as in particular a smaller and more homogeneous size than MSC-derived cells described earlier. The smaller and more homogeneous size of the MSC-derived cells of chondro-osteoblastic lineage obtainable by the methods as described herein makes the cells having improved transplantation properties. More particularly, the smaller and more homogeneous size of the MSC-derived cells of chondro-osteoblastic lineage obtainable by the methods as described herein makes the cells suited for all routes of administration and in particular intravascular administration, inter alia, by reducing or eliminating the risk at pulmonary embolism and infarction, by offering a good *in vivo* safety profile and/or syringability. Furthermore, the MSC-derived cells of chondro-osteoblastic lineage obtainable by the methods as described herein allow a tunable and high cell concentration to be delivered at site with a limited volume administered.

In particular embodiments, the average diameter of the MSC-derived cells of chondro-osteoblastic lineage in suspension is less than 25 µm, less than 24 µm, less than 23 µm, less than 22 µm, less than 21 µm, or less than 20 µm. Preferably, the average diameter of the MSC-derived cells of chondro-osteoblastic lineage in suspension is less than 20 µm.

The terms "suspension" and "cell suspension" generally refers to MSC-derived cells, particularly viable MSC-derived cells, dispersed in a liquid phase.

In particular embodiments, the average diameter of the MSC-derived cells of chondro-osteoblastic lineage in suspension is more than 10 µm, more than 11 µm, more than 12 µm, more than 13 µm, more than 14 µm, or more than 15 µm.

In particular embodiments, the average diameter of the MSC-derived cells of chondro-osteoblastic lineage in suspension is between 10 µm and 25 µm, preferably between 15 µm and 20 µm.

In particular embodiments, at least 90% of the MSC-derived cells of chondro-osteoblastic lineage in suspension have a diameter equal to or less than 25 µm (D₉₀ ≤ 25 µm), equal to or less than 24 µm (D₉₀ ≤ 24 µm), equal to or less than 23 µm (D₉₀ ≤ 23 µm), equal to or less than 22 µm (D₉₀ ≤ 22 µm), equal to or less than 21 µm (D₉₀ ≤ 21 µm), or equal to or less than 20µm (D₉₀ ≤ 20 µm), preferably equal to or less than 25 µm (D₉₀≤ 25 µm).

In particular embodiments, the MSC-derived cells of chondro-osteoblastic lineage in suspension have a D₉₀ equal to or less than 25 µm (D₉₀ ≤ 25 µm) and at most 1% of the MSC-derived cells of chondro-osteoblastic lineage in suspension have a diameter of more than 35 µm. In particular embodiments, the MSC-derived cells of chondro-osteoblastic lineage in suspension have a D₉₀ equal to or less than 24 µm (D₉₀ ≤ 24 µm), equal to or less than 23 µm (D₉₀ ≤ 23 µm), equal to or less than 22 µm (D₉₀ ≤ 22 µm), equal to or less than 21 µm (D₉₀ ≤ 21 µm), or equal to or less than 20µm (D₉₀ ≤ 20 µm), and at most 1% of the MSC-derived cells of chondro-osteoblastic lineage in suspension have a diameter of more than 35 µm.

In particular embodiments, the MSC-derived cells of chondro-osteoblastic lineage in suspension have a D₉₀ equal to or less than 25 µm (D₉₀ ≤ 25 µm) and at most 1% of the MSC-derived cells of chondro-osteoblastic lineage in suspension have a diameter of more than 30 µm. In particular embodiments, the MSC-derived cells of chondro-osteoblastic lineage in suspension have a D₉₀ equal to or less than 24 µm (D₉₀ ≤ 24 µm), equal to or less than 23 µm (D₉₀ ≤ 23 µm), equal to or less than 22 µm (D₉₀ ≤ 22 µm), equal to or less than 21 µm (D₉₀ ≤ 21 µm), or equal to or less than 20µm (D₉₀ ≤ 20 µm), and at most 1% of the MSC-derived cells of chondro-osteoblastic lineage in suspension have a diameter of more than 30 µm.

In particular embodiments, the MSC-derived cells of chondro-osteoblastic lineage in suspension have a D₉₀ between about 25 µm and about 10 µm (10 µm ≤ D₉₀ ≤ 25 µm), between about 24 µm and about 10 µm (10 µm ≤ D₉₀≤ 24 µm), between about 23 µm and about 10 µm (10 µm ≤ D₉₀ ≤ 23 µm), between about 22 µm and about 10 µm (10 µm ≤ D₉₀ ≤ 22 µm), between about 21 µm and about 10 µm (10 µm ≤ D₉₀ ≤ 21 µm) or between about 20 µm and about 10 µm (10 µm ≤ D₉₀≤ 20 µm), preferably between about 25 µm and about 10 µm (10 µm ≤ D₉₀ ≤ 25 µm).

The diameter of a cell may be determined by any method known in the art, for example by a digital microscope and accompanying software for image analysis (e.g. Motic Image Plus^{®} 2.02). The average cell diameter as referred to herein should be determined based on the diameter of the cells in a free-floating, non-attached state, hence of the cells in suspension. The cells are preferably suspended in a solution comprising a transparent, non-toxic, isotonic buffer, such as PBS, and optionally a dye to differentiate living and dead cells, such as trypan blue. Preferably, at least hundred cells should be measured to consider the analysis statistically significant.

A further aspect relates to a composition comprising the MSC-derived cells of chondro-osteoblastic linage or the population of MSC-derived cells MSC-derived cells of chondro-osteoblastic linage as defined herein. In certain embodiments, the compositions may further comprise one or more other components. For example, components may be included that can maintain or enhance the viability of cells. By means of example and without limitation, such components may include salts to ensure substantially isotonic conditions, pH stabilisers such as buffer system(s) (e.g. to ensure substantially neutral pH, such as phosphate or carbonate buffer system), carrier proteins such as for example albumin, media including basal media and/or media supplements, serum or plasma, nutrients, carbohydrate sources, preservatives, stabilisers, anti-oxidants or other materials well known to those skilled in the art. Also disclosed are methods of producing said compositions by admixing the respective MSC-derived cells of chondro-osteoblastic linage with said one or more additional components as above. The compositions may be for example liquid or may be semi-solid or solid (e.g. may be frozen compositions or may exist as gel or may exist on solid support or scaffold, etc.).

The terms "composition", "formulation", or "preparation" may be used interchangeably herein.

In particular embodiments, the composition may be a pharmaceutical formulation comprising the MSC-derived cells of chondro-osteoblastic linage as defined herein, and optionally one or more pharmaceutically acceptable excipients.

Hence, in a further aspect, the invention provides a pharmaceutical formulation comprising the population of MSC-derived cells of chondro-osteoblastic lineage as defined herein.

The term "pharmaceutically acceptable" as used herein is consistent with the art and means compatible with the other ingredients of a pharmaceutical formulation and not deleterious to the recipient thereof.

As used herein, "carrier" or "excipient" includes any and all solvents, diluents, buffers (e.g. neutral buffered saline or phosphate buffered saline), solubilizers, colloids, dispersion media, vehicles, fillers, chelating agents (e.g. EDTA or glutathione), amino acids (e.g. glycine), proteins, disintegrants, binders, lubricants, wetting agents, emulsifiers, sweeteners, colorants, flavourings, aromatisers, thickeners, agents for achieving a depot effect, coatings, antifungal agents, preservatives, stabilisers, antioxidants, tonicity controlling agents, absorption delaying agents, and the like. The use of such media and agents for pharmaceutical active substances is well known in the art. Such materials should be non-toxic and should not interfere with the activity of the cells.

The precise nature of the carrier or excipient or other material will depend on the route of administration. For example, the composition may be in the form of a parenterally acceptable aqueous solution, which is pyrogen-free and has suitable pH, isotonicity and stability. For general principles in medicinal formulation, the reader is referred to Cell Therapy: Stem Cell Transplantation, Gene Therapy, and Cellular Immunotherapy, by G. Morstyn & W. Sheridan eds., Cambridge University Press, 1996; and Hematopoietic Stem Cell Therapy, E. D. Ball, J. Lister & P. Law, Churchill Livingstone, 2000.

Liquid pharmaceutical formulations may generally include a liquid carrier such as water or a pharmaceutically acceptable aqueous solution. For example, physiological saline solution, tissue or cell culture media, dextrose or other saccharide solution or glycols such as ethylene glycol, propylene glycol or polyethylene glycol may be included.

The composition may include one or more cell protective molecules, cell regenerative molecules, growth factors, anti-apoptotic factors or factors that regulate gene expression in the cells. Such substances may render the cells independent of its environment.

Such pharmaceutical formulations may contain further components ensuring the viability of the cells therein. For example, the compositions may comprise a suitable buffer system (e.g. phosphate or carbonate buffer system) to achieve desirable pH, more usually near neutral pH, and may comprise sufficient salt to ensure isosmotic conditions for the cells to prevent osmotic stress. For example, suitable solution for these purposes may be phosphate-buffered saline (PBS), sodium chloride solution, Ringer's Injection or Lactated Ringer's Injection, as known in the art. Further, the composition may comprise a carrier protein, e.g. albumin (e.g. bovine or human albumin), which may increase the viability of the cells.

Further suitably pharmaceutically acceptable carriers or additives are well known to those skilled in the art and for instance may be selected from proteins such as collagen or gelatine, carbohydrates such as starch, polysaccharides, sugars (dextrose, glucose and sucrose), cellulose derivatives like sodium or calcium carboxymethylcellulose, hydroxypropyl cellulose or hydroxypropylmethyl cellulose, pregeletanized starches, pectin agar, carrageenan, clays, hydrophilic gums (acacia gum, guar gum, arabic gum and xanthan gum), alginic acid, alginates, hyaluronic acid, polyglycolic and polylactic acid, dextran, pectins, synthetic polymers such as water-soluble acrylic polymer or polyvinylpyrrolidone, proteoglycans, calcium phosphate and the like.

If desired, cell preparation can be administered on a support, scaffold, matrix or material to provide improved tissue regeneration. For example, the material can be a granular ceramic, or a biopolymer such as gelatine, collagen, or fibrinogen. Porous matrices can be synthesized according to standard techniques (e.g. Mikos et al., Biomaterials 14: 323, 1993; Mikos et al., Polymer 35:1068, 1994; Cook et al., J. Biomed. Mater. Res. 35:513, 1997). Such support, scaffold, matrix or material may be biodegradable or non-biodegradable. Hence, the cells may be transferred to and/or cultured on suitable substrate, such as porous or non-porous substrate, to provide for implants. For example, cells that have proliferated, or that are being differentiated in culture dishes, can be transferred onto three-dimensional solid supports in order to cause them to multiply and/or continue the differentiation process by incubating the solid support in a liquid nutrient medium of the invention, if necessary. Cells can be transferred onto a three-dimensional solid support, e.g. by impregnating said support with a liquid suspension containing said cells. The impregnated supports obtained in this way can be implanted in a subject. Such impregnated supports can also be re-cultured by immersing them in a liquid culture medium, prior to being finally implanted. The three-dimensional solid support needs to be biocompatible so as to enable it to be implanted in a human. It may be biodegradable or non-biodegradable.

The cells or cell populations can be administered in a manner that permits them to survive, grow, propagate and/or differentiate towards desired cell types such as osteoblasts. The cells or cell populations may be grafted to or may migrate to and engraft within the intended organ, such as bone defects. Engraftment of the cells or cell populations in other places may be envisaged.

In an embodiment, the pharmaceutical cell preparation as defined above may be administered in a form of liquid composition. In embodiments, the cells or pharmaceutical formulation comprising such can be administered systemically, topically, within an organ, at a site of organ dysfunction or lesion or at a site of tissue lesion.

Preferably, the pharmaceutical formulations may comprise a therapeutically effective amount of the desired cells. The term "therapeutically effective amount" refers to an amount which can elicit a biological or medicinal response in a tissue, system, animal or human that is being sought by a researcher, veterinarian, medical doctor or other clinician, and in particular can prevent or alleviate one or more of the local or systemic symptoms or features of a disease or condition being treated. Appropriate therapeutically effective amounts may be determined by a qualified physician with due regard to the nature of the desired cells, the disease condition and severity, and the age, size and condition of the subject.

Also provided are methods of producing said pharmaceutical formulations by admixing the cells of the invention with one or more additional components as described above as well as with one or more pharmaceutical excipients as described above.

In an embodiment, the pharmaceutical formulation as define above may be administered in a form of liquid or viscous composition.

In certain embodiments of the methods, uses, or cell products as taught herein, the pharmaceutical formulation may further comprise a component with osteo-conductive properties, such as tricalcium phosphate, hydroxyapatite, combination of hydroxyapatite/tricalcium phosphate particles, poly-lactic acid, poly-lactic glycolic acid, hyaluronic acid or a derivative thereof, chitosan, poly-L-lysine, gelatine, collagen, osteonectin, fibrinogen, osteocalcin, or a combination thereof.

The term "osteo-conductive" refers to the ability of a component to serve as a scaffold on which cells, such as the MSC-derived cells of chondro-osteoblastic lineage as taught herein, can attach, migrate, grow and produce new bone.

As mentioned above, the pharmaceutical formulations as taught herein may comprise components useful in the repair of bone wounds and bone defects. The pharmaceutical formulations may comprise a scaffold or matrix with osteo-conductive properties. The pharmaceutical formulations may be combined with demineralized bone matrix (DBM) or other matrices to make the composite osteogenic as well as osteo-conductive and osteo-inductive. Similar methods using autologous bone marrow cells with allogeneic DBM have yielded good results (Connolly et al. 1995. Clin Orthop 313: 8-18).

The pharmaceutical formulations as taught herein may further include or be co-administered with a complementary bioactive factor or osteo-inductive protein such as a bone morphogenetic protein, such as BMP-2, BMP-7 or BMP-4, or any other growth factor. Other potential accompanying components include inorganic sources of calcium or phosphate suitable for assisting bone regeneration (WO 00/07639). If desired, cell preparation can be administered on a carrier matrix or material to provide improved tissue regeneration. For example, the material can be a hydrogel, or a biopolymer such as gelatine, collagen, hyaluronic acid or derivatives thereof, osteonectin, fibrinogen, or osteocalcin. Biomaterials can be synthesized according to standard techniques (e.g. Mikos et al., Biomaterials 14:323, 1993; Mikos et al., Polymer 35:1068, 1994; Cook et al., J. Biomed. Mater. Res. 35:513, 1997).

Also disclosed is an arrangement or kit of parts comprising a surgical instrument or device for administration of the MSC-derived cells of chondro-osteoblastic lineage as taught herein or the pharmaceutical formulation as taught herein to a subject, such as for example systemically, for example, by injection, and further comprising the MSC-derived cells of chondro-osteoblastic lineage as taught herein or the pharmaceutical formulation as taught herein.

In another aspect, the invention provides the population of MSC-derived cells of chondro-osteoblastic lineage as defined herein, or the pharmaceutical formulation as defined herein for use as a medicament.

In another aspect, the invention provides the population of MSC-derived cells of chondro-osteoblastic lineage as defined herein, or the pharmaceutical formulation as defined herein for use in the treatment of a subject in need of transplantation of cells of chondro-osteoblastic lineage.

In related aspects, the invention provides a method of treating a subject in need of transplantation of cells of chondro-osteoblastic lineage comprising administering to said subject a therapeutically effective amount of the herein defined MSC-derived cells of chondro-osteoblastic lineage or population of MSC-derived cells of chondro-osteoblastic lineage or pharmaceutical formulations comprising the MSC-derived cells of chondro-osteoblastic lineage or population of MSC-derived cells of chondro-osteoblastic lineage to the subject.In related aspects, the invention provides the use of the above defined MSC-derived cells of chondro-osteoblastic lineage or population of MSC-derived cells of chondro-osteoblastic lineage or pharmaceutical formulations comprising the MSC-derived cells of chondro-osteoblastic lineage or population of MSC-derived cells of chondro-osteoblastic lineage for the manufacture of a medicament for treatment of a subject in need of transplantation of cells of chondro-osteoblastic lineage.

As used herein, the terms "treat" or "treatment" refer to both therapeutic treatment and prophylactic or preventative measures, wherein the object is to prevent or slow down (lessen) an undesired physiological change or disorder. Beneficial or desired clinical results include, but are not limited to, alleviation of symptoms, diminishment of extent of disease, stabilised (i.e. not worsening) state of disease, delay or slowing of disease progression and occurrence of complications, amelioration or palliation of the disease state. "Treatment" can also mean prolonging survival as compared to expected survival if not receiving treatment.

The term "subject in need of transplantation of cells of chondro-osteoblastic lineage" as used herein, includes subjects, such as mammalian or human subjects, that would benefit from treatment of a given condition, preferably a condition or disease as defined herein. Such subjects will typically include, without limitation, those that have been diagnosed with the condition, those prone to have or develop the said condition and/or those in whom the condition is to be prevented.

The term "transplantation" or "cell transplantation" carries its normal meaning and particularly refers to the administration of cells to a subject. The term "cell transplantation" can be used interchangeably with "cell therapy". Cell transplantation may be performed by any technique known in the art. By means of example, and without limitation, cells may be transplanted by infusion into a subject. Typically, cell infusion may be performed parenterally, e.g. intravascularly, subcutaneously, intradermally, or intramuscularly, preferably intravascularly. Cells may be administered for instance, and without limitation, systemically, topically or at the site of a lesion. It may be clear that, depending on the specific application, targeted tissues, therapeutic purpose or cell type, adjustment may be made accordingly in respect of routes of administration, as well as formulations, concentrations, etc.

In certain embodiments of the methods, uses, or cell products as taught herein, the population of MSC-derived cells of chondro-osteoblastic lineage or the pharmaceutical formulation may be suitable for percutaneous, intra-osseous, intra-articular, intervertebral or intravascular administration.

In certain embodiments of the methods, uses, or cell products as taught herein, the population of MSC-derived cells of chondro-osteoblastic lineage or the pharmaceutical formulation may be suitable for administration at a site of a bone defect.

The homogeneous and small cell size of the MSC-derived cells of chondro-osteoblastic lineage as taught herein leads to a reduced or abrogated acute toxicity upon intravenous administration of said cells to a subject. Therefore, the MSC-derived cells of chondro-osteoblastic lineage as taught herein are particular suitable for intravascular or percutaneous administration.

Therefore, in particular embodiments, the above defined MSC-derived cells of chondro-osteoblastic lineage or population of MSC-derived cells of chondro-osteoblastic lineage or the pharmaceutical formulation may be administered to said subject in need of transplantation of cells of chondro-osteoblastic lineage percutaneous or intravascular.

Furthermore, the inventors found that MSC-derived cells of chondro-osteoblastic lineage obtained by the methods as taught herein have osteogenic properties.

Accordingly, in a particular embodiment, the subject in need of transplantation of cells of chondro-osteoblastic lineage may be a subject having a musculoskeletal disease.

The term "musculoskeletal disease", as used herein, refers to any type of bone disease, muscle disease, joint disease, or chondrodystrophy, the treatment of which may benefit from the administration of the present pharmaceutical formulation to a subject having the disease. In particular, such disease may be characterized, e.g. by decreased bone and/or cartilage formation or excessive bone and/or cartilage resorption, by decreased number, viability or function of osteoblasts or osteocytes present in the bone and/or chondroblasts or chondrocytes present in the cartilage, decreased bone mass and/or cartilage mass in a subject, thinning of bone, compromised bone strength or elasticity, etc.

Non-limiting examples of musculoskeletal diseases may include local or systemic disorders, such as, any type of osteoporosis or osteopenia, e.g. primary, postmenopausal, senile, corticoid-induced, bisphosphonates-induced, and radiotherapy-induced; any secondary, mono- or multisite osteonecrosis; any type of fracture, e.g. non-union, mal-union, delayed union fractures or compression, maxillo-facial fractures; conditions requiring bone fusion (e.g. spinal fusions and rebuilding); congenital bone defect; bone reconstruction, e.g. after traumatic injury or cancer surgery, and cranio-facial bone reconstruction; traumatic arthritis, focal cartilage and/or joint defect, focal degenerative arthritis; osteoarthritis, degenerative arthritis, gonarthrosis, and coxarthrosis; osteogenesis imperfecta; osteolytic bone cancer; Paget's Disease; endocrinological disorders; hypophosphatemia; hypocalcemia; renal osteodystrophy; osteomalacia; adynamic bone disease, hyperparathyroidism, primary hyperparathyroidism, secondary hyperparathyroidism; periodontal disease; Gorham-Stout disease and McCune-Albright syndrome; rheumatoid arthritis; spondyloarthropathies, including ankylosing spondylitis, psoriatic arthritis, enteropathic arthropathy, and undifferentiated spondyloarthritis and reactive arthritis; systemic lupus erythematosus and related syndromes; scleroderma and related disorders; Sjogren's Syndrome; systemic vasculitis, including Giant cell arteritis (Horton's disease), Takayasu's arteritis, polymyalgia rheumatica, ANCA-associated vasculitis (such as Wegener's granulomatosis, microscopic polyangiitis, and Churg-Strauss Syndrome), Behcet's Syndrome, and other polyarteritis and related disorders (such as polyarteritis nodosa, Cogan's Syndrome, and Buerger's disease); arthritis accompanying other systemic inflammatory diseases, including amyloidosis and sarcoidosis; crystal arthropathies, including gout, calcium pyrophosphate dihydrate disease, disorders or syndromes associated with articular deposition of calcium phosphate or calcium oxalate crystals; chondrocalcinosis and neuropathic arthropathy; Felty's Syndrome and Reiter's Syndrome; Lyme disease and rheumatic fever.

In a particular embodiment, the subject in need of transplantation of cells of chondro-osteoblastic lineage may be a subject having a bone-related disorder.

Accordingly, the term "bone-related disorder" as used herein refers to any type of bone disease, the treatment of which may benefit from the transplantation of cells of chondro-osteoblastic lineage, e.g. osteochondroprogenitors, osteoprogenitors, pre-osteoblasts, osteoblasts or osteoblast phenotype cells to a subject having the disorder. In particular, such disorders may be characterized, e.g. by decreased bone formation or excessive bone resorption, by decreased number, viability or function of osteoblasts or osteocytes present in the bone, decreased bone mass in a subject, thinning of bone, compromised bone strength or elasticity, etc.

By way of example, but not limitation, bone-related disorders which can benefit from transplantation of MSC-derived cells of chondro-osteoblastic lineage (e.g. cells of osteoblastic lineage) obtained by the method of the present invention may include local or systemic disorders, such as, any type of osteoporosis or osteopenia, e.g. primary, postmenopausal, senile, corticoid-induced, any secondary, mono- or multisite osteonecrosis, any type of fracture, e.g. non-union, mal-union, delayed union fractures or compression, conditions requiring bone fusion (e.g. spinal fusions and rebuilding), maxillo-facial fractures, bone reconstruction, e.g. after traumatic injury or cancer surgery, cranio-facial bone reconstruction, osteogenesis imperfecta, osteolytic bone cancer, Paget's Disease, endocrinological disorders, hypophosphatemia, hypocalcemia, renal osteodystrophy, osteomalacia, adynamic bone disease, rheumatoid arthritis, hyperparathyroidism, primary hyperparathyroidism, secondary hyperparathyroidism, periodontal disease, Gorham-Stout disease and McCune-Albright syndrome.

The MSC-derived cells of chondro-osteoblastic lineage, the population of MSC-derived cells of chondro-osteoblastic lineage and pharmaceutical formulations described herein may be used alone or in combination with any of the known therapies or active compounds for the respective disorders. The administration may be simultaneous or sequential in any order, as described elsewhere.

If the cells are derived from heterologous (i.e. non-autologous, non-homologous or non-allogeneic) source, concomitant immunosuppression therapy may be typically administered, e.g. using immunosuppressive agents, such as cyclosporine or tacrolimus (FK506).

The quantity of cells to be administered will vary for the subject being treated. In a preferred embodiment, the quantity of cells to be administered is between 10² to 10¹⁰ or between 10² to 10⁹, or between 10³ to 10¹⁰ or between 10³ to 10⁹, or between 10⁴ to 10¹⁰ or between 10⁴ to 10⁹, such as between 10⁴ and 10⁸, or between 10⁵ and 10⁷, e.g. about 1×10⁵, about 5×10⁵, about 1×10⁶, about 5×10⁶, about 1×10⁷, about 5×10⁷, about 1×10⁸, about 5×10⁸, about 1×10⁹, about 2×10⁹, about 3×10⁹, about 4×10⁹, about 5×10⁹, about 6×10⁹, about 7×10⁹, about 8×10⁹, about 9×10⁹ or about 1×10¹⁰ cells can be administered to a human subject. In further embodiments, between 10⁶ to 10⁸ cells per kg body weight or between 1x10' to 9×10⁷ cells per kg body weight, e.g. about 1×10⁷, about 2x10', about 3×10⁷, about 4×10⁷, about 5×10⁷, about 6×10⁷, about 7×10⁷, about 8×10⁷, about 9×10⁷ or about 1×10⁸ cells per kg body weight can be administered to a human subject. For example, such number of cells or such number of cells per kg body weight may particularly refer to the total number of cells to be administered to a subject, which administration may be suitably distributed over one or more doses (e.g. distributed over 2, 3, 4, 5, 6, 7, 8 9 or 10 or more doses) administered over one or more days (e.g. over 1, 2, 3, 4 or 5 or more days). However, the precise determination of a therapeutically effective dose may be based on factors individual to each patient, including their size, age, size tissue damage, and amount of time since the damage occurred, and can be readily ascertained by those skilled in the art from this disclosure and the knowledge in the art.

Suitably, in a pharmaceutical formulation to be administered, the MSC-derived cells of chondro-osteoblastic lineage may be present at a concentration between about 10⁴ cells/ml to about 10⁹ cells/ml, preferably between about 10⁵ cells/ml and about 10⁸ cells/ml, yet more preferably between about 1×10⁶ cells/ml and about 1×10⁸ cells/ml.

In certain embodiments, the pharmaceutical formulation to be administered comprises the MSC-derived cells of chondro-osteoblastic lineage at a concentration of between about 1×10⁷ cells/ml and about 1×10⁸ cells/ml. In certain embodiments, the pharmaceutical formulation to be administered comprises the MSC-derived cells of chondro-osteoblastic lineage at a concentration of between about 2×10⁷ cells/ml and about 4×10⁷ cells/ml.

In certain embodiments, the pharmaceutical formulation to be administered comprises the MSC-derived cells of chondro-osteoblastic lineage in a cryopreservation medium at a concentration of between about 1×10⁷ cells/ml and about 1×10⁸ cells/ml. In certain embodiments, the pharmaceutical formulation to be administered comprises the MSC-derived cells of chondro-osteoblastic lineage in a cryopreservation medium at a concentration of between about 2×10⁷ cells/ml and about 4×10⁷ cells/ml.

In certain embodiments, the pharmaceutical formulation to be administered comprises the MSC-derived cells of chondro-osteoblastic lineage in a cryopreservation medium comprising DMSO, HSA, adenosine, polypeptide, benzopyran, or a combination thereof, at a concentration of between about 1x10' cells/ml and about 1×10⁸ cells/ml. In certain embodiments, the pharmaceutical formulation to be administered comprises the MSC-derived cells of chondro-osteoblastic lineage in a cryopreservation medium comprising DMSO, HSA, adenosine, polypeptide, benzopyran, or a combination thereof, at a concentration of between about 2×10⁷ cells/ml and about 4×10⁷ cells/ml. In certain embodiments of the methods, uses, or cell products as taught herein, the MSC-derived cells of chondro-osteoblastic lineage may be present, e.g. in a pharmaceutical formulation to be administered, at a concentration between about 1×10⁷ cells/ml and about 1×10⁸ cells/ml, preferably between about 2×10⁷ cells/ml and about 4×10⁷ cells/ml.

The reduced cell size of the MSC-derived cells of chondro-osteoblastic lineage as taught herein allows a tunable and/or high cell concentration. Accordingly, if the composition is a liquid composition, the volume of the composition comprising MSC-derived cells of chondro-osteoblastic lineage obtained by the method as taught herein to be administered to the subject in need of transplantation of MSC-derived cells is smaller than the volume of the composition comprising MSC-derived cells obtained by other methods.

### EXAMPLES

### Example 1: Method for obtaining MSC-derived cells of chondro-osteoblastic lineage according to an embodiment of the invention

Conventional culture medium supplemented with 5% OctaPlasLG^{®} (Octapharma), 0.1 UI/ml heparin (LEO Pharma), FGF-b (CellGenix) and TGFβ-1 (Humanzyme) was used as the culture medium.

20 to 60 ml of human bone marrow (BM) aspirates was obtained from the iliac crest of a healthy volunteer donor. After harvesting, bone marrow white blood cells were counted, seeded at a density of 50,000 cells/cm² in the culture medium, and incubated at 37°C in a humidified incubator containing 5% CO₂. 4 days after cell seeding, non-adherent cells were removed and the medium was renewed with culture medium. 7 days and 11 days after seeding, half of the culture medium was removed and replaced with fresh one to renew growth factors. Cells were cultured during primary culture for 14 days. At day 14, cells were harvested by detachment with Trypzean (Lonza) and by swirling and pipetting up and down (passage 1: P1). The intermediate cells were cryopreserved (in CryoStor^{®} CS10) and stored in liquid nitrogen. Each cell stock was issued from one donor, with no pooling between donors.

Next, intermediate cells were thawed and re-plated for secondary culture at a density of 572 cells/cm². Cells were cultured during secondary culture for 10 days. At day 24, cells were harvested by detachment with Trypzean (Lonza) and by swirling and pipetting up and down (passage 2: P2). The intermediate cells were cryopreserved (in CryoStor^{®} CS10) and stored in liquid nitrogen.

Subsequently, intermediate cells were thawed and re-plated for tertiary culture at a density of 572 cells/cm². Cells were cultured during tertiary culture for 10 day. At day 34, cells were harvested by detachment with Trypzean (Lonza) and by swirling and pipetting up and down (passage 3: P3). To obtain the final cell product, cells were resuspended in OctaPlasLG^{®} at a final concentration of 25×10⁶ cells/ml. This cell product will be referred to herein as "Cell product C - fresh".

At the end of the tertiary culture, cells were also cryopreserved for long-time storage. Thereto, the cells were resuspended in cryopreservation medium to reach the desired concentration (25×10⁶ cells/ml). The cell suspension was then transferred into cryotubes which were stored in liquid nitrogen. This cell product will be referred to herein as "Cell product C - cryo" or "bone-forming cells C cryo(preserved)" also abbreviated as "B-F cells C". The cryopreservation medium was:
- CryoStor^{®} CS10 (BioLife Solutions Inc.), or
- 50% (v/v) CryoStor^{®} CS10 (BioLife Solutions Inc.) and 50% (v/v) human serum albumin (Octapharma), or
- 95% (v/v) CryoStor^{®} CS10 (BioLife Solutions Inc.) and 5% (v/v) human serum albumin (Octapharma), or
- 80% (v/v) Hypothermosol^{®} (BioLife Solutions Inc.), 10% (v/v) DMSO, and 10% (v/v) human serum albumin (Octapharma).

### Comparative Example 1: Prior art methods for obtaining mesenchymal stem cells and MSC-derived cells

Comparative cell products according to the prior art and their manufacturing methods are described below.

### Mesenchymal stem cells

Undifferentiated MSC were prepared by obtaining human bone marrow (BM) aspirates from the iliac crest of healthy volunteer donors. After harvesting, bone marrow white blood cells were counted, seeded at a density of 50,000 cells/cm² in a conventional culture medium, and incubated at 37°C in a humidified incubator containing 5% CO₂. After 24h, the culture medium was removed and cells fresh culture medium was added. The culture medium was replaced every 2-3 days. When over half of the colonies reached a confluence of 80% or when some colonies reached a confluence of 100%, cells were harvested (passage 1: P1). At this first passage, cells were directly cryopreserved in CryoStor^{®} CS10 (BioLife Solutions Inc.). To finish the culture process, MSCs were thawed, plated at 572 cells/cm² for second culture, and cultivated. Cells were harvested when over half of the colonies reached a confluence of 80% or when some colonies reached a confluence of 100% to obtain MSCs at passage 2 (P2). This cell product is referred to herein as "MSC".

### Cell product A

Conventional culture medium supplemented with 5% Octaserum (50:50 autologous serum and OctaPlasLG^{®} (Octapharma)), FGF-b (CellGenix) and TGFβ-1 (Humanzyme) was used as the culture medium.

Freezing medium: 80% conventional culture medium, 10% Octaserum (50:50 autologous serum and OctaPlasLG^{®} (Octapharma)), 10% DMSO.

The *in vitro* differentiated MSC-derived cells referred to herein as "Cell product A" were prepared by obtaining human BM aspirates from the iliac crest of healthy volunteer donors. After harvesting, bone marrow white blood cells were counted, seeded at a density of 50,000 cells/cm² in the culture medium, and incubated at 37°C in a humidified incubator containing 5% CO₂. 4 days after cell seeding, non-adherent cells were removed and the medium was renewed with culture medium. 7 days and 11 days after seeding, half of the culture medium was removed and replaced with fresh one. Cells were cultured during primary culture for 14 days. At day 14, cells were harvested by detachment with Trypzean (Lonza) and by swirling and pipetting up and down (passage 1: P1). The intermediate cells were cryopreserved in CryoStor^{®} CS10 (BioLife Solutions Inc.) or freezing medium and stored in liquid nitrogen.

For secondary culture, cells were thawed and re-plated at a density of 1144 cells/cm². Cells were cultured during secondary culture for 14 days. At day 28, cells were harvested by detachment with Trypzean (Lonza) and by swirling and pipetting up and down (passage 2: P2). To obtain the final cell product, cells were resuspended in OctaPlasLG^{®} at a final concentration of 25×10⁶ cells/ml. This cell product is referred to herein as "Cell product A".

### Cell product B

Conventional culture medium supplemented with 5% OctaPlasLG^{®} (Octapharma), 0.1 UI/ml heparin (LEO Pharma), FGF-b (CellGenix) and TGFβ-1 (Humanzyme) was used as the culture medium.

*In vitro* differentiated MSC-derived cells were prepared by obtaining human BM aspirates from the iliac crest of healthy volunteer donors. After harvesting, bone marrow white blood cells were counted, seeded at a density of 50,000 cells/cm² in the culture medium, and incubated at 37°C in a humidified incubator containing 5% CO₂. Four days after cell seeding, non-adherent cells were removed and the medium was renewed with culture medium. Seven days and eleven days after seeding, half of the culture medium was removed and replaced with fresh one to renew growth factors. Cells were cultured during primary culture for 14 days. At day 14, cells were harvested by detachment with Trypzean (Lonza) and by swirling and pipetting up and down (passage 1: P1). The intermediate cells were cryopreserved in CryoStor^{®} CS10 (BioLife Solutions Inc.) and stored in liquid nitrogen.

Next, intermediate cells were thawed and re-plated for secondary culture at a density of 286 cells/cm². Cells were cultured during secondary culture for 14 days. At day 28, cells were harvested by detachment with Trypzean (Lonza) and by swirling and pipetting up and down (passage 2: P2). To obtain the final cell product, cells were resuspended in OctaPlasLG^{®} at a final concentration of 25×10⁶ cells/ml. This cell product is referred to herein as "Cell product B".

### Example 2: in vitro cell characterisation of MSC-derived cells of chondro-osteoblastic lineage obtained by methods according to embodiments of the invention, and of MSCs and MSC-derived cells obtained by prior art methods

### Material and methods

### Cells

The cell products illustrating the invention (i.e. cell product C fresh and cell products C cryo) were obtained as described in Example 1. The comparative cell products (i.e. MSC, cell product A and cell product B) were obtained as described in Comparative Example 1.

### Cell counting and viability

Cell density and viability were determined using a trypan blue exclusion assay. After harvesting, cells were diluted 1:2 with Trypan Blue (0.4%, Lonza Bio Whittaker^{®}) and cell viability was analysed using a Bürker chamber (Sigma-Aldrich^{®}) and an inverted microscope (AE31, Motic^{®}). Cell viability was also analysed by flow cytometry using Amino-Actinomycin D (7-AAD, BD Biosciences^{®}), the BD FACSCanto II^{™} and the BD FACSDiva^{™} softwares (Becton Dickinson^{®}). After harvesting, 50,000 cells were incubated in the dark for 10 min at room temperature in phosphate-buffered saline (PBS) - 1% bovine serum albumin (BSA) (Lonza Bio Whittaker^{®}) with 2.5 µl of 7-AAD.

### Flow cytometry

Comparative cell products (i.e. MSC and MSC-derived cells: Cell products A and B) and cell products illustrating the invention (i.e. MSC-derived cells of chondro-osteoblastic lineage: cell products C) obtained as described in Comparative Example 1 and Example 1 above, were harvested and cell surface markers were analysed by flow cytometry (BD FACSCanto^{™} II and the BD FACSDiva^{™} softwares; Becton Dickinson). Cells were incubated with the following conjugated monoclonal antibodies: anti-CD73, anti-CD90 and anti-CD166 (which are mesenchymal markers, and should be highly expressed by the MSC or MSC-derived cells), anti-CD3, anti-CD34 and anti-CD45 (which are hematopoietic markers, and should be substantially absent from the MSC or MSC-derived cells), anti-CD44, anti-CD51/61, anti-CD49a-e, anti-CD29 (which are adhesion markers), anti-CD40, anti-CD86 and anti-HLA-DR (which are immunogenicity markers), and anti-alkaline phosphatase (ALP) for 15 min at room temperature, and then washed with PBS before centrifugation and re-suspension in 0.3 ml PBS.

For the characterization of cell surface markers CD105, CD73, CD10 and CD44, 5×10⁴ cells at a concentration of 1×10⁶cells/ml in PBS - 1% BSA were incubated 10 min in the dark with 5µl of antibodies. After this incubation time, cells were washed once with PBS. The different antibodies used for extracellular staining are the following: allophycocyanin (APC)-conjugated antibodies against CD105 (BD Biosciences^{®}, Cat N°: 562408), CD73 (BD Biosciences^{®}, Cat N°:560847), Phycoerythrin (PE)-conjugated antibodies against CD10 (BD Biosciences^{®}, Cat N°: 555375), CD44 (BD Biosciences^{®}, Cat N°: 550989). Nonspecific staining was determined by incubating cells with immunoglobulin G (IgG) control conjugated with FITC, APC and PE (all BD Biosciences^{®}, Cat N°: 556649; 555751; 556650 respectively). Before analysis, gating of singlets and population of interest were performed. The flow cytometry analysis was done on 1×10⁴ events of the gated population using FACSCanto^{™} II (BD Biosciences^{®}) and FACSDiva^{®} 8.0 software (BD Biosciences^{®}). Settings parameters used for the analysis were performed automatically with beads (BD CompBeads Plus^{®}, Cat N°560497). For each conjugate, the positivity cut-off was fixed at 1% of positivity of the control isotype antibody and the positivity of each marker was determined. The median of fluorescence intensity (MFI) of the whole analysed population was also determined and divided by the MFI of the corresponding isotype control antibody to obtain the normalized MFI (nMFI).

**Table 1: Overview vendors and catalogue numbers of antibodies used in examples**

| **Anti-body** | **Supplier** | **Catalogue number** |
|---|---|---|
| Anti-ALP | BD Biosciences | 561433 |
| Anti-CD166 | BD Biosciences | 560903 |
| Anti-CD3 | BD Biosciences | 555340 |
| Anti-CD34 | BD Biosciences | 555824 |
| Anti-CD40 | BD Biosciences | 555588 |
| Anti-CD44 | BD Biosciences | 550989 |
| Anti-CD45 | BD Biosciences | 555485 |
| Anti-CD49a | BD Biosciences | 559596 |
| Anti-CD49b | BD Biosciences | 555669 |
| Anti-CD49c | BD Biosciences | 556025 |
| Anti-CD49d | BD Biosciences | 555503 |
| Anti-CD49e | BD Biosciences | 555617 |
| Anti-CD51/61 | BD Biosciences | 550037 |
| Anti-CD73 | BD Biosciences | 561254 |
| Anti-CD29 | BD Biosciences | 556048 |
| Anti-CD86 | BD Biosciences | 555660 |
| Anti-CD90 | R&D System | FAB7335P |
| Anti-HLA-DR | BD Biosciences | 555558 |
| Anti-CD105 | BD Biosciences | 562408 |
| Anti-CD10 | BD Biosciences | 555375 |
| Anti-HLA-DR-DP-DQ | BD Biosciences | 555558 |
| Anti-HLA-ABC | BD Biosciences | 555552 |

### ALP enzymatic activity measurement

ALP enzymatic activity was measured by a biochemical assay based on the hydrolysis of p-nitrophenyl phosphate (pNPP). After being dephosphorylated by ALP, the pNPP become yellow and can be detected by a spectrophotometer at 410 nm. The ALP enzymatic activity of the cells is determined with respect to a standard curve based on purified calf intestinal ALP activity. The ALP activity is reported in Unit of ALP/mg of protein. One unit of ALP hydrolyzes 1 µmol of pNPP in 1 min at 37°C.

### Reverse Transcription-quantitative Polymerase Chain Reaction (RT-qPCR)

After harvesting, cells were stored at -80°C as dry pellets (500,000 cells) until RNA extraction. Total RNAs were extracted using RNeasy^{®} Mini Kit (Qiagen^{®}) according to manufacturer's instructions. RNA concentration was measured using DropSense^{®} 16 (Trinean^{®}). RNA reverse transcription (RT) were performed from 1 µg of total RNA extracts, using PrimeScript^{®} RT reagent Kit (Takara^{®}) according to manufacturer's instructions. qPCRs were performed using Premix Ex Taq^{®} (Takara^{®}) from 2 µl of cDNA following manufacturer's instructions. The expression levels of the following genes of interest were quantified: The expression levels of the following genes of interest were quantified: RUNX2 (Forward:GGTTCCAGCAGGTAGCTGAG (SEQ ID NO: 1), Reverse:AGACACCAAACTCCACAGCC (SEQ ID NO: 2)), SOX9 (F:TAAAGGCAACTCGTACCCAA (SEQ ID NO: 3), R: ATTCTCCATCATCCTCCACG (SEQ ID NO: 4), BMP2 (F:GGAACGGACATTCGGTCCTT (SEQ ID NO: 5), R:CACCATGGTCGACCTTTAGGA (SEQ ID NO: 6)), ALPL (F:ACCATTCCCACGTCTTCACATTTG (SEQ ID NO: 7), R: AGACATTCTCTCGTTCACCGCC (SEQ ID NO: 8)), MMP13 (F:TGGAATTAAGGAGCATGGCGA (SEQ ID NO: 9), R: AACTCATGCGCAGCAACAAG (SEQ ID NO: 10)), CHI3L1(F:TGGGTCTCAAAGATTTTCCAAGA (SEQ ID NO: 11), R: GCTGTTTGTCTCTCCGTCCA (SEQ ID NO: 12)), DCN (F:AAAATGCCCAAAACTCTTCAGG (SEQ ID NO: 13), R:GCCCCATTTTCAATTCCTGAG (SEQ ID NO: 14)), OCN (F:AAGGTGCAGCCTTTGTGT (SEQ ID NO: 15), R:GCTCCCAGCCATTGATACAG (SEQ ID NO: 16)), SPON1 (F:CCTGCGGAACTGCCAAGTA(SEQ ID NO: 17), R:CACGGGTGAGCCCAATTCT (SEQ ID NO: 18)), POSTN (F:TTTGGGCACCAAAAAGAAAT (SEQ ID NO: 19), R:TTCTCATATAACCAGGGCAACA (SEQ ID NO: 20)). qPCRs were run in duplicates using a LightCycler^{®} 480 (Roche^{®}). Normalization was performed using the geometric mean obtained from three housekeeping genes: RPL13A (F:CATAGGAAGCTGGGAGCAAG (SEQ ID NO: 21), R:GCCCTCCAATCAGTCTTCTG (SEQ ID NO: 22)), TBP (F:AACAACAGCCTGCCACCTTA (SEQ ID NO: 23), R:GCCATAAGGCATCATTGGAC (SEQ ID NO: 24)), HPRT (F:CCCTGGCGTCGTGATTAGT (SEQ ID NO: 25), R: GTGATGGCCTCCCATCTCCTT (SEQ ID NO: 26)). Comparison between the different MSC-derived cells products from the same donors were performed by calculating the gene expression (fold change) using the 2-ΔΔCt method for each gene of interest (Schmittgen and Livak, 2008, 3(6), 1101-8; Nature Protocols, 3(6), 1101-1108). Statistical analysis was performed using JMP^{®} (13.1.0) software. RT-qPCR data expressed in fold change were log transformed and Student tests (with α=0.05) were performed to evaluate the statistical significance of differences observed between cell types. Statistical significance was graphically represented depending on the p-value (p) obtained: * for p<0.05, ** for p<0.01, and *** for p<0.001.

### Multiplex assay

After harvesting, cells were plated at a density of 50,000 cells/cm². After 48 hours of incubation at 37°C in a humidified atmosphere containing 5% CO₂, cell culture supernatants were harvested, centrifuged (5 min at 1500 rpm at room temperature) and stored at -80°C. Supernatants were analysed by Luminex^{®} assay using Human Magnetic Luminex^{®} Assays (R&D System^{®}). The premixed Multiplex was custom-made (R&D System^{®}). The following secreted factors were investigated: BMP-2, COL1A1, MMP13, OPN, OPG, SPARC, RANKL, CHI3L1. The assay was performed following manufacturer's instructions and the analyses were performed using MAGPIX^{®} (R&D System^{®}) and the Bio-Plex Manager 5.0TM Software (Bio-Rad^{®}).

### Cell size measurement

Comparative cell products (i.e. MSC and MSC-derived cells: Cell products A and B) and cell products illustrating the invention (i.e. MSC-derived cells of chondro-osteoblastic lineage: cell products C) obtained as described in Comparative Example 1 and Example 1, were harvested and suspended in PBS with 0.4% trypan blue at a cell density of 12.5×10⁶ cells/ml. Ten µl of the cell suspension were placed on a graduated slide (Motic^{®}) and then protected by a coverslip to be placed under an inverted microscope at magnification 40X (AE31; Motic). Images taken with a camera (Moticam, Motic^{®}) placed on the microscope were analysed by Motic Image Plus^{®} 2.02 software in order to measure cell diameters. At least 100 cells were measured to consider the analysis statistically significant.

The size of the cells obtained at different times of the *ex vivo* culture was also analysed by flow cytometry (BD FACSCanto^{™} II and the BD FACSDiva^{™} software; Becton Dickinson). Briefly, at day 21, 23, 26 and 28 after initiation of the *ex vivo* cell culture as described in Example 1 or Comparative Example 1, cells were harvested, suspended in phosphate-buffered saline (PBS) at a cell density of 1×10⁶ cells/ml and analysed with the flow cytometer for forward scatter (FSC) measurement (expressed in relative fluorescence unit). Forward scatter measures scattered light in the direction of the laser path, and therefore gives a relative size for the cells passing through the flow chamber.

### Results

### Culture yields

The method illustrating the invention significantly increased the allogeneic cells availability (i.e. the number of cells obtained from one bone marrow donation) for clinical use as the global culture yields were dramatically increased (Table 2, cell product C fresh versus cell products A and B).

**Table 2: Respective culture yields of comparative cell products (i.e. cell product A, cell product B) and cell products illustrating the invention (i.e. cell products C)**

| | **Cell product A** | **Cell product B** | **Cell product C fresh** |
|---|---|---|---|
| Primary culture yield | 63 ± 168% (n=93) | 139 ± 91% (n= | 212 ± 74% (n= 6) |

| | | 57) | |
|---|---|---|---|
| Secondary culture yield (first passage at Day 14) | 1,656 ± 1,126% (n=183) | 25,254 ± 7,547% (n= 41) | 17,837 ± 5,973% (n=6) |
| Third culture yield | NA | NA | 10,641% ± 4,295 (n=6) |
| Global yield* | 1,068 ± 2,353% (n=183) | 46,768 ± 33,201% (n= 41) | 5,145,960 ± 6,103,554% (n=6) |
| Cumulative (theoretical) PDL (from D0 to end of manufacturing process) | 16 ± 2 | 22 ± 2 | 28 ± 1 |

| | | | |
|---|---|---|---|
| *Mean* ± *SD, taking into account variability between batches including variability due to donor effect (variability between bone marrow starting materials); PDL: Population doubling level is the number of time cell number was doubled;* * *Global yield takes into account the accumulation of cultures yields from all the successive culture (from cell plating at Day 0 to the end of the manufacturing process and generation of the MSC-derived cells); NA : not available* | | | |

### Cell marker expression profile

Flow cytometry analysis revealed that the general cell identity based on the cell surface marker expression profiles of cell product A, cell product B (generated with comparative methods according to the prior art), and cell products C with or without final cryopreservation (generated with a method illustrating the invention) were comparable.

All of them expressed the mesenchymal markers CD73, CD90 and CD105 and did not express the hematopoietic markers CD45, CD34 and CD3 (less than 5% of the cell population expressed these markers) (Table 3). Cell product B and cell products C (with or without final cryopreservation) (i) expressed low levels of MHC class II cell surface receptor such as the HLA-DR and (ii) highly expressed ALP (Tables 3 and 4). Weak immunogenicity represented by weak expression of HLA-DR advantageously allows cell transplantation for instance to allogeneic subjects (Table 3). In addition, cell product A, cell product B, and cell products C (with or without final cryopreservation) highly expressed the adhesion marker CD49e and the enzyme ALP on their surface compared to undifferentiated MSCs (Tables 3 and 4). The high expression of ALP highlights the commitment of cell product A, cell product B, and cell products C (with or without final cryopreservation) toward the osteoblastic lineage.

**Table 3: Cell surface marker expression profile of comparative cell products (i.e. MSC, cell product A, cell product B) and cell products illustrating the invention (i.e. cell products C)**

| **Marker expression (in %)** | **Statistics** | **MSCs** | **Cell product A** | **Cell product B** | **Cell product C fresh** | **Cell product C cryopreserved** | | | |
|---|---|---|---|---|---|---|---|---|---|
| | | | | | | **CS10** | **CS10 diluted HSA 1:1** | **CS10 5%HSA** | **HTS 10%DMSO 10%HSA** |
| **CD73-APC** | **Mean** | 100.0 | 100.0 | 100.0 | 100.0 | 100.0 | 100.0 | 100.0 | 100.0 |
| | **Std Dev** | 0.0 | 0.0 | 0.0 | 0.1 | 0.0 | 0.0 | 0.0 | 0.0 |
| | **N** | 6 | 11 | 22 | 15 | 10 | 2 | 3 | 3 |
| **CD90-PE** | **Mean** | 100.0 | 99.9 | 99.9 | 99.7 | 99.9 | 99.5 | 99.9 | 99.8 |
| | **Std Dev** | 0.1 | 0.2 | 0.2 | 0.6 | 0.2 | 0.7 | 0.2 | 0.4 |
| | **N** | 8 | 12 | 22 | 18 | 10 | 2 | 3 | 3 |
| **CD105-APC** | **Mean** | 100.0 | 99.8 | 100.0 | 99.9 | 100.0 | 100.0 | 100.0 | 100.0 |
| | **Std Dev** | 0.0 | 0.5 | 0.1 | 0.3 | 0.0 | 0.0 | 0.0 | 0.0 |
| | **N** | 8 | 12 | 20 | 18 | 10 | 2 | 3 | 3 |
| **CD45-FITC** | **Mean** | ND | ND | ND | 1.3 | 1.0 | 0.9 | 0.6 | 1.0 |
| | **Std Dev** | | | | 0.7 | 0.3 | 0.0 | 0.3 | 0.1 |
| | **N** | 0 | 0 | 0 | 16 | 10 | 2 | 3 | 3 |
| **CD45-APC** | **Mean** | 0.4 | 0.3 | 1.0 | ND | ND | ND | ND | ND |
| | **Std Dev** | 0.2 | 0.2 | 2.9 | | | | | |
| | **N** | 8 | 12 | 19 | 0 | 0 | 0 | 0 | 0 |
| **CD34-APC** | **Mean** | 0.6 | 1.0 | 1.6 | 2.1 | 1.6 | 2.8 | 3.3 | 1.5 |
| | **Std Dev** | 0.4 | 0.6 | 1.8 | 1.6 | 0.9 | 2.2 | 2.2 | 1.0 |
| | **N** | 8 | 12 | 22 | 16 | 10 | 2 | 3 | 3 |
| **CD3-PE** | **Mean** | 0.2 | 0.1 | 0.2 | 0.0 | 0.1 | 0.1 | 0.0 | 0.0 |
| | **Std Dev** | 0.1 | 0.1 | 0.1 | 0.1 | 0.1 | | 0.1 | 0.1 |
| | **N** | 6 | 10 | 17 | 16 | 10 | 1 | 3 | 3 |
| **HLA-DR-PE** | **Mean** | 0.7 | 1.0 | 1.8 | 1.6 | 0.9 | 1.4 | 1.4 | 1.4 |
| | **Std Dev** | 1.2 | 0.6 | 2.0 | 1.8 | 0.7 | 0.8 | 0.8 | 0.9 |
| | **N** | 8 | 12 | 22 | 16 | 10 | 2 | 3 | 3 |
| **HLA-DR/DP/DQ-FITC** | **Mean** | 1.0 | 1.6 | 1.6 | 2.0 | 1.5 | 1.8 | 1.8 | 1.6 |
| | **Std Dev** | 0.4 | 1.1 | 1.1 | 1.6 | 0.7 | 0.6 | 0.8 | 0.2 |
| | **N** | 8 | 12 | 22 | 16 | 10 | 2 | 3 | 3 |
| **ALP-PE** | **Mean** | 40.7 | 88.7 | 94.8 | 96.2 | 96.2 | 97.7 | 98.7 | 98.4 |
| | **Std Dev** | | 5.6 | 6.6 | 4.4 | 3.6 | 2.0 | 0.9 | 1.7 |
| | **N** | 1 | 5 | 10 | 18 | 10 | 3 | 2 | 3 |
| **CD49e-PE** | **Mean** | 92.7 | 99.6 | 99.8 | 99.9 | 100.0 | 99.9 | 99.8 | 99.9 |
| | **Std Dev** | 20.5 | 1.1 | 0.5 | 0.4 | 0.1 | 0.2 | 0.4 | 0.2 |
| | **N** | 8 | 12 | 19 | 18 | 10 | 3 | 2 | 3 |
| **CD44-PE** | **Mean** | 99.9 | 99.7 | 100.0 | 100.0 | 100.0 | 100.0 | 99.9 | 100.0 |
| | **Std Dev** | 0.2 | 0.5 | 0.0 | 0.1 | 0.0 | 0.1 | 0.2 | 0.1 |
| | **N** | 8 | 12 | 22 | 18 | 10 | 3 | 2 | 3 |
| **CD10-PE** | **Mean** | 19.6 | 99.6 | 98.8 | 99.3 | 99.5 | 99.3 | 99.1 | 98.9 |
| | **Std Dev** | 14 | 0.4 | 1.5 | 16 | 0.5 | 0.6 | 0.4 | 0.7 |
| | **N** | 10 | 12 | 25 | 16 | 10 | 2 | 3 | 3 |

| | | | | | | | | | |
|---|---|---|---|---|---|---|---|---|---|
| *Abbreviations: ALP: alkaline phosphatase; APC: allophycocyanin; FITC: fluorescein isothiocyanate; HLA-DR: human leukocyte antigen* - *DR isotype; HLA-DR*/*DP*/*DQ: human leukocyte antigen* - *DR*/*DP*/*DQ isotypes; MSC: mesenchymal stem cells; ND: not determined; PE: phycoerythrin; SD: standard deviation* | | | | | | | | | |

**Table 4: ALP expression levels of comparative cell products (i.e. MSC, cell product A, cell product B) and cell products illustrating the invention (i.e. cell products C)**

| | **Statistics** | **MSCs** | **Cell product A** | **Cell product B** | **Cell product** C **fresh** | **Cell product C cryopreservation (also referred to as Cell product C cryopreserved)** | | | |
|---|---|---|---|---|---|---|---|---|---|
| | | | | | | **CS10** | **CS10 diluted HSA 1:1** | **CS10 5%HSA** | **HTS 10%DMSO 10%HSA** |
| **ALP-PE population positivity (%)** | **Mean** | 40.7 | 88.7 | 94.8 | 96.2 | 96.2 | 98.7 | 97.7 | 98.4 |
| | **Std Dev** | | 5.6 | 6.6 | 4.4 | 3.6 | 0.9 | 2.0 | 1.7 |
| | **N** | 1 | 5 | 10 | 18 | 10 | 2 | 3 | 3 |
| **ALP-PE cell surface expression level (nMFI)** | **Mean** | 2.4 | 19.8 | 56.1 | 60.3 | 38.0 | 57.7 | 42.7 | 41.2 |
| | **Std Dev** | | 10.8 | 27.4 | 38.9 | 24.2 | 43.1 | 37.2 | 31.3 |
| | **N** | 1 | 5 | 10 | 18 | 10 | 2 | 3 | 3 |
| **ALP enzymatic activity (mU/mg of total protein)** | **Mean** | 176.3 | 671.9 | 874.7 | 895.5 | 801.5 | ND | 719.9 | 633.6 |
| | **Std Dev** | 252.9 | 305.8 | 772.9 | 387.3 | 351.3 | | 277.0 | 263.9 |
| | **N** | 3 | 9 | 26 | 12 | 5 | 0 | 2 | 2 |

| | | | | | | | | | |
|---|---|---|---|---|---|---|---|---|---|
| *Abbreviations: ALP: alkaline phosphatase; ND: not determined; PE: phycoerythrin* | | | | | | | | | |

The cell surface marker expression profile was not only characterized by the presence of markers on cell surface (population positivity percentage) but also by analysing the quantity of markers expressed on cell surface (population normalized median of fluorescence) of different markers. These analyses highlighted some differences between the different MSC-derived cells.

Cell product B and cell products C (with or without final cryopreservation) cultured in presence of heparin expressed higher level of ALP than MSCs and cell product A cultured in absence of heparin (ALP-PE nMFI results) strengthening their commitment toward the osteoblastic lineage of bone-forming cells.

The expression of the mesenchymal markers CD73 and CD105 on cell surface were also dependent on the cell types. Cell products generated in presence of heparin (cell product B and cell products C with or without final cryopreservation) expressed higher level of CD73 and CD105 than cell product A. In addition, cell products C seemed to possess more CD73 and CD105 on their surface than cell product B especially when cell product C did not undergo a final cryopreservation (Table 5). Differentiated cell products A, B and C express higher amount of cell marker CD10 than undifferentiated MSC. In addition, cell products C possess more CD10 on their surface than cells products A and B (Table 5).

The nMFI flow cytometry analysis revealed that CD73 and CD44 protein expressions were more elevated in cell product C than in the other cell types (Figure 1).

**Table 5: Additional cell surface marker expression results of comparative cell products (i.e. MSC, cell product A, cell product B) and cell products illustrating the invention (i.e. cell products C)**

| **Marker expression (in nMFI)** | **Statistics** | **MSCs** | **Cell product A** | **Cell product B** | **Cell product C fresh** | **Cell product C cryopreservation (also referred to as cell product C cryopreserved)** | | | |
|---|---|---|---|---|---|---|---|---|---|
| | | | | | | **CS10** | **CS10 diluted HSA 1:1** | **CS10 5%HSA** | **HTS 10%DMSO 10%HSA** |
| **ALP-PE** | **Mean** | 2.4 | 19.8 | 56.1 | 60.3 | 38.0 | 57.7 | 42.7 | 41.2 |
| | **Std Dev** | / | 10.8 | 27.4 | 38.9 | 24.2 | 43.1 | 37.2 | 31.3 |
| | **N** | 1 | 5 | 10 | 18 | 10 | 2 | 3 | 3 |
| **CD73-APC** | **Mean** | 234.8 | 130.7 | 646.3 | 996.9 | 703.9 | 777.8 | 719.2 | 784.2 |
| | **Std Dev** | 84.3 | 80.1 | 138.8 | 181.1 | 150.0 | 73.3 | 90.6 | 47.3 |
| | **N** | 6 | 11 | 22 | 15 | 10 | 2 | 3 | 3 |
| **CD105-APC** | **Mean** | 207.7 | 26.6 | 59.1 | 99.7 | 70.2 | 74.6 | 72.6 | 67.0 |
| | **Std Dev** | 67.6 | 15.2 | 13.1 | 27.0 | 13.1 | 2.3 | 4.2 | 3.9 |
| | **N** | 8 | 12 | 20 | 18 | 10 | 2 | 3 | 3 |
| **CD44-PE** | **Mean** | 139.8 | 62.0 | 156.6 | 362.8 | 378.4 | 185.5 | 227.5 | 261.3 |
| | **Std Dev** | 57.5 | 19.1 | 40.7 | 250.4 | 205.3 | 19.6 | 80.1 | 147.7 |
| | **N** | 8 | 12 | 22 | 18 | 10 | 2 | 3 | 3 |
| **CD49e-PE** | **Mean** | 81.0 | 22.5 | 33.5 | 44.3 | 39.6 | 35.7 | 35.2 | 36.87 |
| | **Std Dev** | 51.4 | 9.9 | 11.0 | 8.0 | 7.4 | 3.3 | 3.5 | 10.0 |
| | **N** | 8 | 12 | 19 | 18 | 10 | 2 | 3 | 3 |
| **HLA-ABC-FITC** | **Mean** | 26.1 | 21.6 | 80.2 | 115.7 | 104.7 | 71.5 | 79.5 | 70.1 |
| | **Std Dev** | / | 5.2 | 17.4 | 22.0 | 24.9 | 27.2 | 22.1 | 18.0 |
| | **N** | 1 | 4 | 8 | 16 | 10 | 2 | 3 | 3 |
| **CD10-PE** | **Mean** | 0.8 | 36.2 | 32.2 | 64.0 | 59.3 | 63.8 | 64.1 | 57.9 |
| | **Std Dev** | 1.1 | 16.4 | 16.8 | 38.5 | 30.5 | 45.5 | 35.4 | 32.9 |
| | **N** | 8 | 12 | 22 | 18 | 10 | 2 | 3 | 3 |

| | | | | | | | | | |
|---|---|---|---|---|---|---|---|---|---|
| *Abbreviations: ALP: alkaline phosphatase; APC: allophycocyanin; FITC: fluorescein isothiocyanate; HLA-ABC: human leukocyte antigen ABC; HLA-DR: human leukocyte antigen - DR isotype; MSC: mesenchymal stem cells; NA: not available; ND: not determined; PE: phycoerythrin; SD: standard deviation* | | | | | | | | | |

### RT-qPCR and multiplex assay

The analysis revealed that gene RUNX2 in cell product C fresh was significantly (*) overexpressed compared to MSC but was significantly (*) down regulated compared to cell product B (Table 6a). MMP13 gene in cell product C fresh was significantly (*) up-regulated compared to MSCs and cell product A but its expression remained significantly (*) higher in cell product B (Table 6a).

On the other hand, the genes SPARC and KI67 were significantly (**) down regulated in cell product C fresh compared to all the other cell types (Table 6a). The gene PPARG was equally expressed for cell product C fresh and MSC and significantly (***) down-regulated compared to cell products A and B (Table 6a).

Furthermore, genes BMP2, SOX9, MMP13 and ALPL in cell product C cryopreserved were significantly overexpressed compared to MSC (Table 6b), indicating their engagement into the chondro-osteogenic lineage.

**Table 6a: Gene expression profile of comparative cell products (i.e. MSC, cell product A, cell product B) and cell products illustrating the invention (i.e. cell product C fresh) (expressed in fold change relative to mean MSCs values - statistical significance is graphically represented depending on the p-value (p) obtained: * for p<0.05, ** for p<0.01, and *** for p<0.001, NS: not statistically significant)**

| | **Gene expression** | **Statistics** | **MSC** | **Cell product A** | **Cell product B** | **Cell product C fresh** |
|---|---|---|---|---|---|---|
| **Mesenchymal markers** | **CD73** | **Mean** | 1.000 | 0.367 | 1.080 | 1.336 |
| | | **SD** | | 0.058 | 0.217 | 0.665 |
| | | **N** | 1 | 3 | 5 | 16 |
| | **CD105** | **Mean** | 1.000 | 0.533 | 0.500 | 0.397 |
| | | **SD** | | 0.115 | 0.100 | 0.033 |
| | | **N** | 1 | 3 | 5 | 4 |
| **Differentiation master genes** | **RUNX2** | **Mean** | 1.029 | 1.467 | 1.661 | 1.354 |
| | | **SD** | 0.287 | 0.274 | 0.425 | 0.364 |
| | | **N** | 7 | 9 | 28 | 16 |
| | **SOX9** | **Mean** | 1.143 | 2.967 | 2.057 | 2.743 |
| | | **SD** | 0.735 | 1.434 | 0.861 | 1.030 |
| | | **N** | 7 | 9 | 28 | 16 |
| | **PPARG** | **Mean** | 1.186 | 6.922 | 3.086 | 0.696 |
| | | **SD** | 0.760 | 2.142 | 1.848 | 0.393 |
| | | **N** | 7 | 9 | 28 | 4 |
| | **ZNF521** | **Mean** | 1.471 | 49.833 | 63.586 | 63.081 |
| | | **SD** | 1.174 | 29.088 | 23.485 | 32.999 |
| | | **N** | 7 | 9 | 28 | 16 |
| | **DKK1** | **Mean** | 1.000 | 0.000 | 0.000 | 0.018 |
| | | **SD** | | 0.000 | 0.000 | 0.004 |
| | | **N** | 1 | 3 | 5 | 4 |
| **Extracellular matrix-related markers** | **SPON1** | **Mean** | 1.083 | 576.244 | 546.964 | 496.801 |
| | | **SD** | 0.431 | 397.782 | 343.407 | 167.873 |
| | | **N** | 6 | 9 | 28 | 3 |
| | **COL1A1** | **Mean** | 1.014 | 2.089 | 0.896 | 0.949 |
| | | **SD** | 0.291 | 0.417 | 0.403 | 0.295 |
| | | **N** | 7 | 9 | 28 | 4 |
| | **BGN** | **Mean** | 1.014 | 2.189 | 1.279 | 1.654 |
| | | **SD** | 0.069 | 0.372 | 0.347 | 0.498 |
| | | **N** | 7 | 9 | 28 | 4 |
| | **SPARC** | **Mean** | 1.029 | 2.222 | 0.914 | 0.695 |
| | | **SD** | 0.150 | 0.576 | 0.318 | 0.495 |
| | | **N** | 7 | 9 | 28 | 16 |
| | **IBSP** | **Mean** | 1.114 | 8.244 | 14.848 | 4.457 |
| | | **SD** | 0.467 | 11.279 | 15.446 | 4.085 |
| | | **N** | 7 | 9 | 27 | 4 |
| | **ALPL** | **Mean** | 1.500 | 13.833 | 9.096 | 11.813 |
| | | **SD** | 1.615 | 5.980 | 6.777 | 3.895 |
| | | **N** | 7 | 9 | 28 | 4 |
| | **BMP2** | **Mean** | 1.029 | 10.767 | 33.489 | 24.517 |
| | | **SD** | 0.275 | 6.210 | 25.988 | 19.700 |
| | | **N** | 7 | 9 | 28 | 16 |
| | **CHI3L1** | **Mean** | 1.857 | 430.189 | 770.104 | 503.885 |
| | | **SD** | 2.202 | 309.051 | 470.030 | 447.824 |
| | | **N** | 7 | 9 | 28 | 16 |
| | **MMP13** | **Mean** | 1.329 | 216.256 | 2827.021 | 1289.936 |
| | | **SD** | 1.246 | 254.257 | 2900.256 | 1572.880 |
| | | **N** | 7 | 9 | 28 | 16 |
| | **CD10** | **Mean** | 1.000 | 62.633 | 63.940 | 125.704 |
| | | **SD** | | 14.459 | 30.435 | 78.670 |
| | | **N** | 1 | 3 | 5 | 4 |
| | **KI67** | **Mean** | 1.271 | 0.122 | 0.143 | 0.097 |
| | | **SD** | 1.131 | 0.259 | 0.140 | 0.053 |
| | | **N** | 7 | 9 | 28 | 16 |
| | **PCNA** | **Mean** | 1.086 | 0.633 | 0.611 | 0.623 |
| | | **SD** | 0.515 | 0.087 | 0.238 | 0.119 |
| | | **N** | 7 | 9 | 28 | 4 |
| **Apoptosis-associated markers** | **BCL2** | **Mean** | 1.071 | 3.426 | 0.993 | 0.944 |
| | | **SD** | 0.454 | 0.968 | 0.440 | 0.238 |
| | | **N** | 7 | 9 | 28 | 4 |
| | **BAX** | **Mean** | 1.014 | 1.633 | 1.957 | 1.185 |
| | | **SD** | 0.121 | 0.180 | 2.468 | 0.126 |
| | | **N** | 7 | 9 | 28 | 4 |

| | | | | | | |
|---|---|---|---|---|---|---|
| *SD: standard deviation* | | | | | | |

**Table 6b: Gene expression profile of comparative cell products (i.e. MSC) and cell products illustrating the invention (i.e. cell product C cryopreserved) (expressed in fold change relative to mean MSCs values)**

| | **Gene expression** | **Statistics** | **MSC** | **Cell product C cryopreserved** |
|---|---|---|---|---|
| **Mesenchymal markers** | **CD73** | **Mean** | 1.065 | 1.394 |
| | | **SD** | 0.461 | 0.719 |
| | | **N** | 2 | 6 |
| | **CD105** | **Mean** | 1.039 | 0.397 |
| | | **SD** | 0.439 | 0.217 |
| | | **N** | 2 | 6 |
| **Differentiation master genes** | **RUNX2** | **Mean** | 1.041 | 1.360 |
| | | **SD** | 0.539 | 0.659 |
| | | **N** | 8 | 6 |
| | **SOX9** | **Mean** | 1.170 | 3.163 |
| | | **SD** | 0.801 | 1.603 |
| | | **N** | 8 | 6 |
| | **PPARG** | **Mean** | 1.162 | 0.853 |
| | | **SD** | 0.829 | 0.863 |
| | | **N** | 8 | 6 |
| | **ZNF521** | **Mean** | 1.400 | 65.271 |
| | | **SD** | 1.076 | 41.226 |
| | | **N** | 8 | 6 |
| | **DKK1** | **Mean** | 1.158 | 0.029 |
| | | **SD** | 0.536 | 0.019 |
| | | **N** | 2 | 6 |
| **Extracellular matrix-related markers** | **SPON1** | **Mean** | 1.074 | 330.737 |
| | | **SD** | 0.626 | 256.467 |
| | | **N** | 7 | 6 |
| | **COL1A1** | **Mean** | 1.055 | 1.149 |
| | | **SD** | 0.594 | 0.742 |
| | | **N** | 8 | 6 |
| | **BGN** | **Mean** | 1.018 | 1.357 |
| | | **SD** | 0.513 | 0.701 |
| | | **N** | 8 | 6 |
| | **SPARC** | **Mean** | 3.755 | 4.093 |
| | | **SD** | 2.224 | 2.739 |
| | | **N** | 8 | 6 |
| | **IBSP** | **Mean** | 1.092 | 6.072 |
| | | **SD** | 0.662 | 5.551 |
| | | **N** | 8 | 6 |
| | **ALPL** | **Mean** | 1.418 | 10.558 |
| | | **SD** | 1.344 | 10.476 |
| | | **N** | 8 | 6 |
| | **BMP2** | **Mean** | 1.040 | 14.338 |
| | | **SD** | 0.545 | 8.558 |
| | | **N** | 8 | 6 |
| | **CHI3L1** | **Mean** | 1.710 | 404.507 |
| | | **SD** | 1.738 | 291.544 |
| | | **N** | 8 | 6 |
| | **MMP13** | **Mean** | 1.346 | 701.802 |
| | | **SD** | 1.290 | 721.829 |
| | | **N** | 8 | 6 |
| | **CD10** | **Mean** | 1.071 | 60.284 |
| | | **SD** | 0.466 | 39.484 |
| | | **N** | 2 | 6 |
| | **KI67** | **Mean** | 1.261 | 0.118 |
| | | **SD** | 1.115 | 0.089 |
| | | **N** | 8 | 6 |
| | **PCNA** | **Mean** | 1.084 | 0.659 |
| | | **SD** | 0.645 | 0.340 |
| | | **N** | 8 | 6 |
| **Apoptosis-associated markers** | **BCL2** | **Mean** | 1.067 | 0.866 |
| | | **SD** | 0.612 | 0.467 |
| | | **N** | 8 | 6 |
| | **BAX** | **Mean** | 1.011 | 1.287 |
| | | **SD** | 0.491 | 0.653 |
| | | **N** | 8 | 6 |

| | | | | |
|---|---|---|---|---|
| *SD: standard deviation* | | | | |

### Cell size

The cell size measurements using (i) Motic Image Plus^{®} 2.0 software and (ii) flow cytometry FSC analysis confirmed that cell product B, cell product C fresh and cell products C cryo were smaller and more homogeneous than cell product A (Table 7, Figure 2).

Very interestingly, the large majority of cell products C (at least 90 %) did not exceed 25 µm diameter and less than 1% of them exceed 35 µm diameter. In contrast, the cell product A included only 35.4% of cells which do not exceed 25 µm diameter and 26.9% of cells with a diameter higher than 35 µm (Table 8, Figure 2). The cell product B included 73.7% of cells which do not exceed 25 µm diameter and 3.3% of cells with a diameter higher than 35 µm (Table 8, Figure 2).

**Table 7: Diameter of comparative cell products (i.e. MSC, cell product A, cell product B) and cell products illustrating the invention (i.e. cell products C)**

| **Cell diameter** (**µm**) | **Mean ± SD** | **Min** - **max** | **N** |
|---|---|---|---|
| **MSCs** | 19.2 ± 4.8 | 9.8 - 41.8 | 450 |
| **Cell product A** | 30.2 ± 9.9 | 11.4 - 67 | 1205 |
| **Cell product B** | 22.4 ± 6.4 | 7.9 - 74.5 | 1744 |
| **Cell product C - fresh** | 17.6 ± 7.3 | 7.3 - 44.3 | 2238 |
| **Cell product C cryo CS10** | 17.9 ± 4.8 | 9.1 - 53.8 | 876 |
| **Cell product C cryo CS10 diluted HSA 1:1** | 18.3 ± 3.9 | 10.9 - 33.8 | 209 |
| **Cell product cryo CS10 5%HSA** | 18.7 ± 4.3 | 11.2 - 35.7 | 339 |
| **Cell product C cryo** | 19.7 ± 3.5 | 12.9 - 31.4 | 361 |
| **HTS 10%HSA 10%DMSO** | | | |

**Table 8: Distribution of cell size of comparative cell products (i.e. MSC, cell product A, cell product B) and cell products illustrating the invention (i.e. cell products C)**

| **Cell with a diameter** | **≤ 25 µm** | **> 35 µm** |
|---|---|---|
| **MSCs** | 89.9% | 1.3% |
| **Cell product A** | 35.4% | 26.9% |
| **Cell product B** | 73.7% | 3.3% |
| **Cell product C - fresh** | 94.6% | 0.9% |
| **Cell product C cryo CS10** | 91.3% | 0.3% |
| **Cell product C cryo CS10 diluted HSA 1:1** | 94.3% | 0.0% |
| **Cell product C cryo CS10 5%HSA** | 92.3% | 0.3% |
| **Cell product C cryo** | 92.0% | 0.0% |
| **Cell with a diameter** | **≤ 25 µm** | **> 35 µm** |
| **HTS 10%HSA 10%DMSO** | | |

### Example 3: In vivo bone formation of MSC-derived cells of chondro-osteoblastic lineage obtained by the method of Example 1

### Material and methods

### Cells

The cell product illustrating the invention (i.e. cell product C cryo) were obtained as described in Example 1.

### Mice

Female NMRI-Nude (nu/nu) mice of 10-11 weeks were purchased from Janvier S.A.S. (Le Genest-St-Isle, France) and housed in standard conditions with food and water *ad libitum.*

### Calvaria bone formation mouse model

Twelve-week-old female NMRI-Nude (nu/nu) mice were anesthetized with isoflurane (IsoFlo^{®}) and received a single subcutaneous administration of cell product C cryo (2.5 × 10⁶ cells in 100 µl per mouse) or excipient (100 µl) over the calvaria bone. To label bone neo-formation over time, calcium-binding fluorochromes were sequentially administered to mice. Alizarin red (red), calceins (green and blue) and tetracycline (yellow) (all from Sigma-Aldrich^{®}) were injected intraperitoneally 2 or 3 days before and 5, 12, and 19 days after cell administration, respectively. Experimental animals were monitored for body weight, general clinical signs, and clinical signs at site of administration for 4 weeks following the administration. Mice were euthanized 4 weeks after cell administration by cervical dislocation and the calvaria of each mouse was harvested to assess bone formation properties of bone-forming cells by X-ray imaging, histomorphometry (quantification of bone formation) and immunofluorescence.

### Quantification of bone formation by X-ray analysis

At euthanasia, *ex vivo* X-ray imaging of the calvaria of each mouse placed side by side was performed using the Faxitron^{®} MX-20 device. Digital images were taken at a 1.5X magnification in manual mode with voltage set at 35 kV, exposure time at 4.8 sec, brightness/contrast at 8300/6000. The X-ray images generated are grey level images with grey intensity values ranging from 0 (black region) to 255 (white region) and are directly proportional to radio-opacity and therefore to bone opacity or bone thickness. The grey level intensity value of the osteoinduction part of the bone formation (mineralized nodules discarded from the selection) on parietal bones (manual selection) was analyzed using histogram tool of AdobePhotoshop^{®} software.

X-ray imaging and AdobePhotoshop^{®} software were also used to quantify the surface of mineralized nodules (manual selection).

### Sample embedding and histological sectioning

For histomorphometry, ALP, TRAP (tartrate-resistant acid phosphatase), Masson Trichrome Goldner stainings and immunofluorescence, calvarias were fixed and dehydrated with successive incubations in 70%, 80% and 90% ethanol bath, for 12 hours each, at 4°C with gentle shaking, and embedded in hydroxyethylmethacrylate (HEMA) plastic resin (HistoResin, Leica^{®}). Four micron-thick and 8 µm-thick coronal sections were cut using a microtome (Leica^{®}, RM2255).

### Immunofluorescence staining

Assessment of the human and murine collagen I by immunofluorescence was performed on 4 µm-thick coronal plastic histological sections of calvaria. Briefly, after a step of permeabilization using a solution of PBS 1X/Triton 0.3% for 30 min at room temperature (RT), the histological sections were incubated for 1 hour at RT in the blocking solution (i.e. PBS/BSA/horse serum/Triton^{™}) to sature non-specific binding sites. The histological slides were then incubated overnight at 4°C with mouse anti-human and rabbit anti-murine collagen I primary antibodies (Abcam; #ab138492 and Abcam; #ab21286 respectively). After 3 steps of rinsing in PBS for 5 min at RT, blocking was realized with the blocking solution for 1 hour at RT. The secondary antibodies diluted in the blocking solution was then added for 2 hours at RT protected from the light. The secondary antibody Alexa Fluor^{®} 488 Donkey anti-rabbit IgG H&L (ThermoFisher, #A21206) and Alexa Fluor^{®} Cy3^{®} Goat anti-mouse IgG H&L (Abcam; #ab97035) were used to visualize the murine collagen I in green and the human collagen I in red, respectively. The slides were then rinsed 3 times in PBS 1X for 5 min at RT and incubated with NucBlue^{®} solution for 1 min at RT to stain the nucleus. Finally, the slides were briefly rinsed once in PBS then mounted in GlycerGel^{®} reagent. As negative control of immunofluorescence, omission of primary antibody was performed on adjacent histological slide.

### Histological staining

Osteoblastic and osteoclastic activities were assessed on calvaria sections respectively using ALP and TRAP enzymatic activity detection methods respectively. For ALP staining, 4 µm-thick calvaria coronal plastic sections were incubated for 1 hour with a solution of Fast Blue RR Salt (Sigma-Aldrich^{®}) and Naphtol AS-MX phosphate alkaline (Sigma-Aldrich^{®}). TRAP staining was performed on 8 µm-thick calvaria coronal plastic sections using the Acid Phosphatase, Leukocyte (TRAP) Kit (Sigma-Aldrich^{®}) according to manufacturer's instructions. To assess the status of mineralization of the neo-formed bone, Masson Trichrome Goldner staining was performed on the calvaria sections stained with ALP using a kit (Bio-Optica^{®}) according to manufacturer's instructions. Digital images were taken with an optical microscope (Leica^{®}) and the Leica^{®} LAS EZ software.

### Histomorphometrical analyses of calvarias

Quantification of bone formation (i.e. absolute bone formation) was performed on plastic embedded tissues. Measures of the absolute neo-formed bone thickness (from basal mineralization front fluorescently labelled by alizarin red to bone neo-formation fluorescently labelled by calcein and tetracycline) with and without mineralized nodules were measured (in µm) on 4 µm-thick coronal section by ZEN^{®} image analysis software (Zeiss). For each animal, 4 measurements of absolute thicknesses were performed on 5 independent levels, with a distance of 200 µm between each level. As the first step, mean of thickness (without or without nodules) ± SD (i.e. mean of the 4 measures per level on the 5 levels) were calculated for each animal.

### Statistical analyses

Results were expressed as means ± standard deviation (SD). Statistical analyses were performed using JMP^{®} (SAS Institute Inc.) or GaphPad Prism^{®} software. Differences between groups were considered statistically significant when p<0.05.

### Results

Mice which have been administered cell products C cryo showed higher bone formation than controls 4 weeks after administration (Figure 8A-C). The bone opacity was significantly higher for bone-forming cells C cryo compared to excipient (Figure 8B). The surface of osteogeny was significantly higher compared to excipient in which no mineralized nodules were observed (Figure 8C). Histomorphometric measures of the osteoinduction with or without osteogeny (represented by the absolute bone formation) was significantly higher for bone-forming cells C cryo compared to excipient (Figure 8D-E). Also, in addition to osteoinduction activities, bone-forming cells C cryo promoted a high osteogenic activity highlighted by the presence of mineralized nodules. This osteogenic activity was observed in 4/5 bone marrow donors (or batch production) and 65% of mice (Figure 8F). One donor/batch was considered to be osteogenic (positive) when at least one mineralized nodule was observed in one mouse per group. No nodule was observed after excipient administration.

More particularly, cell products C cryo displayed both osteoinductive properties (homogenous bone formation from murine origin over the calvaria) and osteogenic properties (mineralized nodules from human and murine origin) (Figure 9).

Intramembranous host ossification was induced along the calvarial surface (Figures 9 and 10). More particularly, bone-forming cells C cryo displayed osteoinduction and osteogenic properties (Figure 10, "fluo"). Mouse/human type I collagen double-immunolabeling (Figure 10, "human type I collagen") revealed the presence of bone of host and donor origins (osteogeny). Osteoblast (Figure 10, "ALP+") and osteoclast (Figure 10, "TRAP") activities were mostly detected in mineralized nodules showing that the bone remodeling process in the nodules was still ongoing 4 weeks post-administration. This observation was dependent on the size of the nodule: the larger the nodule, the more ALP and TRAP activities are still present at 4 weeks post-administration. Weak osteoid (Figure 10, "Goldner's Masson trichrome staining") was highlighted indicating that the bone formation process is completed.

Accordingly, bone-forming cells C cryopreserved increased bone neo-formation.

This demonstrates the usefulness of cell products and cell composition as described in the specification and the examples for treatment of bone defects in flat bones as well as long bones.

### Example 4: In vivo mouse segmental femoral sub-critical size defect (sub-CSD) repaired by cell product C cryo obtained by the method of Example 1

### Experimental procedures

### Cells

The cell products illustrating the invention (i.e. cell product C cryo) are obtained as described in Example 1.

### Segmental femoral (sub)critical size defect (SFCSD) model

The surgical procedure was performed under aseptic conditions according to literature (Manassero et al., 2013, Tissue Engineering, Part C Methods, 19(4):271-80; Manassero et al., 2016, Journal of Visualized Experiments; (116): 52940). Briefly, 13-week-old female NMRI-Nude (nu/nu) mice were anaesthetized with isoflurane (IsoFlo^{®}) or with an intraperitoneal injection of a mix of dexmedetomidine hydrochloride (Dexdomitor^{®}, Orion Pharma, 1 mg/kg of body weight) and ketamine (Nimatek^{®}, Euronet, 150 mg/kg of body weight) and were placed in a ventral position on a warming plate. After applying a 6-hole PEEK micro-locking plate (RISystem AG^{®}) on the anterior side of the left femur, a 2-mm long mid-diaphyseal femoral osteotomy was performed using a Gigli saw and a jig (RISystem AG^{®}). As preventive medication, antibiotics (Baytril^{®}, 10 mg/kg of body weight) were administered the day before the surgery (in drinking water) and analgesic (buprenorphine hydrochloride, Temgesic^{®}, Schering-Plough, 0.1 mg/kg of body weight) was administered the day before the surgery and every 12 hours for at least 3 days following the surgery. MSC-derived cells C cryopreserved (1.25 × 10⁶ cells in a volume of 50 µl per mouse) or the excipient (control group) was administered on the day after the surgery, locally at the site of the bone defect, by percutaneous injection using a 100 µl-Hamilton^{®} syringe. Mice were euthanized 10 weeks after cell or excipient administration by cervical dislocation. The left femur of each mouse was dissected, harvested and kept in 0.9% NaCl at room temperature until X-ray imaging.

### Quantification of bone repair by X-ray analyses

*In vivo* X-ray imaging of the left femur of each mouse was performed, using the Faxitron^{®} MX-20 device just after the surgery to control the plate fixation, the segmental femoral defect size and to get a baseline, and every two weeks up to 10 weeks after administration of MSC-derived cells or excipient. Digital images were taken in mediolateral and anteroposterior views at a 5X magnification in manual mode with voltage set at 35 kV, exposure time at 4.8 sec, brightness at 4,300 and contrast at 7,100.

Efficacy was measured by 3 different methods, *i.e.* bone repair percentage, radiographic union score (RUS) adapted, and fusion score, every two weeks of the monitoring:
- The bone repair percentage was calculated by divided the repair defect size by the initial defect size. The defect size was quantified for each mouse over time by measuring the distance (µm) between the two edges of the bone defect at two (both cortices) locations on mediolateral and anteroposterior X-ray images (total of 4 measures), using ImageJ^{®} software. The mean of the four measurements was calculated for each mouse at each time point.
- The RUS (radiographic union score) adapted for the SFCSD model is a semi-quantitative measurement based on the presence or absence of a bone neo-formation, a bridging and a fracture line (from antero-posterior and medio-lateral radiographic images). The scoring corresponds to the sum of 4 scores determined at 2 cortical defect sites on both views (total of 4 scores ranging from 1 to 4 each). The scoring ranges therefore from 4 (no sign of healing) to 16 (complete fusion).
- Fusion score is a binary score which assess the fusion rate between the edges of the femoral defect. The radiological criteria utilized to define fusion is the visualization of bridging of the defect in at least 3 cortices (Cekiç E et al., Acta Orthop Traumatol Turc. 2014, 48(5), 533-40). The score is 0 (no fusion) or 1 (fusion). For this parameter, only the last time point was analysed (W10, here).

### Statistical analyses

Results are expressed as means ± standard deviation (SD). Statistical analyses comprising Two-way ANOVA repeated measures followed by a Bonferroni post-hoc test are performed using JMP^{®} (SAS Institute Inc.) or GraphPad Prism^{®} software. Differences between groups were considered statistically significant when p<0.05.

### Results

In the segmental femoral sub-CSD model, bone-forming cells C cryopreserved significantly improved and accelerated the percentage of bone fracture repair (Figures 11 and 12) compared to the excipient from 2 to 10 weeks after administration (p<0.001). Moreover, the RUS score was significantly increased for bone-forming cells C cryopreserved group compared to excipient group (Figure 13). Finally, the fusion rate was also improved with fusion for 9/19 (47%) of mice 10W after administration of bone-forming cells C cryopreserved compared to no fusion after excipient administration.

This demonstrates the usefulness of cell products and cell composition as described in the specification and the examples for treatment of bone defects in long bones as well as flat bones.

### Example 5: Duration of secondary and tertiary culture according to kinetics studies and marker expression analysis

MSC-derived cells according to the embodiment of the invention obtained after secondary culture as described in Example 1 above were harvested at different time points (D21, D22, D23, D24, D25, and D28) and cell cycle was analysed by flow cytometry (BD FACSCanto II^{™} and the BD FACSDiva^{™} softwares; Becton Dickinson).

Total cell DNA was stained with 7-amino-actinomycin D (7-AAD) using the BD Pharmigen^{™} BrdU Flow Kits. In brief, 10⁵ cells were fixed in 100 µl of BD Cytofix/Cytoperm Buffer followed by incubation for 20 min at ambient temperature and washed with 1 ml BD Perm/Wash Buffer then centrifuged 5 min at 300 g. The supernatant was discarded, and the cells were permeabilized in 100 µl of BD Cytoperm Permeabilization Buffer Plus followed by incubation for 10 min at 4°C and washed with BD Perm/Wash Buffer, then centrifuged 5 min at 300 g. The supernatant was discarded, and the cells were again fixed in 100 µl of BD Cytofix/Cytoperm Buffer (5 min at ambient temperature) and washed with BD Perm/Wash Buffer, centrifuged 5 min at 300 g. The total cell DNA was stained in 20 µl of 7-AAD solution followed by incubation for 10 min at ambient temperature in the dark. One ml of Staining Buffer was added to the samples before being transferred in flow cytometry adapted tubes.

Samples were analysed on a FACSCanto^{™} II flow cytometer (Becton-Dickinson) and 7-AAD emission was collected after passing through a 650 nm long pass filter.

Figures 3 and 4 shows that the cells at D24 were situated close to the end of the proliferation curve. The cells had not yet exited the cell cycle whereas from D25, the cells were mainly exited from the cell cycle, i.e. they did not proliferate anymore.

Figure 4 illustrates the cell cycle analysis with the events/phases (G0/G1, S and G2/M) of the cells in secondary culture at different culture durations. At D24, 42% of the cells were still proliferating (11% S and 31% G2/M) whereas from D25, the cells were mainly exited from the cell cycle. Therefore, the optimal duration for the secondary culture was 24 days.

MSC-derived cells obtained after tertiary culture as described in Example 1, were harvested at different time points: each day from day 34 (D34) to 42 (D42) and cell surface markers expression was analysed by flow cytometry as described in Example 2, Flow cytometry.

Flow cytometry marker expression analysis performed on a cell population of MSC-derived cells of chondro-osteoblastic lineage showed that differentiation marker (BMP2, RUNX2, ZNFS21, SPARC, MMP13, CHI3L1) expressions increased from D34 to D42 (Figure 5), and proliferation marker KI67 expression decreased during the culture duration (from D34 to D42).

Figure 6 illustrates the cell density at different culture durations for 8 batches. The cell density was generally higher at D36. The cells were counted by cytometry (BD Trucount^{™}). Figure 7 illustrates the mean cell diameter size at different culture durations for 2 batches. The cell diameter was minimal at D35 and D36. Therefore, cell counting and cell size measurement show that the cells were reaching a plateau of proliferation between D35 and D38 and the cell size was minimal at D35 and D36 (Figures 6 and 7).

In view of thereof and considering the cell density which is the highest at D36, the optimal duration for the tertiary culture was D36.

## Claims

1. A method for obtaining mesenchymal stem cell (MSC)-derived cells of chondro-osteoblastic lineage from MSC, the method comprising:
(a) culturing MSC recovered from a biological sample of a subject in a culture medium comprising fibroblast growth factor-2 (FGF-2), transforming growth factor beta (TGFβ) and heparin or a derivative or analogue thereof at a concentration of at least 0.01 IU/ml, thereby obtaining mesenchymal stem cell (MSC)-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are cultured in step (b) for a period of x days, wherein day x is the last day on which at least 20% of the MSC-derived cells are proliferating.

2. The method according to claim 1, wherein the MSC-derived cells are cultured in step (b) for a period of from about 8 days to about 12 days, preferably for a period of about 10 days.

3. The method according to claim 1 or 2, wherein the MSC-derived cells are cultured in step (a) for a period of from about 13 days to about 15 days, preferably for a period of about 14 days.

4. The method according to any one of claims 1 to 3, wherein the MSC-derived cells are cultured in step (c) for a period of from about 10 days to about 14 days, preferably for a period of about 12 days.

5. The method according to any one of claims 1 to 4, wherein the proliferating MSC-derived cells are in S phase, G2 phase or M phase of the cell cycle.

6. A method for obtaining MSC-derived cells of chondro-osteoblastic lineage from MSC, the method comprising:
(a) culturing MSC recovered from a biological sample of a subject in a culture medium comprising FGF-2, TGFβ and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a); and
(c) passaging the MSC-derived cells for a second time and further culturing the MSC-derived cells in a medium as defined in (a), thereby obtaining the MSC-derived cells of chondro-osteoblastic lineage,
wherein the MSC-derived cells are plated for the further culturing in step (b) at a density of 3×10² to 1×10³ cells/cm², preferably at a density of 3×10² to 8×10² cells/cm².

7. The method according to claim 6, wherein the MSC-derived cells are plated for the further culturing in step (c) at a density of 3×10² to 1×10³ cells/cm², preferably at a density of 3×10² to 8×10² cells/cm².

8. A method for obtaining MSC-derived cells of chondro-osteoblastic lineage from MSC, the method comprising:
(a) culturing MSC recovered from a biological sample of a subject in a culture medium comprising FGF-2, TGFβ and heparin or a derivative or analogue thereof at a concentration of at least 0.01 IU/ml, thereby obtaining MSC-derived cells;
(b) passaging the MSC-derived cells for a first time and further culturing the MSC-derived cells in a medium as defined in (a);
(c) passaging the MSC-derived cells for a second time and further culturing the MSC-derived cells in a medium as defined in (a), thereby obtaining the MSC-derived cells of chondro-osteoblastic lineage;
(d) resuspending the MSC-derived cells of chondro-osteoblastic lineage in a cryopreservation medium suitable for administration to a subject; and
(e) cryopreserving the MSC-derived cells of chondro-osteoblastic lineage.

9. The method according to claim 8, wherein the MSC-derived cells of chondro-osteoblastic lineage are resuspended in the cryopreservation medium in step (d) at a concentration of between about 1×10⁷/ml and about 1×10⁸/ml, preferably at a concentration of between about 2×10⁷/ml and about 4×10⁷/ml.

10. The method according to claim 8 or 9, wherein the cryopreservation medium comprises dimethylsulfoxide (DMSO), human serum albumin (HSA), adenosine, polypeptide, benzopyran, or a combination thereof.

11. The method according to any one of claims 1 to 10, wherein TGFβ is selected from the group consisting of TGFβ1, TGFβ2, TGFβ3, and mixtures thereof; preferably wherein TGFβ is TGFβ1.

12. The method according to any one of claims 1 to 11, wherein:
- the concentration of heparin or derivative or analogue thereof is about 0.1 IU/ml; and/or
- heparin or heparin derivative or analogue thereof is selected from the group consisting of unfractionated heparin (UFH); low molecular weight heparin (LMWH), such as enoxaparin, dalteparin, nadroparin, tinzaparin, certoparin, reviparin, ardeparin, pamaparin, bemiparin, or mixtures thereof; a heparinoid, such as heparan sulfate, dermatan sulfate, chondroitin sulfate, acharan sulfate, keratan sulfate, or mixtures thereof, such as danaparoid; a heparin salt; a heparinoid salt; a heparin fragment; a heparinoid fragment; and mixtures thereof.

13. The method according to any one of claims 1 to 12, wherein the MSC or MSC-derived cells are additionally contacted with, such as wherein the medium additionally comprises one or more of, plasma, serum or a substitute thereof.

14. The method according to any one of claims 1 to 13, wherein the subject is a human subject.

15. A population of MSC-derived cells of chondro-osteoblastic lineage obtainable or obtained by *in vitro* or *ex vivo* expansion of MSC, whereby at least 90% of the MSC-derived cells of chondro-osteoblastic lineage in suspension have a diameter equal to or less than 25 µm (D₉₀ ≤ 25 µm) and wherein at most 1 % of the MSC-derived cells of chondro-osteoblastic lineage in suspension have a diameter of more than 35 µm, and wherein the MSC-derived cells of chondro-osteoblastic lineage are obtainable or obtained by the methods as defined in any one of claims 1 to 14.

16. A pharmaceutical formulation comprising the population of MSC-derived cells of chondro-osteoblastic lineage as defined in claim 15.

17. The pharmaceutical formulation according to claim 16, wherein the pharmaceutical formulation further comprises a component with osteo-conductive properties, such as tricalcium phosphate, hydroxyapatite, combination of hydroxyapatite/tricalcium phosphate particles, poly-lactic acid, poly-lactic glycolic acid, hyaluronic acid or a derivative thereof, chitosan, poly-L-lysine, gelatine, collagen, osteonectin, fibrinogen, osteocalcin, or a combination thereof.

18. The population of MSC-derived cells of chondro-osteoblastic lineage according to claim 15, or the pharmaceutical formulation according to claim 16 or 17, for use as a medicament, preferably for use in the treatment of a subject in need of transplantation of cells of chondro-osteoblastic lineage.

19. The population or the pharmaceutical formulation for use according to claim 18, wherein:
- the MSC-derived cells of chondro-osteoblastic lineage are present at a concentration between about 1×10⁷/ml and about 1×10⁸/ml, preferably between about 2×10⁷/ml and about 4×10⁷ cells/ml; and/or
- the population of MSC-derived cells of chondro-osteoblastic lineage or the pharmaceutical formulation is suitable for percutaneous, intra-osseous, intra-articular, intervertebral or intravascular administration;
- the population of MSC-derived cells of chondro-osteoblastic lineage or the pharmaceutical formulation is suitable for administration at a site of a bone defect.

## Patentansprüche

1. Verfahren zur Gewinnung von aus mesenchymalen Stammzellen (MSC) abgeleiteten Zellen chondro-osteoblastischer Abstammung aus MSC, wobei das Verfahren Folgendes umfasst:
(a) Kultivieren von MSC, die aus einer biologischen Probe eines Subjekts gewonnen wurden, in einem Kulturmedium, das Fibroblasten-Wachstumsfaktor-2 (FGF-2), transformierenden Wachstumsfaktor beta (TGFβ) und Heparin oder ein Derivat oder Analogon davon in einer Konzentration von mindestens 0,01 IU/ml umfasst, wodurch Zellen erhalten werden, die von mesenchymalen Stammzellen (MSC) abgeleitet sind;
(b) erstmaliges Passagieren der MSC-abgeleiteten Zellen und weiteres Kultivieren der MSC-abgeleiteten Zellen in einem Medium wie in (a) definiert, und
(c) zweites Passagieren der MSC-abgeleiteten Zellen und weiteres Kultivieren der MSC-abgeleiteten Zellen in einem Medium wie in (a) definiert, wodurch die MSC-abgeleiteten Zellen chondro-osteoblastischer Abstammung erhalten werden,
wobei die MSC-abgeleiteten Zellen in Schritt (b) für einen Zeitraum von x Tagen kultiviert werden, wobei Tag x der letzte Tag ist, an dem mindestens 20 % der MSC-abgeleiteten Zellen proliferieren.

2. Verfahren nach Anspruch 1, wobei die MSC-abgeleiteten Zellen in Schritt (b) für einen Zeitraum von etwa 8 Tagen bis etwa 12 Tagen, vorzugsweise für einen Zeitraum von etwa 10 Tagen, kultiviert werden.

3. Verfahren nach Anspruch 1 oder 2, wobei die MSC-abgeleiteten Zellen in Schritt (a) für einen Zeitraum von etwa 13 Tagen bis etwa 15 Tagen, vorzugsweise für einen Zeitraum von etwa 14 Tagen, kultiviert werden.

4. Verfahren nach einem der Ansprüche 1 bis 3, wobei die MSC-abgeleiteten Zellen in Schritt (c) für einen Zeitraum von etwa 10 Tagen bis etwa 14 Tagen, vorzugsweise für einen Zeitraum von etwa 12 Tagen, kultiviert werden.

5. Verfahren nach einem der Ansprüche 1 bis 4, wobei sich die proliferierenden MSC-abgeleiteten Zellen in der S-Phase, G2-Phase oder M-Phase des Zellzyklus befinden.

6. Verfahren zur Gewinnung von MSC-abgeleiteten Zellen chondro-osteoblastischer Abstammung aus MSC, wobei das Verfahren umfasst:
(a) Kultivieren von MSC, die aus einer biologischen Probe eines Subjekts gewonnen wurden, in einem Kulturmedium, das FGF-2, TGFß und Heparin oder ein Derivat oder Analogon davon in einer Konzentration von mindestens 0,01 IU/ml umfasst, wodurch MSC-abgeleitete Zellen erhalten werden;
(b) erstmaliges Passagieren der MSC-abgeleiteten Zellen und weiteres Kultivieren der MSC-abgeleiteten Zellen in einem Medium, wie in (a) definiert, und
(c) zweites Passagieren der MSC-abgeleiteten Zellen und weiteres Kultivieren der MSC-abgeleiteten Zellen in einem Medium wie in (a) definiert, wodurch die MSC-abgeleiteten Zellen mit chondro-osteoblastischer Abstammung erhalten werden,
wobei die MSC-abgeleiteten Zellen für die weitere Kultivierung in Schritt (b) in einer Dichte von 3×10² bis 1×10³ Zellen/cm², vorzugsweise in einer Dichte von 3×10² bis 8×10² Zellen/cm² plattiert werden.

7. Verfahren nach Anspruch 6, wobei die MSC-abgeleiteten Zellen für die weitere Kultivierung in Schritt (c) in einer Dichte von 3×10² bis 1×10³ Zellen/cm², vorzugsweise in einer Dichte von 3×10² bis 8×10² Zellen/cm² plattiert werden.

8. Verfahren zur Gewinnung von MSC-abgeleiteten Zellen mit chondro-osteoblastischer Abstammung aus MSC, wobei das Verfahren folgendes umfasst:
(a) Kultivieren von MSC, die aus einer biologischen Probe eines Subjekts gewonnen wurden, in einem Kulturmedium, das FGF-2, TGFß und Heparin oder ein Derivat oder Analogon davon in einer Konzentration von mindestens 0,01 IU/ml umfasst, wodurch MSC-abgeleitete Zellen erhalten werden;
(b) erstmaliges Passagieren der MSC-abgeleiteten Zellen und weiteres Kultivieren der MSC-abgeleiteten Zellen in einem Medium wie in (a) definiert,
(c) zweites Passagieren der MSC-abgeleiteten Zellen und weiteres Kultivieren der MSC-abgeleiteten Zellen in einem Medium wie in (a) definiert, wodurch die MSC-abgeleiteten Zellen chondro-osteoblastischer Abstammung erhalten werden;
(d) Resuspendieren der MSC-abgeleiteten Zellen hondro-osteoblastischer Abstammung in einem Kryokonservierungsmedium, das zur Verabreichung an ein Subjekt geeignet ist; und
(e) Kryokonservieren der MSC-abgeleiteten Zellen chondro-osteoblastischer Abstammung.

9. Verfahren nach Anspruch 8, wobei die MSC-abgeleiteten Zellen chondro-osteoblastischer Abstammung in dem Kryokonservierungsmedium in Schritt (d) in einer Konzentration zwischen etwa 1×10⁷/ml und etwa 1×10⁸/ml, vorzugsweise in einer Konzentration zwischen etwa 2×10⁷/ml und etwa 4×10⁷/ml resuspendiert werden.

10. Verfahren nach Anspruch 8 oder 9, wobei das Kryokonservierungsmedium Dimethylsulfoxid (DMSO), humanes Serumalbumin (HSA), Adenosin, Polypeptid, Benzopyran oder eine Kombination davon umfasst.

11. Verfahren nach einem der Ansprüche 1 bis 10, wobei TGFß ausgewählt ist aus der Gruppe bestehend aus TGFß1, TGFß2, TGFß3 und Mischungen davon, wobei TGFß vorzugsweise TGFß1 ist.

12. Verfahren nach einem der Ansprüche 1 bis 11, wobei:
- die Konzentration von Heparin oder einem Derivat oder Analogon davon etwa 0,1 IU/ml beträgt; und/oder
- Heparin oder das Heparinderivat oder Analogon davon ausgewählt ist aus der Gruppe bestehend aus unfraktioniertem Heparin (UFH); niedermolekularem Heparin (LMWH), wie Enoxaparin, Dalteparin, Nadroparin, Tinzaparin, Certoparin, Reviparin, Ardeparin, Pamaparin, Bemiparin oder Mischungen davon; einem Heparinoid, wie Heparansulfat, Dermatansulfat, Chondroitinsulfat, Acharansulfat, Keratansulfat oder Mischungen davon, wie Danaparoid; einem Heparinsalz; einem Heparinoidsalz; einem Heparinfragment; einem Heparinoidfragment; und Mischungen davon.

13. Verfahren nach einem der Ansprüche 1 bis 12, wobei die MSC oder die MSC-abgeleiteten Zellen zusätzlich kontaktiert werden mit, wie z. B., wobei das Medium zusätzlich eines oder mehrere von Plasma, Serum oder einem Ersatz davon umfasst.

14. Verfahren nach einem der Ansprüche 1 bis 13, wobei das Subjekt ein menschliches Subjekt ist.

15. Population von MSC-abgeleiteten Zellen chondro-osteoblastischer Abstammung, erhältlich oder erhalten durch *In-vitro-* oder *Ex-vivo*-Expansion von MSC, wobei mindestens 90 % der MSC-abgeleiteten Zellen chondro-osteoblastischer Abstammung in Suspension einen Durchmesser von gleich oder weniger als 25 µm (D₉₀ ≤ 25 µm) haben und wobei höchstens 1 % der MSC-abgeleiteten Zellen chondro-osteoblastischer Abstammung in Suspension einen Durchmesser von mehr als 35 µm haben, wobei die MSC-abgeleiteten Zellen chondro-osteoblastischer Abstammung durch die in einem der Ansprüche 1 bis 14 definierten Verfahren erhältlich sind oder erhalten werden.

16. Pharmazeutische Formulierung, umfassend die Population von MSC-abgeleiteten Zellen chondro-osteoblastischer Abstammung, wie in Anspruch 15 definiert.

17. Pharmazeutische Formulierung nach Anspruch 16, wobei die pharmazeutische Formulierung ferner eine Komponente mit osteokonduktiven Eigenschaften umfasst, wie Tricalciumphosphat, Hydroxylapatit, eine Kombination aus Hydroxyapatit/Tricalciumphosphat-Partikeln, Polymilchsäure, Polymilchsäure-Glykolsäure, Hyaluronsäure oder ein Derivat davon, Chitosan, Poly-L-Lysin, Gelatine, Kollagen, Osteonectin, Fibrinogen, Osteocalcin oder eine Kombination davon.

18. Population von MSC-abgeleiteten Zellen der chondro-osteoblastischen Abstammung nach Anspruch 15 oder pharmazeutische Formulierung nach Anspruch 16 oder 17 zur Verwendung als Arzneimittel, vorzugsweise zur Verwendung bei der Behandlung eines Subjekts, das eine Transplantation von Zellen chondro-osteoblastischer Abstammung benötigt.

19. Population oder pharmazeutische Formulierung zur Verwendung nach Anspruch 18, wobei:
- die MSC-abgeleiteten Zellen chondro-osteoblastischer Abstammung in einer Konzentration zwischen etwa 1×10⁷/ml und etwa 1×10⁸/ml, vorzugsweise zwischen etwa 2×10⁷/ml und etwa 4×10⁷ Zellen/ml, vorliegen; und/oder
- die Population von MSC-abgeleiteten Zellen chondro-osteoblastischer Abstammung oder die pharmazeutische Formulierung zur perkutanen, intraossären, intraartikulären, intervertebralen oder intravaskulären Verabreichung geeignet ist;
- die Population von MSC-abgeleiteten Zellen chondro-osteoblastischer Abstammung oder die pharmazeutische Formulierung zur Verabreichung an einer Stelle eines Knochendefekts geeignet ist.

## Revendications

1. Procédé pour l'obtention de cellules issues de cellules souches mésenchymateuses (MSC) de lignée chondro-ostéoblastique de MSC, le procédé comprenant :
(a) la culture de MSC récupérées d'un échantillon biologique d'un sujet dans un milieu de culture comprenant le facteur-2 de croissance de fibroblastes (FGF-2), le facteur de croissance transformant bêta (TGFβ) et de l'héparine ou un dérivé ou analogue correspondant à une concentration d'au moins 0,01 UI/ml, obtenant ainsi des cellules issues de cellules souches mésenchymateuses (MSC) ;
(b) le repiquage des cellules issues de MSC pour une première fois et la culture supplémentaire des cellules issues de MSC dans un milieu tel que défini en (a) ; et
(c) le repiquage des cellules issues de MSC pour une deuxième fois et la culture supplémentaire des cellules issues de MSC dans un milieu tel que défini en (a), obtenant ainsi les cellules issues de MSC de lignée chondro-ostéoblastique,
les cellules issues de MSC étant cultivées dans l'étape (b) pendant une période de x jours, le jour x étant le dernier jour auquel au moins 20 % des cellules issues de MSC sont proliférantes.

2. Procédé selon la revendication 1, les cellules issues de MSC étant cultivées dans l'étape (b) pendant une période allant d'environ 8 jours à environ 12 jours, préférablement pendant une période d'environ 10 jours.

3. Procédé selon la revendication 1 ou 2, les cellules issues de MSC étant cultivées dans l'étape (a) pendant une période allant d'environ 13 jours à environ 15 jours, préférablement pendant une période d'environ 14 jours.

4. Procédé selon l'une quelconque des revendications 1 à 3, les cellules issues de MSC étant cultivées dans l'étape (c) pendant une période allant d'environ 10 jours à environ 14 jours, préférablement pendant une période d'environ 12 jours.

5. Procédé selon l'une quelconque des revendications 1 à 4, les cellules issues de MSC proliférantes étant en phase S, en phase G2 ou en phase M du cycle cellulaire.

6. Procédé pour l'obtention de cellules issues de MSC de lignée chondro-ostéoblastique de MSC, le procédé comprenant :
(a) la culture de MSC récupérées d'un échantillon biologique d'un sujet dans un milieu de culture comprenant FGF-2, TGFβ et de l'héparine ou un dérivé ou analogue correspondant à une concentration d'au moins 0,01 UI/ml, obtenant ainsi des cellules issues de MSC ;
(b) le repiquage des cellules issues de MSC pour une première fois et la culture supplémentaire des cellules issues de MSC dans un milieu tel que défini en (a) ; et
(c) le repiquage des cellules issues de MSC pour une deuxième fois et la culture supplémentaire des cellules issues de MSC dans un milieu tel que défini en (a), obtenant ainsi les cellules issues de MSC de lignée chondro-ostéoblastique,
les cellules issues de MSC étant plaquées pour la culture supplémentaire dans l'étape (b) à une densité de 3 × 10² à 1 × 10³ cellules/cm², préférablement à une densité de 3 × 10² à 8 × 10² cellules/cm².

7. Procédé selon la revendication 6, les cellules issues de MSC étant plaquées pour la culture supplémentaire dans l'étape (c) à une densité de 3 × 10² à 1 × 10³ cellules/cm², préférablement à une densité de 3 × 10² à 8 × 10² cellules/cm².

8. Procédé pour l'obtention de cellules issues de MSC de lignée chondro-ostéoblastique de MSC, le procédé comprenant :
(a) la culture de MSC récupérées d'un échantillon biologique d'un sujet dans un milieu de culture comprenant FGF-2, TGFβ et de l'héparine ou un dérivé ou analogue correspondant à une concentration d'au moins 0,01 UI/ml, obtenant ainsi des cellules issues de MSC ;
(b) le repiquage des cellules issues de MSC pour une première fois et la culture supplémentaire des cellules issues de MSC dans un milieu tel que défini en (a) ;
(c) le repiquage des cellules issues de MSC pour une deuxième fois et la culture supplémentaire des cellules issues de MSC dans un milieu tel que défini en (a), obtenant ainsi les cellules issues de MSC de lignée chondro-ostéoblastique ;
(d) la remise en suspension des cellules issues de MSC de lignée chondro-ostéoblastique dans un milieu de cryoconservation approprié pour une administration à un sujet ; et
(e) la cryoconservation des cellules issues de MSC de lignée chondro-ostéoblastique.

9. Procédé selon la revendication 8, les cellules issues de MSC de lignée chondro-ostéoblastique étant remises en suspension dans le milieu de cryoconservation dans l'étape (d) à une concentration comprise entre environ 1 × 10⁷/ml et environ 1 × 10⁸/ml, préférablement à une concentration comprise entre environ 2 × 10⁷/ml et environ 4 × 10⁷/ml.

10. Procédé selon la revendication 8 ou 9, le milieu de cryoconservation comprenant du diméthylsulfoxyde (DMSO), de l'albumine sérique humaine (HSA), de l'adénosine, un polypeptide, du benzopyranne, ou une combinaison correspondante.

11. Procédé selon l'une quelconque des revendications 1 à 10, TGFβ étant choisi dans le groupe constitué par TGFβ1, TGFβ2, TGFβ3, et des mélanges correspondants ; préférablement TGFβ étant TGFβ1.

12. Procédé selon l'une quelconque des revendications 1 à 11,
- la concentration en héparine ou en dérivé ou analogue correspondant étant d'environ 0,1 UI/ml ; et/ou
- l'héparine ou un dérivé d'héparine ou analogue correspondant étant choisi(e) dans le groupe constitué par l'héparine non fractionnée (HNF) ; l'héparine de faible poids moléculaire (HBPM), telle que l'énoxaparine, la daltéparine, la nadroparine, la tinzaparine, la certoparine, la réviparine, l'ardéparine, la parnaparine, la bémiparine, ou des mélanges correspondants ; un héparinoïde, tel que le sulfate d'héparane, le sulfate de dermatane, le sulfate de chondroïtine, le sulfate d'acharane, le sulfate de kératane ou des mélanges correspondants, tel qu'un danaparoïde ; un sel d'héparine ; un sel d'héparinoïde ; un fragment d'héparine ; un fragment d'héparinoïde ; et des mélanges correspondants.

13. Procédé selon l'une quelconque des revendications 1 à 12, les MSC ou les cellules issues de MSC étant de plus mises en contact avec, par exemple le milieu comprenant de plus l'un ou plusieurs parmi, du plasma, du sérum ou un substitut correspondant.

14. Procédé selon l'une quelconque des revendications 1 à 13, le sujet étant un sujet humain.

15. Population de cellules issues de MSC de lignée chondro-ostéoblastique pouvant être obtenue ou obtenue par expansion de MSC *in vitro* ou *ex vivo,* moyennant quoi au moins 90 % des cellules issues de MSC de lignée chondro-ostéoblastique en suspension ont un diamètre égal ou inférieur à 25 µm (D₉₀ ≤ 25 µm) et, au plus 1 % des cellules issues de MSC de lignée chondro-ostéoblastique en suspension ayant un diamètre de plus de 35 µm, et les cellules issues de MSC de lignée chondro-ostéoblastique pouvant être obtenues ou étant obtenues par les procédés tels que définis dans l'une quelconque des revendications 1 à 14.

16. Formulation pharmaceutique comprenant la population de cellules issues de MSC de lignée chondro-ostéoblastique telle que définie dans la revendication 15.

17. Formulation pharmaceutique selon la revendication 16, la formulation pharmaceutique comprenant en outre un composant doté de propriétés ostéo-conductrices, tel que le phosphate tricalcique, l'hydroxyapatite, une combinaison de particules d'hydroxyapatite/phosphate tricalcique, un poly(acide lactique), un poly-acide lactique glycolique, l'acide hyaluronique ou un dérivé correspondant, un chitosane, une poly-L-lysine, une gélatine, un collagène, l'ostéonectine, le fibrinogène, l'ostéocalcine, ou une combinaison correspondante.

18. Population de cellules issues de MSC de lignée chondro-ostéoblastique selon la revendication 15, ou formulation pharmaceutique selon la revendication 16 ou 17, pour une utilisation en tant que médicament, préférablement pour une utilisation dans le traitement d'un sujet ayant besoin d'une transplantation de cellules de lignée chondro-ostéoblastique.

19. Population ou formulation pharmaceutique pour une utilisation selon la revendication 18,
- les cellules issues de MSC de lignée chondro-ostéoblastique étant présentes à une concentration comprise entre environ 1 × 10⁷/ml et environ 1 × 10⁸/ml, préférablement comprise entre environ 2 × 10⁷/ml et environ 4 × 10⁷ cellules/ml ; et/ou
- la population de cellules issues de MSC de lignée chondro-ostéoblastique ou la formulation pharmaceutique étant appropriée pour une administration percutanée, intraosseuse, intraarticulaire, intervertébrale ou intravasculaire ;
- la population de cellules issues de MSC de lignée chondro-ostéoblastique ou la formulation pharmaceutique étant appropriée pour une administration au niveau d'un site d'un défaut osseux.
